# FASCICULE DE BREVET EUROPEEN

(11) **EP 0 434 570 B1**
(45) Date de publication et mention de la délivrance du brevet: **27.03.1996**
(21) Numéro de dépôt: 90403726.4
(22) Date de dépôt: 21.12.1990
(51) Int. Cl.: C07J 31/00, C07J 41/00, A61K 31/56, C07J 21/00

(54) **Produits stéroides comportant, en position 10, un radical thioéthyle substitué, leur procédé de préparation et les intermédiaires de ce procédé, leur application comme médicaments et les compositions pharmaceutiques les renfermant**
Steroidderivate, die ein substituiertes Thioethylradikal an der Stelle 10 des Steroids tragen, Verfahren zu ihrer Herstellung und Zwischenprodukte davon, ihre Verwendung als Arzneimittel und pharmazeutische Präparate davon
Steroid products carrying a substituted thioethyl group in position 10, a process for their preparation and intermediates of this process, their use as medicines and pharmaceutical preparations containing them

(30) Priorité: 22.12.1989 FR 8917047
(43) Date de publication de la demande: 26.06.1991
(73) Titulaire: ROUSSEL UCLAF, F-93230 Romainville (FR)
(72) Inventeur: Gourvest, Jean-François, F-77410 Claye-Souilly (FR); Lesuisse, Dominique, F-75018 Paris (FR)
(74) Mandataire: Vieillefosse, Jean-Claude

(56) Documents cités:
- EP-A- 0 149 499
- EP-A- 0 289 450
- EP-A- 0 289 451
- J.MED.CHEM., VOL.28, PAGE 803 (1985)
- JACS, VOL.60, PAGE 1702 (1938)
- ANN., VOL. 568, PAGE 52 (1950)
- "STEROIDS", FIESER AND FIESER, 1959, PAGES 307-311
- CHEM. BER., VOL. 20, PAGE 2845 (1887)
- ORGANIC FUNCTIONAL GROUP PREPARATIONS, SANDLER AND KARO, VOL. 3, PAGES 372-381 (1972)
- MARCH, 3rd edition, Page 796

## Description

La présente invention concerne de nouveaux produits stéroïdes comportant, en position 10, un radical thioéthyle éventuellement substitué, leur procédé de préparation et les intermédiaires de ce procédé, leur application comme médicaments et les compositions pharmaceutiques les renfermant.

L'invention a pour objet les produits de formule générale (I) : 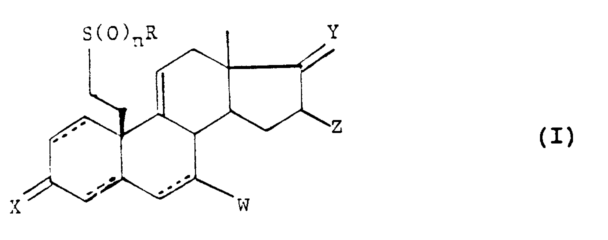
dans laquelle R représente :
un atome d'hydrogène,
un radical alkyle, alcényle, alcynyle, alkylthio ayant au plus 6 atomes de carbone ou arylthio éventuellement substitué ayant au plus 10 atomes de carbone,
un radical, acyle ayant au plus 12 atomes de carbone ou un radical CN,
- X représente un atome d'oxygène, un radical N-O-R₁ dans lequel R₁ représente un atome d'hydrogène ou un radical alkyle ayant de 1 à 6 atomes de carbone ou X représente 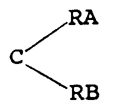 dans lequel RA et RB sont identiques et représentent un atome d'hydrogène, ou =X représente 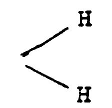 ou un reste 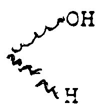 dans lequel le radical OH peut se trouver en position alpha ou bêta, le radical hydroxyle étant éventuellement acylé, - Y représente un atome d'oxygène, un radical N-O-R₁ dans lequel R₁ a la définition indiquée ci-dessus, ou =Y représente 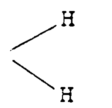 ou un reste 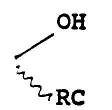 dans lequel RC représente un atome d'hydrogène le radical hydroxyle étant éventuellement acylé ou =Y représente un groupe 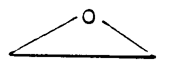 (CH₂)q dans lequel q représente un nombre de 1 à 3,
- W représente un atome d'hydrogène, un radical alkylthio ou arylthio éventuellement substitué renfermant jusqu'à 10 atomes de carbone,
- Z représente un atome d'hydrogène ou un radical alkyle renfermant jusqu'à 10 atomes de carbone,
- n représente un entier de 0 à 1 et les traits pointillés en positions 1(2), 4(5) et 6(7) indiquent la présence éventuelle d'une seconde liaison entre les carbones qui les portent,
ainsi que leurs sels,
étant entendu que lorsque n est égal à 1, R représente un radical Methyle, X et Y représentent chacun un atome d'oxygène, W et Z représentent chacun un atome d'hydrogène, le trait en pointillé en position 4(5) représente une seconde liaison entre les carbones qui les portent et les traits pointillés en position 1(2) et 6(7) ne représentent pas une seconde liaison entre les carbones qui les portent.

Parmi les radicaux alkyle, on peut citer les radicaux méthyle, éthyle, propyle, isopropyle, butyle, isobutyle, butyle secondaire, tert-butyle, pentyle, hexyle.

Parmi les radicaux alcényle et alcynyle, on peut citer les radicaux vinyle, allyle, 1-propényle, éthynyle, 1 ou 2-propynyle.

Comme radicaux haloalkyle on comprend les radicaux alkyle tels que ceux mentionnés ci-dessus, mono ou plurisubstitués par les atomes d'halogènes : fluor, chlore, brome, iode. Par plurisubstitué on entend plus particulièrement les radicaux alkyle di ou trisubstitués.

Comme exemples particuliers, on peut citer les mono-fluoro, chloro, bromo ou iodométhyle, les difluoro, dichloro ou dibromométhyle, le trifluorométhyle. Les radicaux alkylthio correspondent aux radicaux alkyle mentionnés ci-dessus. On peut citer par exemple les radicaux méthylthio et éthylthio.

Les radicaux aryles peuvent être choisis parmi les aryles carbocycliques tels que le phényle ou le naphtyle ou les aryles hétérocycliques à 5 ou 6 chaînons comportant un ou plusieurs hétéroatomes choisis de préférence parmi l'oxygène, le soufre et l'azote. Parmi les aryles hétérocycliques à 5 chaînons on peut citer les radicaux furyle, thiényle, pyrrolyle, thiazolyle, oxazolyle, imidazolyle, thiadiazolyle, pyrazolyle, isoxazolyle.

Parmi les aryles hétérocycliques à 6 chaînons on peut citer les radicaux pyridyle, pyrimidinyle, pyridazinyle, pyrazinyle.

Parmi les radicaux aryles condensés on peut citer les radicaux indolyle, benzofurannyle, benzothiényle, quinoléïnyle.

Les radicaux Arylthio correspondent aux radicaux aryles mentionnés ci-dessus. On peut citer par exemple les radicaux phénylthio non substitués ou substitués par un ou plusieurs radicaux amino ou nitro.

Parmi les atomes d'halogène, en particulier les atomes de fluor, de chlore, de brome ou d'iode.

Parmi les radicaux cycloalkyle renfermant de 3 à 6 atomes, on peut citer les radicaux cyclopropyle, cyclobutyle, cyclopentyle ou cyclohexyle.

Parmi les radicaux acyles, on préfère le reste d'un acide aliphatique ou cycloaliphatique saturé ou insaturé et notamment le reste d'un acide alcanoïque tel que par exemple l'acide acétique, propionique, butyrique ou isobutyrique, valérique ou undécylique, le reste d'un acide hydroxyalcanoïque tel que par exemple l'acide hydroxyacétique, le reste d'un acide cycloalkylcarboxylique ou (cycloalkyle) alcanoïque tel que par exemple l'acide cyclopropyl, cyclopentyl ou cyclohexylcarboxylique, cyclopentyle ou cyclohexyle acétique ou propionique, le reste d'un acide benzoïque ou d'un acide phénylalcanoïque tel que l'acide phényl acétique ou phényl propionique, le reste d'un amino acide tel que l'acide diéthylamino acétique ou aspartique ou le reste de l'acide formique. On préfère les radicaux acétyle, propionyle ou benzoyle.

Par radical hydroxy acylé, on entend de préférence un radical alcanoyloxy dérivé d'un acide alcanoïque tel que défini ci-dessus ou un radical de formule 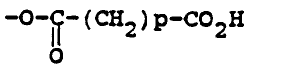
dans lequel p représente un nombre de 2 à 5.

Parmi les valeurs de R, on préfère les radicaux méthyle ou éthyle, acétyle, fluorométhyle, difluorométhyle, méthylthio, vinyle, allyle ou éthynyle.

Parmi les valeurs de R₁, on préfère les valeurs hydrogène ou méthyle.

Parmi les produits de formule (I) on préfère les produits dans lesquels R ne peut pas représenter un atome d'hydrogène lorsque X ne représente pas un radical méthylène.

L'invention a plus particulièrement pour objet les produits préférés de formule I, de formule (I') : 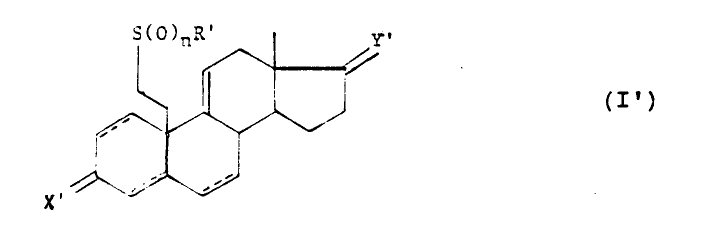
dans laquelle R' représente :
un radical alkyle, alcényle, alcynyle, ou alkylthio ayant au plus 4 atomes de carbone,
un radical CN,
un radical acyle ayant au plus 4 atomes de carbone,
X' représente un atome d'oxygène, un radical CH₂, un radical N-O-R'₁ dans lequel R'₁ représente un atome d'hydrogène ou un radical alkyle ayant de 1 à 4 atomes de carbone, ou X' représente un reste 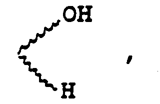
- Y' représente un atome d'oxygène ou =Y' représente un reste, 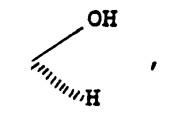 le radical hydroxy éventuellement acylé sous forme de radical alcanoyloxy ou d'un radical 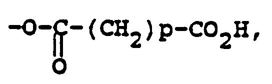 p étant défini comme précédemment.

Les traits pointillés en positions 1(2), 4(5) et 6(7) indiquent la présence éventuelle d'une seconde liaison entre les carbones qui les portent, ainsi que leurs sels.

Parmi les produits de formule (I') telle que définie ci-dessus, on préfère les produits dans lesquels R' est choisi parmi les radicaux alkyle, alcényle, ou alcynyle ayant de 1 à 4 atomes de carbone et X' représente un atome d'oxygène ou un reste

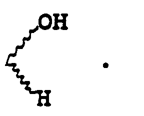

Parmi les produits de formule (I'), l'invention a particulièrement pour objet les produits décrits ci-après dans les exemples et en particulier :
- la 10bêta-[2-(méthylthio) éthyl] estra-4,9(11)-diène-3,17-dione,
- la 10bêta-[2-(éthylthio) éthyl] estra-4,9(11)-diène-3,17-dione,
- la 10bêta-[2-(méthylthio) éthyl] 17bêta-hydroxy estra-4,9(11)-diène-3-one,
- la 10bêta-[2-(éthénylthio) éthyl] estra-4,9(11)-diène-3,17-dione,
- la 10bêta-[2-(éthynylthio) éthyl] estra-4,9(11)-diène-3,17-dione,
- le butane dioate de 10bêta-[2-(méthylthio) éthyl] 3-oxo estra-4,9(11)-dièn 17bêta-yle et de sodium,
- le 10bêta-[2-(méthylthio) éthyl] estra-4,9(11)-diène-3bêta, 17bêta-diol,
- la 10bêta-[2-(méthylthio) éthyl] estra-4,9(11)-diène-3alpha, 17bêta-diol,
- la 10bêta-[2-(méthylthio) éthyl] estr-9(11)-ène-3,17-dione.

La présente invention a également pour objet un procédé de préparation des produits de formule générale (I) telle que définie ci-dessus et caractérisé en ce que l'on soumet à une réduction un produit de formule (II) : 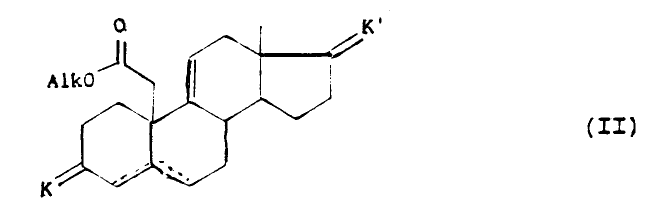
dans laquelle K et K' identiques ou différents représentent un radical céto protégé étant entendu que lorsque K représente un éther d'énol, les doubles liaisons se trouvent en position 3(4) et 5(6) et Alk représente un radical alkyle de 1 à 4 atomes de carbone, les traits pointillés représentant une double liaison en 4(5) ou 5(6), pour obtenir un produit de formule (III) : 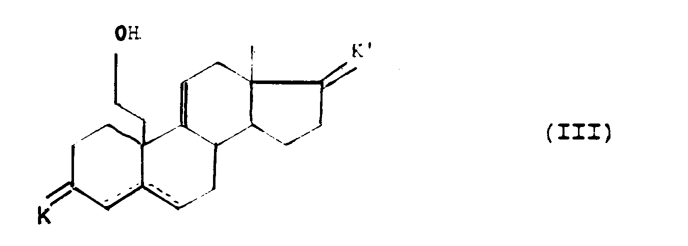
que l'on traite,
ou bien par un reactif de transformation du radical hydroxy en radical thiol pour obtenir un produit de formule (IV) : 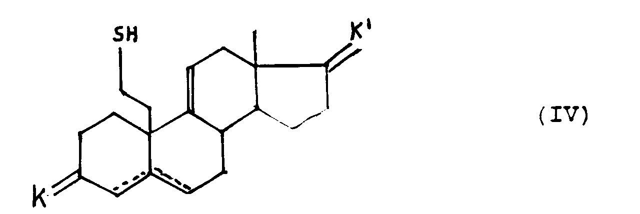
que l'on traite par un dérive reactif du radical R pour obtenir un produit de formule (V) : 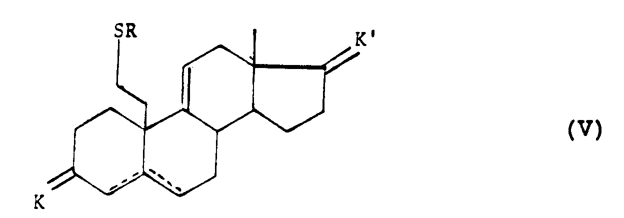
ou bien par un dérivé réactif d'un acide sulfonique pour obtenir un produit de formule (VI) : 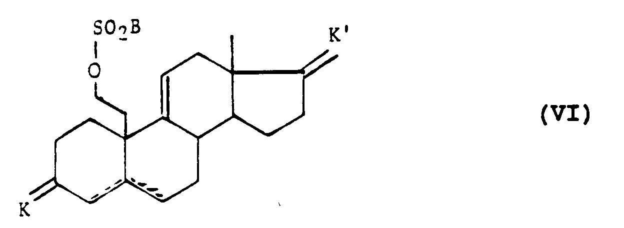
dans laquelle B représente le reste d'un acide sulfonique produit de formule (VI) que l'on traite par un sel de formule RSA dans laquelle A représente un cation monovalent pour obtenir un produit de formule (V) telle que définie ci-dessus, produit de formule (V) que l'on traite par un réactif de déprotection des fonctions cétones protégées pour obtenir un produit de formule (Ia) : 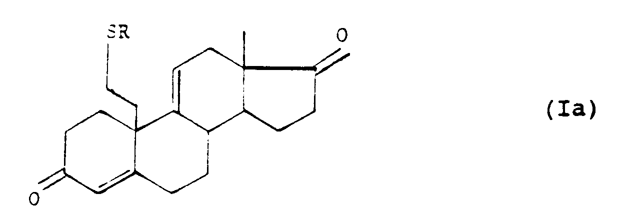
correspondant aux produits de formule (I) dans laquelle R a les significations indiquées ci-dessus, X et Y représentent chacun un atome d'oxygène, n représente le nombre 0, et les traits pointillés en position 4(5) représentent une double liaison et les traits pointillés en positions 1(2) et 6(7) ne représentent pas une seconde liaison entre les carbones qui les portent, produits de formule (Ia) que, si désiré l'on soumet à l'une ou plusieurs des réactions suivantes et dans un ordre quelconque :
- introduction d'une double liaison en position 1(2)
- introduction d'une double liaison en position 6(7)
- oxydation de l'atome de soufre du radical -SR en sulfoxyde
- réduction sélective de la double liaison en position 4(5)
- introduction d'un radical alkyle en position 16
- introduction d'un radical alkylthio ou arylthio éventuellement substitué en position 7,
pour obtenir les produits de formule (Ib) : 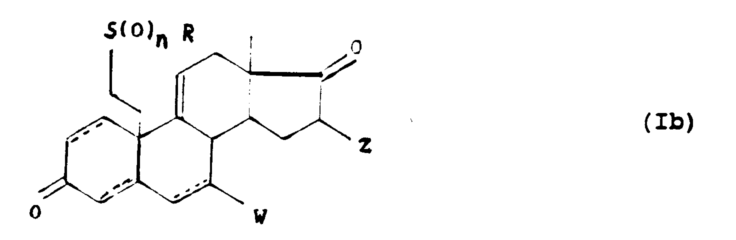
dans laquelle n représente les entiers 0 ou 1 et les traits pointillés en position 1(2), 4(5) et 6(7) indiquent la présence éventuelle d'une double liaison entre les carbones qui les portent et correspondant aux produits de formule (I) dans laquelle R a les significations indiquées ci-dessus, X et Y représentent chacun un atome d'oxygène et W, Z et n et les traits pointillés en positions 1(2), 4(5) et 6(7) ont les significations précédentes à l'exception des produits de formule (Ib) dans laquelle Z=H, W=H, n=O et dans laquelle les traits pointillés en position 1(2) et 6(7) ne representent pas une seconde liaison entre les carbones qui les portent et les traits pontillés en 4(5) représentent une seconde liaison entre les carbones qui les portent et si désiré soumet les produits de formules (Ia) et (Ib) à l'une ou plusieurs des réactions suivantes dans un ordre quelconque :
- introduction d'un radical 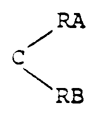 en position 3
- action de l'hydroxylamine ou d'un dérivé de formule H₂N-O-R₁,
- réduction de la fonction cétone en position 3 et/ou 17 et acylation éventuelle de la fonction hydroxy puis si désiré salification de la fonction estérifiée,
- transformation de la fonction cétone en position 17 en groupement 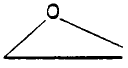 (CH₂)_{q} pour obtenir un produit de formule (Ic) : 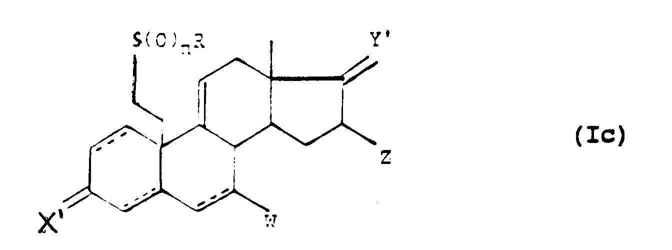 dans laquelle n, R, W, Z et les traits pointillés en positions 1(2), 4(5) et 6(7) ont la signification indiquée ci-dessus et X' et Y' représentent les valeurs indiquées ci-dessus pour X et Y étant entendu que l'un au moins de X ou Y ne représente pas un atome d'oxygène, et etant entendu que =X et ou =Y une représentent pas 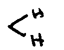
L'invention a également pour objet une variante du procédé décrit ci-dessus pour la préparation des produits de formule (I) telle que définie ci-dessus, caractérisée en ce que l'on soumet un produit de formule (IV) ou (I'a) : 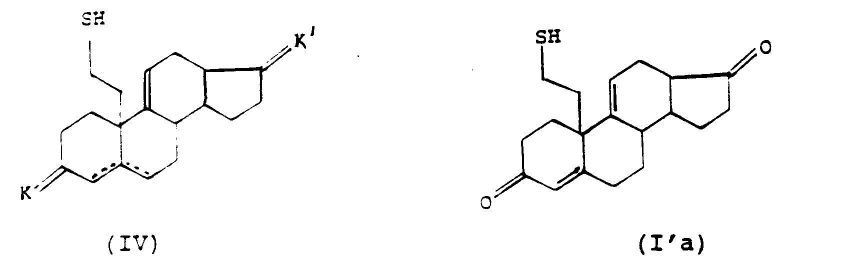
telles que définies précédemment, à l'action d'un halogénure de 2,4-dinitrophénylsulfényle pour obtenir un produit de formule (VII) : 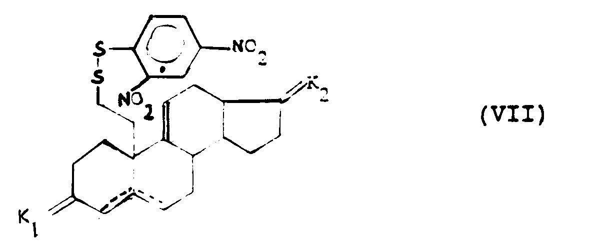
dans laquelle K₁ et K₂ représentent tous deux une fonction cétone ou tous deux une fonction cétone protégée et le trait pointillé représente une double liaison en 4(5) ou 5(6) lorsque K₁ représente une fonction cétone protégée ou en 4(5) lorsque K₁ représente une fonction cétone, produit de formule (VII) que
1) soit l'on soumet le cas échéant à un réactif de déprotection des fonctions cétone en position 3 et 17 pour obtenir un produit de formule (Id) : 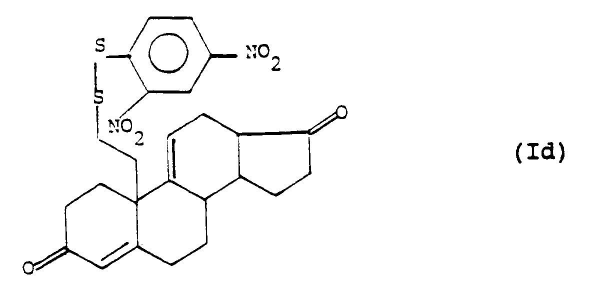
2) soit l'on soumet à l'action d'un réactif organomagnésien de formule R''-Mg-Hal dans laquelle Hal représente un atome d'halogène et R'' représente un radical alkyle, alcényle, alcynyle, ou CN
   ou à l'action d'un dérivé organolithien R''Li protégé puis à un agent de déprotection du radical R'' puis le cas échéant à une réaction de déprotection des fonctions cétone en position 3 et/ou 17 pour obtenir un produit de formule (Ie) : 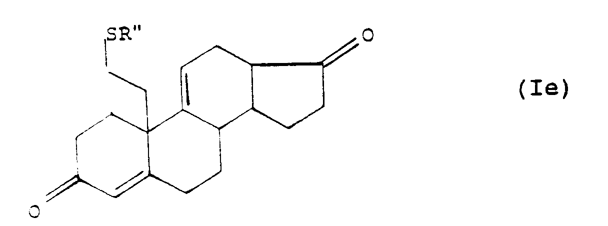 dans laquelle R'' a la signification indiquée ci-dessus,
3) soit l'on soumet à l'action d'un sel de formule R'''SA dans laquelle A a la signification indiquée précédemment et R''' représente un radical alkyle ayant au plus 6 atomes de carbone ou aryle éventuellement substitué ayant au plus 10 atomes de carbone, puis le cas échéant, à une réaction de déprotection des fonctions cétones en position 3 et/ou 17 pour obtenir un produit de formule (If) : 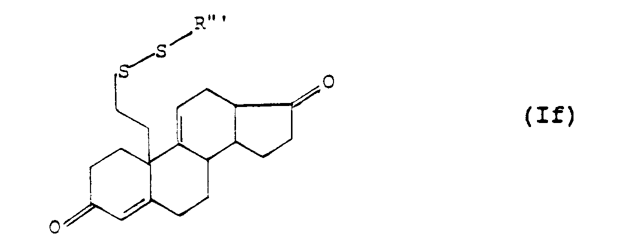 produits de formule (Id), (Ie), (If) que l'on soumet si désiré à une ou plusieurs des réactions indiquées ci-dessus, pour les produits de formule (Ia) ou (Ib), dans un ordre quelconque.

L'invention a également pour objet un procédé de préparation des produits de formule (I) dans laquelle =X et/ou =Y représente 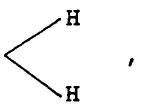
caractérisé en ce que l'on soumet un produit de formule (II) telle que définie ci-dessus à un agent de réduction des fonctions cétone protégées pour obtenir un produit de formule (III) dans laquelle =K et/ou =K' représente des atomes d'hydrogène et poursuit la synthèse comme indiqué ci-dessus.

Dans des conditions préférentielles d'exécution des procédés décrits ci-dessus, on opère comme suit :
- la réduction à laquelle on soumet les produits de formule (II) pour obtenir les produits de formule (III) est effectuée à l'aide d'un hydrure de préférence l'hydrure de lithium aluminium. On opère dans un solvant aprotique tel que le tétrahydrofuranne ou l'éther éthylique, par exemple à température ambiante. Dans le produit de formule (II), alk représente de préférence un radical éthyle,
- le réactif de transformation du radical hydroxyle en radical thiol est l'azodicarboxylate de diéthyle en présence de triphénylphosphine et d'acide thioacétique ; on obtient ainsi intermédiairement un produit comportant en position 10bêta un radical acétylthioéthyle qui est transformé en radical thiol par action de l'hydrazine. Les deux stades de la réaction sont effectués de préférence dans un solvant aprotique tel que le tétrahydrofuranne ou l'éther diéthylique,
- le dérivé reactif du radical R que l'on utilise de préférence est un halogénure tel que le chlorure ou le bromure, on peut également utiliser un pseudohalogénure tel qu'un mésylate ou un tosylate. On opère en présence d'une base forte telle qu'un alcoolate de métal alcalin par exemple le tert-butylate de potassium, ou un amidure par exemple l'amidure de diisopropyl lithium, ou l'hexaméthyldisilylazanate de Lithium ou de potassium. On peut opérer dans un solvant tel que le tétrahydrofuranne à basse température (par exemple entre 0 et -78°C),
- le dérivé réactif de l'acide sulfonique de formule BSO₃H dans Laquelle B représente de préférence un radical méthyle ou tolyle est de préférence un halogénure tel que le chlorure. On utilise de préférence le chlorure de mésyle. On opère de préférence en présence d'une base minérale ou organique de préférence la triéthylamine. On utilise un solvant de réaction tel que le chlorure de méthylène et la réaction est conduite à une température de l'ordre de 0°C,
- le sel de formule RSA ou R'''SA dans laquelle R et R''' ont la signification précédente, est de préférence un sel de métal alcalin tel que le sodium ou le lithium. L'action de ce sel est effectuée de préférence dans un solvant aprotique tel que Le tétrahydrofuranne, l'hexaméthylphosphoramide ou le diméthylformamide ; on peut travailler en présence d'un éther couronne spécifique du métal utilisé comme par exemple le 12-crown-4 pour le lithium, le 15-crown-5 pour le sodium, ou le 18-crown-6 pour le potassium. L'action du sel de formule RSA sur le produit de formule (VI) peut conduire à un déblocage partiel ou total de l'une ou des deux fonctions cétoniques protégées en fonctions 3 et 17. On peut notamment obtenir un produit de formule (V'') : 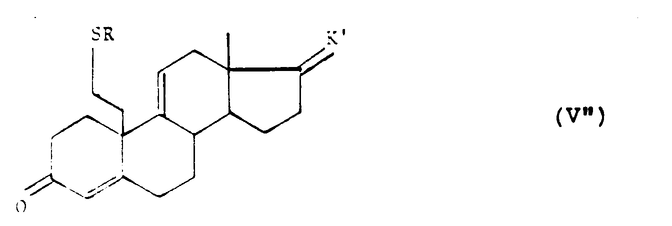 qui peut, bien entendu conduire aux produits de formule (Ia) dans les mêmes conditions que les produits de formule (V),
- l'hydrolyse des produits de formule (V) en produit de formule (Ia) est de préférence une hydrolyse acide par exemple à l'aide d'acide chlorhydrique concentré (2 à 6N) ou d'acide acétique dans un solvant tel que le méthanol, l'éthanol ou le tétrahydrofuranne,
- l'introduction éventuelle d'une double liaison en position 1(2) est effectuée par la DDQ de préférence dans un solvant tel que le dioxanne,
- la réduction sélective éventuelle de la double liaison delta 4 est effectuée au moyen de l'hydrogène en présence d'un catalyseur au palladium en présence de pyridine ou un métal alcalin, de préférence le lithium, dans l'ammoniac liquide,
- L'introduction éventuelle d'une double liaison en position 6(7) est effectuée à l'aide d'un orthoformiate tel que l'orthoformiate d'éthyle en présence par exemple d'acide paratoluène sulfonique dans un solvant usuel tel que l'éthanol, réaction suivie par celle du chloranile dans un solvant tel que l'acétone aqueuse,
- l'oxydation éventuelle de l'atome de soufre en sulfoxyde est effectuée par exemple par action d'un périodate tel que le périodate de sodium dans un solvant aqueux tel que le méthanol ou d'un péracide tel que l'acide métachloroperbenzoïque dans un solvant tel que le dichlorométhane,
- l'introduction éventuelle d'un radical 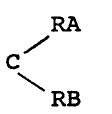 en position 3 est effectuée à l'aide d'une réaction dite de Wittig. On utilise alors un halogénure de triphénylalkyle ou haloalkyle phosphonium de préférence le bromure. On opère en présence d'une base forte telle que le butyl lithium ou le tert-butylate de potassium.

On peut également utiliser le dibromo difluoro méthane et l'hexaméthylphosphotriamide en présence de triglyme.
- l'action de l'hydroxylamine ou d'un de ses dérivés est effectuée de préférence dans un solvant tel que la pyridine, ou en présence d'une base minérale telle que le bicarbonate de sodium dans un solvant aqueux, tel que le méthanol,
- la réduction de la fonction cétone en position 3 et/ou 17 est effectuée à l'aide d'un hydrure tel que le borohydrure de sodium dans un solvant tel que le méthanol, ou l'hydrure d'aluminium lithium dans un solvant tel que le tétrahydrofuranne, soit à l'aide de nickel de Raney ou encore à l'aide d'un métal alcalin tel que le lithium dans l'ammoniac liquide,
- l'acylation éventuelle de la fonction hydroxyle en position 3 ou 17 est effectuée au moyen d'un dérivé d'acide tel qu'un anhydride ou un chlorure d'acide ; on opère alors de préférence en présence d'un capteur d'acide halohydrique tel que la pyridine,
- la salification éventuelle des fonctions ester est effectuée selon les conditions usuelles, par exemple au moyen d'un sel de sodium tel que le carbonate ou le carbonate acide de sodium,
- l'introduction d'un radical alkyle en position 16 est effectuée par formation d'un anion énolate en position 16 au moyen d'une base forte telle que le diisopropylamidure de lithium ou l'hexaméthyldisilylamidure de lithium dans un solvant tel que le tétrahydrofuranne puis traitement à l'aide d'un halogénure d'alkyle tel qu'un iodure,
- l'introduction d'un radical alkylthio ou arylthio en position 7 est effectuée au moyen d'un alkyl ou d'un aryl mercaptan dans un solvant tel que le tétrahydrofuranne ou le dioxanne en présence de quantités catalytiques de sodium,
- la réaction de cyclisation qui conduit au produit comportant un substituant 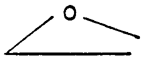 (CH₂)_{q} en position 17 est réalisée en utilisant un halogénure tel que par exemple l'iodure de triméthylsulfonium en présence de diméthylsulfoxyde et d'hydrure de sodium après blocage de la fonction cétone en position 3 sous forme d'éther d'énol, par exemple,
- la protection des fonctions cétone en position 3 ou 17 est effectuée selon les méthodes usuelles, sous forme de cétal cyclique en utilisant par exemple l'éthylène glycol en présence d'acide paratoluène sulfonique, sous forme de dithiocétal en utilisant par exemple le 1,2-éthane dithiol en présence d'acide paratoluènesulfonique ou d'éthérate de trifluorure de bore ou encore sous forme d'éther d'énol en utilisant par exemple un orthoformiate d'alkyle tel que l'orthoformiate d'éthyle en présence d'acide paratoluènesulfonique.

Selon le type de protection utilisée, il peut exister une double liaison entre les carbones 4 et 5 ou entre les carbones 5 et 6.
- La protection de la fonction 3-céto sous forme d'éther d'énol donne lieu à l'obtention d'un système de liaisons delta 3,5.

La déprotection peut être effectuée par les méthodes usuelles notamment par hydrolyse acide comme indiqué pour les produits de formule (V). Lorsque le groupement protecteur est un groupe thiocétal, on effectue de préférence une réduction au moyen de nickel de Raney ou au moyen d'un métal alcalin tel que le lithium dans l'ammoniac liquide.
- Le réactif organométallique que l'on fait agir sur le produit de formule (IV) ou (I'a) est de préférence un magnésien ou un lithien. Dans le cas où le radical à introduire comporte une fonction réactive, cette fonction peut être protégée par les méthodes usuelles, par exemple par un reste triméthylsilyle.

La réaction est conduite selon les méthodes usuelles, de préférence à une temperature comprise entre -100°C et 0°C.

La déprotection du radical R'' peut être réalisée par un fluorure de tétraalkylammonium.

L'observation selon laquelle environ 35 % des cancers du sein sont estrogéno-dépendants a conduit à rechercher les moyens de limiter la production d'estrogènes.

Après avoir utilisé des méthodes chirurgicales consistant à supprimer les sources d'estrogènes (ovaires) ou les sources de leurs précurseurs biosynthétiques, Les androgènes (glandes surrénales) on a cherché à développer des méthodes moins traumatisantes.
(ABUL-HAJJ Y.J.O. Steroid Biochem 13 (1980), 1935 ; BRODIE A.M.H. Cancer Res. 42, (1982), 3312).

A cet égard, l'inhibition spécifique du dernier stade enzymatique de l'aromatisation des androgènes 3-céto delta-4 en estrogènes phénoliques, semble le moyen le plus efficace et le moins perturbant. L'enzyme responsable de cette transformation est une mono-oxygénase connue comme étant un cytochrome P450 : l'AROMATASE (BRODIE A.M.H. J. Endocrinol. Invest. 2 (1979) 445) qui requiert de l'oxygène et la NADPH (Nicotinamide Adenine Dinucleotide phosphate réduit) pour effectuer l'aromatisation des androgènes en estrogènes.

Sur la base d'un autre mécanisme, d'autres auteurs (par exemple MARCOTTE et Al, Biochemistry 21, (1982) 2773, FLYNN et Al Biochem. Biophys. Res. Com. 103 (1981) 713) ont proposé des inhibiteurs suicides pour l'Aromatase. Des inhibiteurs compétitifs tels que l'Aminogluthétimide ont également été proposés dans le traitement des cancers métastasiques du sein. Ce produit s'est cependant révélé comme n'étant pas spécifique de l'Aromatase : il s'attaque en effet à d'autres processus enzymatiques que celui conduisant des androgènes aux estrogènes. Le document EP-A-149,499 décrit des inhibiteurs de l'aromatase qui sont différents de ceux de la demande en ce qu'il n'ont pas de double liaison en 9(11).

Les produits objets de la présente invention présentent au contraire une activité spécifique de l'aromatase (cytochrome P450 aromatase).

Cette propriété inhibitrice de l'aromatase rend les produits de la présente invention aptes à être utilisés dans les pathologies hormono-dépendantes, plus particulièrement estrogéno-dépendantes, comme par exemple :
- cancers du sein, de l'endomètre, de l'ovaire et du pancréas,
- gynécomasties,
- troubles bénins du sein,
- endométriose,
- affections polycystiques de l'ovaire,
- hyperplasie prostatique et plus généralement dans le traitement des hyperestrogènémies,
- certaines formes d'obésité.

L'invention a donc pour objet, à titre de médicaments, les produits de formule générale (I) telle que décrite ci-dessus.

L'invention a plus spécialement pour objet, à titre de médicaments, les produits de formule générale (I') et plus particulièrement ceux dans laquelle R' est choisi parmi les radicaux alkyle ayant de 1 à 4 atomes de carbone et X représente un atome d'oxygène.

L'invention a tout spécialement pour objet, à titre de médicaments :
- la 10bêta-(2-(méthylthio) éthyl) estra-4,9(11)-diène-3,17-dione,
- la 10bêta-[2-(éthylthio) éthyl] estra-4,9(11)-diène-3,17-dione,
- la 10bêta-[2-(méthylthio) éthyl] 17bêta-hydroxy estra-4,9(11)-diène-3-one,
- la 10bêta-[2-(éthénylthio) éthyl] estra-4,9(11)-diène-3,17-dione,
- la 10bêta-[2-(éthynylthio) éthyl] estra-4,9(11)-diène-3,17-dione,
- le butane dioate de 10bêta-[2-(méthylthio) éthyl] 3-oxo estra-4,9(11)-dièn 17bêta-yle et de sodium,
- la 10bêta-[2-(méthylthio) éthyl] estra-4,9(11)-diène-3bêta, 17bêta-diol,
- la 10bêta-[2-(méthylthio) éthyl] estra-4,9(11)-diène-3alpha, 17bêta-diol,
- la 10bêta-[2-(méthylthio) éthyl] estr-9(11)-ène-3,17-dione.

La posologie varie en fonction de l'affection à traiter et de la voie d'administration. Elle peut varier par exemple de 0,1 à 50 mg/kg de préférence 0,5 à 10 mg/kg et par jour chez l'adulte par voie orale.

Les nouveaux produits de formule (I) peuvent être employés pour préparer des compositions pharmaceutiques renfermant, à titre de principe actif, l'un au moins desdits produits.

Les produits de formule (I) sont utilisés par voie digestive, parentérale ou locale par exemple par voie transdermique. Ils peuvent être prescrits sous forme de comprimés simples ou dragéifiés, de gélules, de granulés, de suppositoires, d'ovules, de préparations injectables, de pommades, de crèmes, de gels, de patches, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a donc pour objet les compositions pharmaceutiques renfermant comme principe actif au moins un produit de formule (I) et plus particulièrement un produit de formule (I').

L'invention a également pour objet à titre de produits industriels nouveaux et notamment à titre de produits industriels nouveaux nécessaires pour la préparation des produits de formule (I), les produits de formules générales (III), (IV), (V) et (VI), telles que définies ci-dessus, caractérisé en ce que =K et/ou =K' representent les fonctions cétones protégées sous forme des radicaux éthylenedioxy, éthylenedithio ou de l'ethyl ether d'enol du type 3 ethoxy Δ 3(4), 5(6)-diène, et caractérisés en ce que B represente le reste d'un acide sulfonique choisi parmi un radical

Les produits de formule (II) utilisés au départ du procédé de l'invention sont des produits nouveaux.

Ces produits peuvent être prépares par le procédé suivant :
On effectue sur le produit de formule (A) : 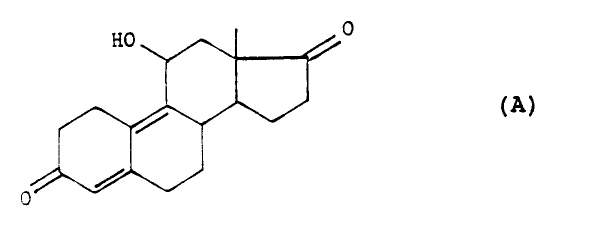
un réarrangement de Claisen pour obtenir un produit de formule (B) : 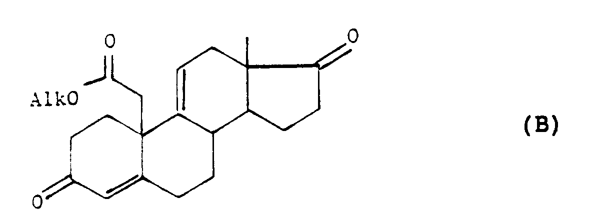
dans Laquelle Alk a la signification indiquée ci-dessus et soumet le produit de formule (B) à un ou deux réactifs de protection du radical cétone pour obtenir un produit de formule (II).

Dans un mode préférentiel d'exécution de ce procédé le réarrangement de Claisen est effectué à l'aide d'un orthoacétate d'alkyle, de préférence d'éthyle, en présence par exemple d'un acide organique tel que l'acide propionique et éventuellement dans un solvant tel que le xylène. La réaction s'effectue de préférence à température élevée.

Le réactif de protection des fonctions cétones est choisi parmi les alcools ou les diols. On utilise de préférence l'éthylèneglycol. La réaction s'effectue de préférence en présence d'un acide tel que l'acide paratoluène sulfonique. La réaction s'effectue en chauffant, par exemple au reflux d'un solvant tel que le dichloroéthane, ou en utilisant l'éthylèneglycol lui-même comme solvant.

Le produit de formule (A) est décrit par exemple dans le brevet américain USP 3.282.785.

En plus des produits décrits dans les exemples, qui illustrent l'invention sans toutefois la limiter, les produits suivants constituent des produits pouvant être obtenus dans le cadre de la présente invention, les substituants R, n, X et Y sont ceux indiqués dans la formule (I).

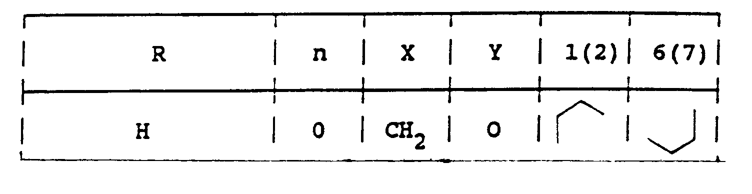

Par l'expression "12-crown-4" on entend le 1,4,7,10 tétraoxacyclododécane ; "15-crown-5" représente le 1,4,7,10,13 pentaoxacyclopentadécane ; "18-crown-6" représent le 1,4,7,10,13,16 hexaoxacyclooctadécane.

Les exemples suivants illustrent l'invention sans toutefois la limiter.

### EXEMPLE 1 : 10bêta-[2-(méthylthio) éthyl] estra-4,9(11)-diène-3,17-dione.

### Stade A : 3,17-bis(éthylènedioxy) 10bêta-(2-hydroxy éthyl) estra-5,9(11)-diène.

On refroidit à -78°C une suspension de 380 mg d'hydrure de Lithium aluminium dans 200 cm³ de tétrahydrofuranne anhydre. On ajoute 4,42 g de 3,17-bis(éthylènedioxy) androsta-5,9(11)-diène-19-carboxylate d'éthyle, dissous dans 50 cm³ de tétrahydrofuranne anhydre. On laisse 30 minutes à -78°C puis une heure à température ambiante. On détruit l'excès d'hydrure par addition au goutte à goutte d'environ 10 cm³ d'acétate d'éthyle. On ajoute, environ 50 cm³ de soude 2M. On extrait le mélange par deux fois 150 cm³ d'acétate d'éthyle, puis une fois 200 cm³ de dichlorométhane.

Les extraits organiques sont séchés puis concentrés pour fournir 3,98 g de produit brut utilisé tel quel dans les réactions suivantes.
RMN (300 MHz CDCl₃ + une goutte de C₅D₅N)
0,83 (s, 18 Me) ; 3,55 à 4,00 (m, -OCH₂CH₂- et CH₂OH) ; 5,53 (m, H6 et H11) ; 2,33 (S, OH) ; 1,30 à 2,70 (m autres protons).

### Stade B : 3,17-bis(éthylènedioxy) 10bêta-[2-(méthylsulfonyloxy) éthyl] estra-5,9(11)-diène.

On refroidit à 0°C une solution de 5,8 g de produit obtenu au stade A et 2,21 cm³ de triéthylamine dans 70 cm³ de dichlorométhane et ajoute goutte à goutte 1,23 cm³ de chlorure de mésyle. On agite ensuite le mélange durant 45 minutes à 0°C. On verse le mélange dans 100 cm³ d'une solution saturée de chlorure d'ammonium. La phase aqueuse est extraite au dichlorométhane. Les extraits organiques sont rassemblés, séchés sur sulfate de magnésium et concentrés pour fournir 6 g de mésylate brut, utilisé tel quel dans les étapes suivantes. RMN (300 MHz CDCl₃ + une goutte de C₅D₅N)
0,83 (s, 18 Me) ; 2,97 (s, SO₂Me) ; 3,85 à 4,3 (m, cétals et CH₂SO₂) ; 5,57 (m) et 5,60 (m) (H6 et H11) ; 1,2 à 2,6 (m, les autres protons).
IR (CHCL₃) 1338 et 1175 cm⁻¹ (SO2Me).

### Stade C : 10bêta-[2-(méthylthio) éthyl] estra-4,9(11)-diène-3,17-dione.

On agite durant 12 heures à température ambiante un mélange de 5,8 g de mésylate obtenu au stade B, 1,81 g de thiométhoxide de sodium et 0,26 cm³ d'éther couronne (15-crown-5) en solution dans 50 cm³ de diméthylformamide anhydre. Le mélange est versé dans environ 50 cm³ d'une solution saturée en chlorure d'ammonium et la phase aqueuse est extraite au dichlorométhane. Les extraits organiques sont rassemblés et séchés sur sulfate de magnésium. Après concentration, on obtient un produit brut que l'on utilise tel quel dans l'étape suivante.

On agite durant 1 heure à température ambiante le mélange brut obtenu précédemment, 20 cm³ d'acide chlorhydrique aqueux 6N et 200 cm³ d'éthanol à 99 %. Le mélange est versé dans de l'eau et la phase organique extraite au dichlorométhane. Les extraits organiques sont rassemblés, lavés successivement avec une solution d'acide chlorhydrique 1N, une solution saturée en bicarbonate de potassium et une solution saturée en chlorure de sodium. Après séchage et concentration, le mélange brut est chromatographié sur silice avec un mélange éluant acétate d'éthyle-cyclohexane (3-7), puis (1-1). On obtient 3,1 g de produit attendu (Rf = 0,45 acétate d'éthyle-cyclohexane (1-1)), que l'on recristallise dans l'éther diéthylique.
RMN (CDCl₃, 300 MHz 0,88 (s, 18 Me) ; 2,10 (s, SMe) ; 5,55 (m, H11) 5,81 (s, H4).
IR (CHCL₃) 1735 cm⁻¹ (17-céto), 1665 cm⁻¹, 1613 (énone), 1633 cm⁻¹ (C=C 9,11).

| Microanalyse : C₂₁H₂₈O₂S = 344,52 | | | | | | |
|---|---|---|---|---|---|---|
| Calculé | C% | 73,21 | H% | 8,19 | S% | 9,3 |
| Trouvé | | 73,0 | | 8,3 | | 9,2 |

### Préparation A :

Le 3,17-bis(éthylènedioxy) androsta-5,9(11)-diène-19-carboxylate d'éthyle utilisé au départ de l'exemple 1 a été préparé comme suit :

### Stade 1 : 3,17-dioxo androsta-4,9(11)-diène-19-carboxylate d'éthyle.

On amène à une température de 137°C un mélange de 500 mg de 11bêta-hydroxy androsta-4,9-diène-3,17-dione (produit décrit dans le brevet américain USP 3.282.785) 5 cm³ d'orthoacétate de triéthyle et 6,4 mg d'acide propionique. Après 4 heures de chauffage, la réaction est concentrée à sec et le mélange brut est chromatographié sur silice avec un mélange éluant d'acétate d'éthyle-hexane (1-1). On obtient 503 mg de produit attendu (Rf = 0,33).
RMN (CDCl₃, 250 MHz) 0,94 (s, 18 Me) ; 1,23 (t, COOCH₂CH₃) 3,94 à 4,29 (m, COOCH₂CH₃), 5,61 (m, H11), 5,84 (large s, H4). IR (CHCL₃) 1732 cm⁻¹ (17-cétone), 1662, 1612 (cétone conjuguée).

### Stade 2 : 3,17-bis(éthylènedioxy) androsta-5,9(11)-diène-19-carboxylate d'éthyle.

On ajoute 2 cm³ d'éthylèneglycol et 100 mg d'acide paratoluène sulfonique à un mélange de 503 mg de produit obtenu au stade 1 en solution dans 30 cm³ de dichloroéthane. On porte au reflux pendant 8 heures.

On ajoute 1 cm³ de triéthylamine et concentre le mélange. On chromatographie sur silice (éluant : acétate d'éthylehexane (3-7)). On obtient 450 mg de produit attendu (RF = 0,47) (acétate d'éthyle-hexane (1-1)).

### EXEMPLE 2 : 10bêta-[2-(éthylthio) éthyl] estra-4,9(11)-diène-3,17-dione.

On agite durant 12 heures à température ambiante un mélange de 1 g de mésylate obtenu au stade B de l'exemple 1, 0,374 g de thioéthoxide de sodium et 0,044 ml d'éther couronne (15-crown-5) en solution dans 20 cm³ de diméthylformamide anhydre. Le mélange est versé dans environ 50 cm³ d'une solution saturée en chlorure d'ammonium et la phase aqueuse est extraite au dichlorométhane. Les extraits organiques sont rassemblés et séchés sur sulfate de magnésium. Après concentration, on obtient un produit brut que l'on utilise tel quel dans l'étape suivante.

On agite durant 1 heure à température ambiante le mélange brut obtenu précédemment, 2 cm³ d'acide chlorhydrique aqueux 6N et 20 cm³ d'éthanol à 99 %. Le mélange est versé dans de l'eau et la phase organique extraite au dichlorométhane. Les extraits organiques sont rassemblés, lavés successivement avec une solution d'acide chlorhydrique 1N, une solution saturée en bicarbonate de potassium et une solution saturée en chlorure de sodium. Après séchage et concentration, le mélange brut est chromatographié sur silice avec un mélange éluant acétate d'éthyle-cyclohexane (3-7), suivi de (1-1). On obtient de cette façon 430 mg de produit attendu. (Fr = 0,45, acétate d'éthyle-cyclohexane (1-1)).
RMN (CDCl₃, 300 MHz) 0,88 (s, 18 Me) ; 1,24 (t, -SCH₂-CH₃) 5,55 (m, H11), 5,81 (s, H4).
IR (CHCL₃) 1735 cm⁻¹ (17-céto), 1665 CM⁻¹, 1613 (énone), 1633 cm⁻¹ (C=C 9,11).

| Microanalyse : C₂₂H₃₀O₂S, PM = 358,55 | | | | | | |
|---|---|---|---|---|---|---|
| Calculé | C% | 73,70 | N% | 8,43 | S% | 8,94 |
| Trouvé | | 73,8 | | 8,5 | | 9,0 |

### EXEMPLE 3 : 10bêta-[2-(acétylthio) éthyl] estra-4,9(11)-diène-3,17-dione.

### Stade A : 3,17-bis(éthylènedioxy) 10bêta-[2-(acétylthio) éthyl] estra-5,9(11)-diène et 17-(éthylènedioxy) 10bêta-[2-(acétylthio) éthyl] 5,9(11)-dièn-3-one.

On porte au reflux un mélange de 3,38 g de produit obtenu au stade B de l'exemple 1, 2,3 g de thioacétate de potassium et 190 mg d'éther couronne (18-crown-6) dans 60 cm³ de tétrahydrofuranne anhydre.

Au bout de 15 heures de reflux, on verse le mélange dans environ 100 cm³ d'une solution saturée de chlorure d'ammonium. La phase aqueuse est extraite au dichlorométhane. Les extraits organiques sont rassemblés, séchés sur sulfate de magnésium et concentrés sous pression réduite. Le résidu brut est chromatographié sur silice avec un mélange éluant d'acétate d'éthylecyclohexane (2-8). On obtient de cette façon une première fraction de 940 mg consistant en produit diprotégé (I) (Rf = 0,74, acétate d'éthyle-cyclohexane (1-1)). La fraction suivante est constituée du produit (II) monoprotégé en position 17 (500 mg, Rf = 0,44).
Analyse du produit diprotégé (I)
RMN (CDCl₃, 400 MHz) 0,86 (s, 18 Me) ; 2,30 (s, SAc) 3,80 à 4,08 (les cétals), 5,54 (H6 et H11).
IR (CHCL₃) 1685 cm⁻¹ (SC=O).
Analyse du produit monoprotégé (II)
RMN (CDCl₃, 400 MHz) 0,88 (s, 18 Me) ; 2,31 (s, SAc) 3,80 à 4,0 (les cétals), 5,56 (H11), 5,77 (H4).
IR (CHCL₃) 1683 cm⁻¹ (SC=O), 1665 cm⁻¹ (énone), 1614 cm⁻¹ (C=C).

### Stade B : 10bêta-[2-(acétylthio) éthyl] estra-4,9(11)-diène-3,17-dione.

On ajoute pendant 1 heure à température ambiante un mélange de 0,5 g de produit (II) monoprotégé, 2 cm³ d'acide chlorhydrique aqueux 6N et 12 cm³ d'éthanol à 99 %. Le mélange est versé dans environ 50 cm³ d'une solution saturée de chlorure d'ammonium et la phase aqueuse est extraite au dichlorométhane. Les extraits organiques sont rassemblés et séchés sur sulfate de magnésium. Le mélange brut est chromatographié sur silice avec un mélange éluant d'acétate d'éthylecyclohexane (1-1). On obtient 340 mg de produit attendu (Rf = 0,3, acétate d'éthyle-cyclohexane (1-1)) que l'on traite avec du charbon actif dans le dichlorométhane puis recristallise dans l'acétate d'éthyle.
RMN (CDCl₃, 300 MHz) 0,96 (s, 18 Me) ; 2,33 (s, SAc) 5,59 (m, H11), 5,81 (S, H4) 1,1 à 2,8 (les autres protons).
IR (CHCL₃) 1735 cm⁻¹, 1680 cm⁻¹ (SC=O), 1667 et 1615 cm⁻¹ (énone).

| Microanalyse : C₂₂H₂₈O₃S, S = 372,53 | | | | | | |
|---|---|---|---|---|---|---|
| Calculé | C% | 70,92 | H% | 7,57 | S% | 8,60 |
| Trouvé | | 71,2 | | 7,6 | | 8,9 |

Le 3,17-bis(éthylènedioxy) 10bêta-(2-(acétylthio) éthyl) estra-5,9(11)-diène obtenu au stade A ci-dessus peut également être obtenu comme suit :
On ajoute 1,65 cm³ d'acide thioacétique à un mélange de 8,6 g de produit obtenu à l'exemple 1, stade A, 10,8 g de triphénylphosphine et 3,6 cm³ de diéthylazodicarboxylate en solution dans 100 cm³ de tétrahydrofuranne anhydre et agite 2 heures 30. On rajoute 0,9 cm³ d'azodicarboxylate de diéthyle et 0,42 cm³ d'acide thioacétique. Après 20 minutes d'agitation, on évapore le solvant et chromatographie le produit brut obtenu (éluant : cyclohexane-acétate d'éthyle (8-2)). On obtient ainsi 5,88 g de produit identique à celui obtenu au stade A ci-dessus.

### EXEMPLE 4 : 10bêta-[2-(méthylsulfinyl) éthyl] estra-4,9(11)-diène-3,17-dione.

On agite pendant 30 minutes à température ambiante, un mélange de 500 mg de produit obtenu à l'exemple 1 dans 3,7 cm³ de méthanol et 373 mg de périodate de sodium solubilisé dans 3,7 cm³ d'eau.

On extrait au chlorure de méthylène, sèche sur sulfate de magnésium et amène à sec. On chromatographie le produit sur silice (éluant : acétate d'éthyle-méthanol (7-3)). On obtient 276 mg de produit attendu. 50-50 de diastéréoisomères.
RMN (CDCl₃, 300 MHz) 0,86 et 0,92 (18 Me) ; 2,56 et 2,58 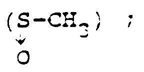
5,59 (m, H11), 5,86 (S, H4). IR (CHCL₃) 1736 cm⁻¹ (17-céto), 1668 cm⁻¹ (cétone conjuguée) 1630 cm⁻¹, 1615 cm⁻¹ (C=C), 1046 cm⁻¹ (S -> O).

### Préparation 1 : 10bêta[[2-(difluorométhyl) thio] éthyl] estra-4,9(11)-diène-3,17-dione.

### Stade A : 3,17-bis(éthylènedioxy) 10bêta-(2-mercaptoéthyl) estra-5,9(11)-diène.

On ajoute à -20°C, 2,3 cm³ d'hydrazine à 64 % dans une solution de 3 g de 3,17-bis(éthylènedioxy) 10bêta-[2-(acétylthio) éthyl] estra-5,9(11)-diène (produit diprotégé (I) obtenu à l'exemple 3 stade A) dans 150 cm³ de tétrahydrofuranne et laisse à -30°C pendant 72 heures.

On verse le milieu réactionnel dans l'eau, extrait au chlorure de méthylène, sèche sur sulfate de magnésium et évapore le chlorure de méthylène.

On obtient 2,65 g de produit attendu Rf= 0,36 (cyclohexane-acétate d'éthyle (1-1)). Le produit est utilisé tel quel pour l'étape suivante.
RMN (CDCl₃, 300 MHz) 0,82 (s, 18 Me) ; 3,8 à 4,0 (cétals) ; 5,49 et 5,53 (m, H6 et H11)
IR (CHCL₃) 1635 cm⁻¹ et 1672 cm⁻¹ (C=C).

### Stade B : 3,17-bis(éthylènedioxy) 10bêta-[2-(difluorométhylthio) éthyl] estra-5,9(11)-diène.

On refroidit à 0°C, 1 g de produit obtenu au stade A en solution dans 20 cm³ de tétrahydrofuranne anhydre et ajoute en une seule fois 322 mg de tert-butoxyde de potassium. On agite 30 minutes à cette température et on fait passer un courant rapide de fréon 22 (ClCHF₂). Le mélange réactionnel est versé sur environ 100 cm³ d'une solution saturée en chlorure d'ammonium et la phase organique est extraite au dichlorométhane. Les extraits organiques sont rassemblés, séchés sur sulfate de magnésium et concentrés. On chromatographie sur silice (éluant : acétate d'éthyle-cyclohexane (2-8)) et obtient 500 mg de produit attendu.
RMN (CDCl₃, 300 MHz) 0,82 (s, 18 Me) ; 3,86 à 4,0 (les cétals), 5,54 (les éthylèniques), 6,76 (t, SCHF₂, J=56,5 Hz).
IR (CHCL₃) Faibles absorptions C=C) 1615 et 1638 cm⁻¹, C-O-C probable.

### Stade C : 10bêta-[[2-(difluorométhyl) thio] éthyl] estra-4,9(11)-diène-3,17-dione.

On agite 10 minutes à 0°C, puis 30 minutes à température ambiante un mélange de 90 mg de produit obtenu au stade B, 1 cm³ d'acide chlorhydrique aqueux 3N et 5 cm³ d'éthanol à 99 %.

Le mélange est versé dans une solution saturée en bicarbonate de sodium et la phase organique est extraite au dichlorométhane. Les extraits organiques sont rassemblés, séchés sur sulfate de magnésium et concentrés sous pression réduite. On obtient 61,7 mg de produit brut que l'on chromatographie sur silice avec un mélange éluant d'acétate d'éthyle-cyclohexane (1-1). On obtient 46,5 mg de produit attendu.
RMN (CDCl₃, 300 MHz) 0,88 (s, 18 Me) ; 5,82 (s, H4) ; 6,8 (t, CHF₂, J=55,5 Hz).
IR (CHCL₃) 1736 cm⁻¹, (17-céto) ; 1666 et 1614 et 867 cm⁻¹ (énone) ; absorptions fortes 1060, 1030 cm⁻¹ (CHF₂).

### EXEMPLE 5 : 10bêta-[2-(méthylthio) éthyl] estra-1,4,9(11)-triène-3,17-dione.

On porte au reflux 1 heure puis agite une nuit à temperature ambiante un mélange de 500 mg de produit obtenu à l'exemple 1, 830 mg de dichlorodicyanoquinone et 20 cm³ de dioxanne. Le mélange est concentré et chromatographié sur silice avec un mélange éluant d'acétate d'éthyle-cyclohexane (3-7), suivi de (1-1). On obtient le produit attendu (Rf = 0,43, acétate d'éthyle-cyclohexane (1-1)) que l'on chromatographie de nouveau avec un mélange éluant d'acétate d'éthyle-cyclohexane (2-8). On obtient 280 mg de produit pur.
RMN (CDCl₃, 300 MHz) 0,95 (s, 18 Me) ; 2,07 (s, SMe) ; 5,62 (m, H11) ; 6,19 (s, H4) ; 6,39 (dd, H2) ; 7,10 (d, H1).
IR (CHCL₃), 1736 cm⁻¹ (17-céto) ; 1664 cm⁻¹, 1625 cm⁻¹, 1607 cm⁻¹, 891 cm⁻¹ (delta 1,4 3-one).

### EXEMPLE 6 : 10bêta-[2-(méthylthio) éthyl] estra-4,6,9(11)-triène-3,17-dione.

On agite 1 h 30 à température ambiante un mélange de 1,15 g de produit obtenu à l'exemple 1, 4,17 cm³ d'orthoformiate d'éthyle et 30 mg d'acide paratoluène sulfonique.

On ajoute ensuite 5 cm³ de triéthylamine et verse le milieu dans environ 150 cm³ d'une solution saturée en carbonate acide de sodium. On extrait au chlorure de méthylène, regroupe les phases organiques et sèche sur sulfate de magnésium. On ajoute 2 cm³ de triéthylamine puis évapore le solvant.

On obtient 1,2 g de produit que l'on mélange avec 1,5 g de chloranile dans 56 cm³ d'un mélange acétone-eau (95-5). Après 1 h 30 d'agitation à température ambiante, on verse le milieu dans 150 cm³ d'une solution saturée en thiosulfate de sodium et ajoute 150 cm³ d'une solution saturée de carbonate acide de sodium. On agite de nouveau 1 h 30 à température ambiante, extrait la phase aqueuse au chlorure de méthylène puis a l'acétate d'éthyle. Les extraits organiques sont séchés sur sulfate de magnésium puis amenés à sec.

Le produit brut obtenu est chromatographié sous pression. On obtient 920 mg de produit attendu.
RMN (CDCl₃, 300 MHz) 0,96 (s, 18 Me) ; 2,10 (s, SMe) ; 2,92 (d large, H8) ; 5,59 (n, H11) ; 5,76 (s, H4) ; 6,22 (AB, H6 et H7).
IR (CHCL₃) 1739 cm⁻¹, (17-céto) ; 1659 cm⁻¹, 1624 cm⁻¹, 1583 cm⁻¹, 877 cm⁻¹ (delta 4,6 3-one).

| Analyse : C₂₁H₂₆O₂S, S = 342,5 | | | | | | |
|---|---|---|---|---|---|---|
| Calculé | C% | 73,64 | H% | 7,65 | S% | 9,36 |
| Trouve | | 73,5 | | 7,7 | | 9,2 |

### EXEMPLE 7 : 10bêta-[2-(méthylthio) éthyl] 3-méthylène estra-4,9(11)-dièn-17-one.

On agite pendant 20 minutes à température ambiante un mélange de 2,07 g de bromure de triphényl méthylphosphonium dans 10 cm³ d'éther et 3,75 cm³ de butyl lithium et ajoute 0,5 g de produit obtenu à l'exemple 1 en solution dans 10 cm³ d'éther. On agite le milieu réactionnel pendant 30 minutes à température ambiante et verse dans 30 cm³ d'eau. On extrait à l'acétate d'éthyle, réunit les extraits organiques et les sèche sur sulfate de magnésium et évapore le solvant.

Le produit brut obtenu est purifié par chromatographie sous pression et on obtient 190 mg de produit attendu (Rf = 0,37, cyclohexane-acétate d'éthyle (8-2)).
RMN (CDCl₃, 300 MHz) 0,86 (s, 18 Me) ; 2,08 (s, SMe) ; 4,68 (s) et 4,73 (s) (CH₂=C) ; 5,47 (n, H11) ; 5,9 (s, H4).

### EXEMPLE 8 : 10bêta-(2-(acétylthio) éthyl] 3-méthylène estra-4,9(11)-dièn-17-one.

On ajoute goutte à goutte et sous agitation 0,34 cm³ de n-butyl lithium dans une suspension de 195,6 mg de bromure de triphényl méthyl phosphonium dans 10 cm³ d'éther éthylique anhydre puis agite 20 minutes à température ambiante. On introduit ensuite lentement une solution de 51 mg de produit obtenu à l'exemple 3 dans 5 cm³ d'éther anhydre. On agite le mélange 12 heures à temperature ambiante puis une heure au reflux d'éther. Le mélange brut est versé dans 30 cm³ environ d'eau glacée et la phase aqueuse est extraite à l'acétate d'éthyle. Les extraits organiques sont rassemblés et concentres. Une chromatographie sur silice avec un mélange éluant d'acétate d'éthyle-cyclohexane (2-8) fournit 7,3 mg de produit attendu (Rf = 0,6).
Analyse
RMN (CDCl₃, 300 MHz) 0,93 (s, 18 Me) ; 2,31 (s, CH₃-C=) ; 4,68 et 4,72 (CH₂-C) ; 5,51 m, H11) ; 5,89 (H4).

### EXEMPLE 9 : 10bêta-[2-(méthyldithio) éthyl] estra-4,9(11)-diène-3,17-dione.

### Stade A : 3,17-bis(éthylènedioxy) 10bêta-[2-(méthyldithio) éthyl] estra-5,9(11)-diène.

On ajoute à -71°C à un mélange de 750 mg de 3,17-bis(éthylènedioxy) 10bêta-(2-mercapto éthyl) estra-5,9(11)-diène dans environ 50 cm³ de tétrahydrofuranne, un mélange de 202 mg de tert-butylate de potassium dans 0,17 cm³ de méthyl méthane-thiol-sulfonate.

On agite en laissant remonter la température à température ambiante pendant 2 heures puis à -30°C pendant 48 heures. On ajoute 20 cm³ d'une solution aqueuse de chlorure d'ammonium, extrait au chlorure de méthylène. On sèche les extraits organiques sur sulfate de magnésium, évapore le solvant et obtient 372,6 mg de produit attendu. Rf = 0,14 (même éluant que ci-dessus).
RMN (CDCl₃, 400 MHz) 0,85 (s, 18 Me) ; 2,36 (s, CH₃-S-) ; 3,82 à 4 (cétals) ; 5,5 et 5,53 (H6 et H11) ; 2,52 (t, S-CH₂-CH₂).

### Stade B : 10bêta-[2-(méthyldithio) éthyl] estra-4,9(11)-diène-3,17-dione.

On agite une heure à température ambiante un mélange de 100 mg de produit obtenu au stade A dans 4 cm³ d'éthanol et 1,2 cm³ d'acide chlorhydrique 6N. On ajoute 5 cm³ d'une solution saturée de carbonate acide de sodium, extrait au chlorure de méthylène. Les extraits organiques sont regroupés, séchés sur sulfate de magnésium.

On évapore le solvant et chromatographie le produit obtenu (éluant : cyclohexane-acétate d'éthyle (1-1)). On obtient 35 mg d'huile jaune. Rf = 0,6 (même éluant que ci-dessus).
RMN (CDCl₃, 300 MHz) 0,90 (18 Me) ; 2,38 (S-S Me) ; 5,54 (H11) ; 5,82 (H4).
IR (CHCL₃) 1735 cm⁻¹, (17-céto) ; 1655 cm⁻¹ (cétone conjuguée), 1663-1614 (C=C).

### Exemple 10 : 10bêta-[2-(thiocyanato) éthyl] estra-4,9(11)-dièn-3,17-dione.

En opérant comme indiqué dans les exemples précédents, on a préparé le produit attendu.
IR (CHCl₃) 1736 cm⁻¹ (17-céto), 1671 cm⁻¹ (cétone conjuguée), 1633 et 1616 cm⁻¹ (C=C), 2158 cm⁻¹ (S-C≡N).
RMN (CDCl₃, 250 MHz) 0,88 (s, 18 Me) ; 5,57 (m, H11) ; 5,85 (d, Hu).

### Exemple 11 : 10bêta-[2-(méthylthio) éthyl] 17bêta-hydroxy estra-4,9(11)-dièn-3-one.

On refroidit à -78°C une solution comprenant 2,38 g de 10bêta-[2-(méthylthio) éthyl] estra-4,9(11)-dièn 3,17-dione préparé comme à l'exemple 1, et 600 cm³ d'un mélange de méthanol-dichlorométhane (1-1). On ajoute 2,34 g de borohydrure de sodium, agite 6 heures à -78°C, ajoute 150 cm³ d'acétone et laisse revenir à température ambiante, concentre partiellement et renouvelle le traitement au borohydrure de sodium et agite encore pendant 6 heures. Après avoir ajouté de nouveau 150 cm³ d'acétone, on concentre sous pression réduite, acidifie à l'aide d'acide chlorhydrique 2N et extrait au dichlorométhane. On sèche les phases organiques, les concentre et chromatographie sur silice (éluant : acétate d'éthylecyclohexane 3-7). On obtient 229 mg de produit attendu.
RMN (300 MHz CDCl₃) 0,75 (s, 18 Me) ; 2,11 (s, S-Me) ; 5,53 (m, H11) ; 5,79 (d, H4) ; 8 à 2,7 (m, les autres protons)
IR (CHCl₃) 3615 cm⁻¹ (OH), 1665 et 1612 cm⁻¹ (énone).

### Exemple 12 : 10bêta-[2-[(2,4-dinitrophényl) dithio] éthyl] estra-4,9(11)-dièn 3,17-dione.

### Stade A : 3,17-bis (éthylènedioxy) 10bêta-[2-[(2,4-dinitrophényl) dithio] éthyl] estra-4,9(11)-diène.

On mélange sous atmosphère inerte 416 mg de 3,17-bis (éthylènedioxy) 10bêta-[2-difluorométhylthio) éthyl] estra-5,9(11)-diène préparé selon Préparation 1, stades A et B, et 81 microlitres de pyridine dans 15 cm³ de chlorure de méthylène, puis ajoute 235 mg de chlorure de 2,4-dinitrobenzène sulfényle en solution dans 5 cm³ de chlorure de méthylène. On agite 30 minutes, verse le mélange réactionnel dans 100 cm³ d'eau et 100 cm³ d'acétate d'éthyle. On sépare la phase organique, sèche, évapore le solvant sous pression réduite et chromatographie le résidu sur silice (éluant : cyclohexane-chlorure de méthylène-acétate d'éthyle). On obtient 500 mg de produit attendu.
IR 1594, 1526 cm⁻¹ (F) (aromatique et nitro)
RMN (CDCl₃, 300 MHz) 0,6 (s, 18 Me) ; 5,46 (d), 5,53 (d) (les éthyléniques) ; 3,8 à 4 (les cétals) ; 8,47 (s) 2H et 9,11 (s) 1H (phényl).

### Stade B : 10bêta-[2-((2,4-dinitrophényl) dithio] éthyl] estra-4,9(11)-dièn 3,17-dione.

On mélange sous atmosphère inerte 1,25 g de produit obtenu comme indiqué au stade A, 10 cm³ d'acide chlorhydrique 6N dans 150 cm³ de méthanol. Apres dissolution, on évapore les solvants, reprend par un mélange eau-bicarbonate de sodiumacétate d'éthyle, décante la phase organique, la sèche, élimine les solvants sous pression réduite et chromatographie le résidu sur silice (éluant : chlorure de méthylène-éther 100-0 puis 90-10. On recueille 1 g de produit attendu.
IR 1595, (S) 1528 cm⁻¹ (F) (aromatique et nitro) ; 1737 et 1668 cm⁻¹ (carbonyle) ; 1638 et 1613 cm⁻¹ (éthyléniques).
RMN (CDCl₃, 300 MHz) 0,63 (s, 18 Me) ; 5,51 (m), 582 (s) (éthyléniques) ; 8,48 (s) 2H et 9,10 (s) 2H (phényl) ; 1,1 à 2,8 (les autres protons).

### Exemple 13 : 10bêta-[2-(éthénylthio) éthyl] estra-4,9(11)-dièn 3,17-dione.

On refroidit à -78°C sous atmosphère inerte 265 mg de produit obtenu à l'exemple 12 dans 50 cm³ de tétrahydrofuranne puis ajoute lentement 1 cm³ de bromure de vinyl magnésium. On agite 1 heure à -78°C puis laisse revenir à température ambiante. On maitient 30 minutes sous agitation, ajoute une solution aqueuse saturée en chlorure d'ammonium, élimine le tétrahydrofuranne sous pression réduite puis extrait à l'acétate d'éthyle. On sépare la phase organique, la sèche, élimine le solvant sous pression réduite et cristallise le résidu dans l'éther isopropylique. On recueille 40 mg de produit attendu.
IR 1736 cm³ (carbonyle) ; 1736 cm³ (delta 4-3-one) ; 1585 et 956 cm³ (thiovinyle)/
RMN (CDCl₃, 300 MHz) 0,88 (s, 18 Me) ; 5,59 (m) et 5,82 (d) (les éthyléniques) ; 5,12 (d=16,5 Hz) et 5,23 (d, J=10) (CH₂-CH-S) ; 6,29 (dd J=10 et 16,5) (S-CH-CH₂) ; 1,1 à 2,7 (les autres protons).

### Exemple 14 : Dioxime de 10bêta-[2-(méthylthio) éthyl] estra-4,9(11)-dièn 3,17-dione.

On mélange à température ambiante 200 mg de produit obtenu au stade C de l'exemple 1 dans 10 cm³ de chlorure de methylène et 40 mg de chlorhydrate d'hydroxylamine en présence d'un peu de sulfate de magnésium. On ajoute goutte à goutte 0,08 cm³ de triéthylamine, agite 30 minutes, chauffe au reflux pendant 1 heure et demie, puis maintient sous agitation à température ambiante pendant 48 heures. On filtre sur célite et purifie par chromatographie le milieu réactionnel (éluant : cyclohexane-acétate d'éthyle 8-2). On obtient 145 mg de produit brut que l'on cristallise dans l'éther et obtient 46,5 mg de produit attendu.
IR (CHCl₃) 3885 cm⁻¹ (OH, oxime) ; 1635 cm⁻¹ (C=C) ; 1620 cm⁻¹ (C≡N).
RMN (CDCl₃, 300 MHz) 0,91 (s, 18 Me) ; 2,08 (s, SMe) ; 5,50 (m, H11) ; 5,86 (s, H4 (E)) ; 7,54 et 7,78 (les OH).

### Préparation 2 : 10bêta-[2-(phénylthio) éthyl] estra-4,9(11)-dièn 3,17-dione.

### Stade A : 3,17-bis (éthylènedioxy) 10bêta-[2-(phénylthio) éthyl] estra-4,9(11)-diène.

On prépare du thiophényl oxyde de sodium en mélangeant à température ambiante 0,510 mg de sodium dans 40 cm³ de tétrahydrofuranne et 5,6 cm³ de thiophénol ; après 12 heures d'agitation, le précipité est filtré et lavé au pentane puis séché sous pression réduite. On mélange 300 mg de mésylate préparé au stade B de l'exemple 1 dans 20 cm³ de diméthylformamide, ajoute 165 mg de thiophényloxyde de sodium et agite 24 heures à température ambiante. On ajoute 5 gouttes de 15-Crown S, agite 12 heures puis élimine le solvant sous pression réduite. Après chromatographie sur silice (éluant : hexane-acétate d'éthyle 8-2), on obtient 281 mg de produit attendu.
IR (CHCl₃) 1584, 1481 cm⁻¹ (S-C₆H₅)
RMN (CDCl₃, 300 MHz) 0,80 (s, 18 Me) ; 3,85 à 3,98 (cétals) ; 5,57 (m, H6 et H11) ; 7,14 à 7,33 (S-C₆H₅) ; 1,25 à 2,90 (les autres protons).

### Stabe B : 10bêta-[2-(phénylthio) éthyl] estra-4,9(11)-dièn 3,17-dione.

On agite une heure à température ambiante un mélange de 260 mg de produit obtenu au stade A dans 20 cm³ d'éthanol et 2 cm³ d'acide chlorhydrique 6N. On hydrolyse par une solution saturée de carbonate acide de sodium, extrait au chlorure de méthylène. Les extraits organiques sont regroupés, séchés sur sulfate de magnésium. On évapore le solvant et chromatographie le produit obtenu (éluant : cyclohexane-acétate d'éthyle 8-2). On obtient 121 mg de produit attendu que l'on cristallise dans l'éther. Rf = 0,55 (éluant : cyclohexane-acétate d'éthyle 1-1).
RMN (CDCl₃, 300 MHz) 0,84 (18 Me) ; 2,38 (S-S Me) ; 5,57 (H11) ; 5,78 (H4) ; 7,20 à 7,40 (phényl).
IR (CHCl₃) 1736 cm⁻¹ (17-céto) ; 1665 cm⁻¹ (cétone conjuguée); 1630, 1612, 1577, 1477 cm⁻¹ (C=C + aromatique).

### Exemple 15 : 10bêta-[2-(2-propénylthio) éthyl] estra-4,9(11)-dièn 3,17-dione.

### Stade A : 3,17-bis (éthylènedioxy) 10bêta-[2-(2-propénylthio) éthyl] estra-4,9(11)-diène.

On opère comme à la Préparation 2 Stade A à partir de 120 mg de sodium et 0,36 cm³ d'allyl mercaptan, 1 g de mésylate obtenu à l'exemple 1 stade B, 20 cm³ de diméthylformamide et 0,44 cm³ de 15-Crown-S. On obtient 282 mg de produit attendu.
RMN (CDCl₃, 300 MHz) 0,83 (s, 18 Me) ; 3,11 (m, S-CH₂-C=) ; 3,8 à 4,0 (les cétals) ; 5,05 à 5,20 (m, CH₂=) ; 5,51 (m, H6 et H11) ; 5,77 (m, CH=).

### Stade B : 10bêta-[2-(2-propénylthio) éthyl] estra-4,9(11)-dièn 3,17-dione.

On opère comme au stade B de la Préparation 2 à partir de 300 mg de produit obtenu comme au stade A ci-dessus et 5 cm³ d'acide chlorhydrique 6N. On obtient 230 mg de produit brut que l'on cristallise dans le pentane.
IR (CHCl₃) 1736 cm⁻¹ (17-céto) ; 1665 cm⁻¹ (cétone conjuguée); 1634, 1613 cm⁻¹ (C=C) ; 921 cm⁻¹ (CH=CH₂).
RMN (CDCl₃, 300 MHz) 0,88 (s, 18 Me) ; 3,13 (dd, J=1 et 7, S-CH₂-C=) ; 5,05 à 5,11 (CH₂=CH) ; 5,54 (m, H11) ; 5,73 (m, CH₂=CH) ; 5,81 (H4).

Le 3,17-bis (éthylènedioxy) 10bêta-[2-(2-propénylthio) éthyl] estra-4,9(11)-diène obtenu au stade A peut également être obtenu comme suit :
On dissout 400 mg de produit obtenu comme au stade A de la Préparation 1 dans 10 cm³ de tétrahydrofuranne, ajoute 129 mg de terbutylate de potassium, agite 40 minutes à température ambiante, ajoute 0,16 cm³ de bromure d'allyle et maintient encore 30 minutes sous agitation. On ajoute ensuite une solution aqueuse saturée en chlorure d'ammonium, extrait au chlorure de méthylène, sèche et évapore le solvant. Après chromatographie (éluant : cyclohexane-acétate d'éthyle 8-2), on obtient 325 mg de produit attendu.
RMN (CDCl₃, 300 MHz) 0,82 (s, 18 Me) ; 3,11 (d, S-CH₂-C=) ; 3,8 à 4 (les cétals) ; 5,0( à 5,15 (CH₂=CH) ; 5,50 (m, H6 et H11) ; 5,76 (m, CH₂=CH).

### Exemple 16 : 10bêta-[2-(éthynylthio) éthyl] estra-4,9(11)-dièn 3,17-dione.

### Stade A : 10bêta-[2-(triméthylsilylpropynylthio) éthyl] estra-4,9(11)-dièn 3,17-dione.

On refroidit à -78°C sous atmosphère inerte 0,14 cm³ de triméthylsilylacétylène en solution dans 60 cm³ de tétrahydrofuranne et ajoute 0,91 cm³ de n-butyllithium. On agite 15 minutes, puis verse à -78°C sur 528 mg de disulfure (préparé au stade B de l'exemple 12) en solution dans 20 cm³ de tétrahydrofuranne refroidi à la même température. On agite 1 heure, reprend dans une solution aqueuse saturée en chlorure d'ammonium, élimine le solvant sous pression réduite et extrait à l'acétate d'éthyle. On sèche la phase organique, évapore le solvant et chromatographie le résidu sur silice (éluant : chlorure de méthylène-éther 90-10). On recueille 180 mg de produit attendu.
IR 2092 cm⁻¹ (acétylénique) ; 1636 et 1616 cm⁻¹ (éthylénique); 1736 et 1666 cm⁻¹ (carbonyle).

### Stade B : 10bêta-[2-(éthynylthio) éthyl] estra-4,9(11)-dièn 3,17-dione.

On mélange à temperature ambiante sous atmosphère inerte 110 mg de produit obtenu ci-dessus, 0,3 cm³ de fluorure de tétrabutylammonium dans 20 cm³ de tétrahydrofuranne. On agite 15 minutes, reprend le milieu réactionnel dans l'eau, extrait à l'acétate d'éthyle, sépare la phase organique, la sèche et évapore les solvants. Après chromatographie du résidu sur silice (éluant : cyclohexane-acétate d'éthyle 7-3), on obtient 60 mg de produit attendu. F = 174°C.
IR 3301 cm⁻¹ (acétylénique) ; 1667, 1614 cm⁻¹ (énone) ; 1736 cm⁻¹ (cétone).
RMN (CDCl₃, 300 MHz) 0,88 (s, 18 Me) ; 5,57 (t), 5,83 (d) (les diènes) ; 2,80 (s, l'acétylénique).

### Exemple 17 : 10bêta-[2-(phényldithio) éthyl] estra-4,9(11)-dièn 3,17-dione.

### Stade A : 3,17-bis (éthylènedioxy) 10bêta-[2-(phényldithio) éthyl] estra-4,9(11)-diène.

On mélange à température ambiante sous atmosphère inerte 617 mg de produit cotenu comme à l'exemple 12 et 400 mg de thiophénate de sodium dans 80 cm³ de tétrahydrofuranne. On agite 1 heure, reprend avec une solution aqueuse saturée en chlorure d'ammonium, élimine le tétrahydrofuranne sous pression réduite, extrait à l'acétate d'éthyle. La phase organique est concentrée, on chromatographie le résidu sur silice (éluant : cyclohexane-acétate d'éthyle 75-25) et obtient 270 mg de produit attendu.
IR 1581, 1477 cm⁻¹ (thiophénol)
RMN (CDCl₃) 300 MHz) 0,7 (s, 18 Me) ; 3,8 à 4 (les cétals) ; 5,45, 5,50 (les diènes) ; 7,15 à 7,35 (3H), 7,50 (2H) (phényle).

### Stade B : 10bêta-[2-(phényldithio) éthyl] estra-4,9(11)-dièn 3,17-dione.

On opère Comme au stade B de la Préparation 2 en utilisant 270 mg du produit obtenu au stade A, 5 cm³ d'acide chlorhydrique 6N et 50 cm³ de méthanol et obtient 105 mg de produit attendu.
IR 1630, 1612, 1580 et 1477 cm⁻¹ (aromatique et éthylénique); 1736 et 1665 cm⁻¹ (carbonyle).
RMN (CDCl₃, 300 MHz) 0,69 (s, 18 Me) ; 5,48 (m) ; 5,78 (d) (les diènes) ; 7,23 (m), 7,32 (tm), 7,50 (dm) (le phényle).

### Exemple 18 : 10bêta-[2-(méthylthio) éthyl] estra-4,9(11)-dièn 3bêta,17bêta-diol et isomère 3alpha,17bêta-diol.

On opère à partir de 1 g de produit obtenu à l'exemple 1 stade C, 110 mg d'hydrure d'aluminium lithium dans 25 cm³ de tétrahydrofuranne et en maintenant l'agitation pendant 24 heures. On extrait au chlorure de méthylène, élimine le solvant sous pression réduite et chromatographie le résidu (éluant : cyclohexane-acétate d'éthyle 1-1). On obtient 401 mg d'isomère 3bêta,17bêta que l'on cristallise dans l'éther et 281 mg d'isomère 3alpha,17bêta.
**Isomère 3bêta,17bêta-diol.**
IR (CHCl₃) Absence de cétone ; 3611 cm⁻¹ (OH fort)
RMN (CDCl₃) 0,72 (s, 18 Me) ; 2,09 (s, SMe) ; 3,73 (t, H17) ; 4,18 (m, H3) ; 5,39 (m, H4et H11)
**Isomère 3alpha,17bêta-diol.**
IR (CHCl₃) Absence de cétone ; 3608 cm⁻¹ (OH fort)
RMN (CDCl₃) 0,72 (s, 18 Me) ; 3,73 (t, H17alpha) ; 4,11 (m, H3) ; 5,48 (m, H4et H11)

### Exemple 19 : Butanedioate de 10bêta-[2-(méthylthio) éthyl] 3-oxo estra-4,9(11)-dièn 17bêta-yle et de sodium.

On dissout à température ambiante sous atmosphère inerte 704 mg de produit obtenu à l'exemple 11 dans 10 cm³ de chloroforme, ajoute 814 mg d'acide succinique puis goutte à goutte 2,8 cm³ de triéthylamine et enfin 198 mg de diméthylaminopyridine. On agite 12 heures à température ambiante, ajoute 100 cm³ d'acide chlorhydrique 2N, extrait au chloroforme, sèche et élimine les solvants. On obtient 280 mg de produit sous forme d'acide que l'on dissout dans 1 cm³ d'éthanol, ajoute 1 cm³ d'eau puis 34,5 mg de bicarbonate de sodium. On agite 2 heures à temperature ambiante, élimine le solvant sous pression reduite, reprend dans 10 cm³ d'eau, filtre sur célite et lyophilise. On recueille 199 mg de produit attendu.
IR (CHCl₃) 1720 cm⁻¹ (17-céto) ; 1666 cm⁻¹ (cétone conjuguée); 1611 et 1587 cm⁻¹ (C=C et COO).
RMN (CDCl₃, 300 MHz) 75 (s, 18 Me) ; 2,09 (s, SMe) ; 4,62 (t, H17) ; 5,46 (H11) ; 5,79 (H4).

### Exemple 20 : 10bêta-[2-(méthylthio) éthyl] 16alpha-méthyl estra-4,9(11)-dièn 3,17-dione.

On dissout sous atmosphère inerte 1,1 g de produit obtenu à l'exemple 1 stade C dans 30 cm³ de tétrahydrofuranne, refroidit à - 78°C, ajoute 3,52 cm³ d'une solution d'hexaméthyldisilylamidure de lithium 1M dans le tétrahydrofuranne. On agite 20 minutes, ajoute 0,2 cm³ d'iodure de méthyle et laisse revenir à température ambiante. Après chromatographie sur silice (éluant : acétate d'éthyle-cyclohexane 3-7, on obtient 590 mg de produit attendu.
RMN (CDCl₃, 300 MHz) 0,91 (s, 18 Me) ; 1,12 (d, CH₃-CH) ; 2,1 (s, SMe) ; 5,54 (H11) ; 5,81 (H4).

### Exemple 21 : 7alpha-[(4-aminophényl) thio] 10-bêta-[2-méthylthio) éthyl] estra-4,9(11)-dièn 3,17-dione.

On ajoute 1,2 g de 4-aminothiophénol et 23 mg de sodium à 470 mg de produit obtenu comme à l'exemple 6 dans 10 cm³ de tétrahydrofuranne et maintient le mélange sous agitation pendant 12 heures à température ambiante. On verse le milieu réactionnel dans 30 cm³ d'une solution aqueuse saturée en chlorure d'ammonium et extrait au chlorure de méthylène. On sépare la phase organique, la sèche et élimine les solvants sous pression réduite. Après chromatographie sur silice (éluant : acétate d'éthyle-cyclohexanne 1-1, on obtient 174 mg de produit attendu.
IR (CHCl₃) 3500, 3400 cm⁻¹ (=C-NH₂) ; 1664 cm⁻¹ (énone) ; 1620, 1598, 1495 cm⁻¹ (C=C aromatiques NH₂ déf)
RMN (CDCl₃, 300 MHz) 0,88 (s, 18 Me) ; 2,5 (s, SMe) ; 5,68 (m, H11) ; 5,77 (s, H4) ; 6,6 à 6,8 et 7,0 à 7,4 (m, aromatiques); 3,91, 3,65 et 3,46 (m, H7 + autre absorption) ; 0,8 à 3 (m, les autres protons).

### Exemple 22 : 10bêta-[2-(méthyldithio) éthyl] estra-4,9(11)-dièn 3,17-dione.

### Stade A : 3,17-bis (éthylènedioxy) 10bêta-[2-(méthyldithio) éthyl] estra-4,9(11)-diène.

On opère comme indiqué au stade A de l'exemple 17 en mélangeant à 0°C 625 mg de produit obtenu à l'exemple 12 et 300 mg de thiométhoxyde de sodium dans 50 cm³ de tétrahydrofuranne. On obtient 227 mg de produit attendu. Spectre IR identique à celui obtenu à l'exemple 9A.

### Stade B : 10bêta-[2-(méthyldithio) éthyl] estra-4,9(11)-dièn 3,17-dione.

On opère comme au stade B de la Préparation 2 en utilisant 100 mg de produit obtenu au stade A dans 4 cm³ d'éthanol et 1,2 cm³ d'acide chlorhydrique 6N. On obtient 35 mg de produit attendu identique à celui de l'exemple 9.
IR 1735 cm⁻¹ (17-céto) ; 1655 cm⁻¹ (cétone conjuguée) ; 1633, 1614 cm⁻¹ (C=C)
RMN (CDCl₃, 250 MHz) 0,90 (s, 18 Me) ; 2,38 (s, SMe) : 5,56 (m, H11) ; 5,83 (d, H4).

### Exemple 23 : 10bêta-[2-(méthylthio) éthyl] estr-9(11)-èn 3,17-dione.

On introduit à -78°C 136 mg de lithium en poudre dans 70 cm³ d'ammoniac liquide, puis ajoute 500 mg de produit obtenu comme à l'exemple 1 et agite 3 heures et demie à -78°C. On évapore l'ammoniac à température ambiante, ajoute une solution aqueuse saturée en chlorure d'ammonium, extrait à l'acétate d'éthyle, chromatographie sous pression (éluant : cyclohexane-acétate d'éthyle 8-2 puis 6-4) et récupère 138,5 mg de produit attendu.
IR (CHCl₃) Absence de delta4-3-one ; 1734 cm⁻¹ (cétone en 17); 1710 cm⁻¹ (cétone en 3)
RMN (CDCl₃, 300 MHz) 0,86 (s, 18 Me) ; 2,13 (s, SMe) ; 5,42 (m, H11)

### Exemple 24 : 10bêta-[2-(méthylthio) éthyl] 5bêta-estr-9(11)-èn 3,17-dione.

On hydrogène pendant 2 heures sous une pression de 1200 mbars, 400 mg de produit obtenu a l'exemple 1 dans 8 cm³ de méthyléthyl pyridine en présence de 500 mg de sulfate de baryum a 10% de palladium. On filtre, ajoute au filtrat 200 cm³ de chlorure de methylène et 100 cm³ d'acide chlorhydrique concentre dans 200 cm³ d'eau. On sépare la phase organique, la sèche, élimine les solvants sous pression réduite à 25°C, chromatographie le résidu sur silice (éluant : acétate d'éthyle-cyclohexane 3-7) et obtient 300 mg de produit brut que l'on cristallise dans l'éther et recueille 130 mg de produit attendu. F = 70°C.
RMN (CDCl₃, 300 MHz) 0,86 (s, 18 Me) ; 2,10 (s, SMe) ; 5,60 (m, H11)

### Exemple 25 : Propanoate de 10bêta-[2-(méthylthio) éthyl] 3-oxo estra-4,9(11)-dièn 17bêta-yle.

On refroidit vers 0°C sous atmosphère inerte 500 mg de produit obtenu à l'exemple 11 dans 5 cm³ de dichlorométhane, ajoute 500 microlitres de triéthylamine puis goutte à goutte 250 microlitres de chlorure de propionyle. On agite 1 heure, ajoute 50 cm³ d'une solution aqueuse saturée en chlorure d'ammonium, extrait au dichlorométhane, élimine les solvants sous pression réduite, chromatographie le résidu sur silice (éluant : acétate d'éthyle-cyclohexane 3-7) et récupère 480 mg de produit attendu.
RMN (CDCl₃, 300 MHz) 0,79 (s, 18 Me) : 1,15 et 2,34 (t et q COCH₂CH₃) ; 2,10 (s, SMe) ; 4,72 (t, H17) ; 5,49 (m, H11) ; 5,79 (d, H4)

### Exemple 26 : Hexanoate de 10bêta-[2-(méthylthio) éthyl] 3-oxo estra-4,9(11)-dièn 17bêta-yle.

On opère comme à l'exemple 25 en utilisant 350 mg de produit obtenu à l'exemple 11, 154 microlitres de triéthylamine et 154 microlitres de chlorure de caproyle. On obtient 280 mg de produit attendu.
RMN (CDCl₃, 300 MHz) 0,79 (s, 18 Me) ; 0,91 (masque CH₃-CH₂) ; 134 (m, CH₂) ; 2,10 (s, SMe) ; 2,31 (t, COCH₂) ; 4,70 (m, H17); 5,49 (m, H11) ; 5,79 (d, H4)

### Exemple 27 : 10bêta-[2-(2-propynylthio) éthyl] estra-4,9(11)-dièn 3,17-dione.

### Stade A : 3,17-bis (éthylènedioxy) 10bêta-[2-(2-propynylthio) éthyl] estra-4,9(11)-diène.

On refroidit à -78°C 800 mg de produit obtenu à la préparation 1 stade A dans 4 cm³ de tétrahydrofuranne en présence de 0,39 cm³ de tétraméthyléthylènediamine. On ajoute 2,25 cm³ de butyllithium, agite 30 minutes, ajoute 0,2 cm³ de bromure de propynyle, agite 1 heure et demie à -78°C, ajoute une solution aqueuse saturée en chlorure d'ammonium, extrait au chlorure de méthylène, sèche et élimine les solvants sous pression réduite. On purifie par chromatogrphie sur silice (éluant : cyclohexane-acétate d'éthyle 8-2) et récupère 462 mg de produit attendu.
IR (CHCl₃) 3307, 2105 cm⁻¹ (-C≡CH)

### Stade B : 10bêta-[2-(2-propynylthio) éthyl] estra-4,9(11)-dièn 3,17-dione.

On opère comme au stade B de la Préparation 2 en utilisant 460 mg de produit obtenu au stade A dans 10 cm³ d'éthanol et 2 cm³ d'acide chlorhydrique 6N. On obtient 265 mg de produit attendu.
IR (CHCl₃) 3307 cm⁻¹ (-C≡CH) ; 1735 cm⁻¹ (17-céto) ; 1665 cm⁻¹ (cétone conjuguée) ; 1630, 1608 cm⁻¹ (C=C)
RMN (CDCl₃, 300 MHz) 0,90 (s, 18 Me) ; 2,23 (t, J=2,5 C=CH) ; 3,26 (m, S-CH₂-C=) ; 5,58 (m, H11) ; 5,82 (d, H4)

### Préparation 3 : 17-méthylène 10bêta-[2-(méthylthio) éthyl] estra-4,9(11)-dièn 3-one.

### Stade A : 3-éthoxy 17-méthylène 10bêta-[2-(méthylthio) éthyl] estra-3,5,9(11)-triène.

On ajoute sous atmosphère inerte 6,5 cm³ d'orthoformiate d'éthyle à 3 g de produit obtenu à l'exemple 1 dans 9 cm³ d'éthanol. On chauffe à 70°C, ajoute 4 mg d'acide paratoluènesulfonique en solution dans 1 cm³ d'éthanol, agite quelques minutes puis ajoute 500 microlitres de triéthylamine. On refroidit, ajoute 100 cm³ de chlorure de méthylène, lave une solution aqueuse saturée en chlorure d'ammonium, sèche la phase organique, élimine le solvant sous pression réduite et recueille après chromatographie sur silice (éluant : cyclohexane-acétate d'éthyle 9-1 à 1% de triéthylamine) 2,6 g de produit attendu utilise tel duel pour le stade suivant.

### Stade B : 17-méthylène 10bêta-[2-(méthylthio) éthyl] estra-4,9(11)-dièn 3-one.

On mélange sous atmosphère inerte 1,5 g de bromure de triphénylmethylphosphonium dans 50 cm³ de dioxane et ajoute 227 mg de méthoxyde de sodium. On agite 1 heure et demie à temperature ambiante, ajoute lentement 500 mg de produit obtenu au stade A dissous dans 2 cm³ de dioxane et chauffe 3 heures à 70°C. On ajoute 100 cm³ d'une solution aqueuse saturée en bicarbonate de sodium, extrait au chlorure de méthylène, sèche et élimine les solvants sous pression réduite. On ajoute 50 cm³ d'acide chlorhydrique 2N et 50 cm³ d'éthanol, agite 1 heure, neutralise avec de la soude, extrait au chlorure de méthylène, sèche et élimine le solvant sous pression réduite et recueille 830 mg de produit brut que l'on purifie par chromatographie sur silice (éluant : acétate d'éthylecyclohexane 3-7). On obtient 320 mg de produit attendu.
IR peu ou pas de cétone en 17 ; 1663 cm⁻¹ (cétone conjuguée) ; 1632, 1612 cm⁻¹ (C=C)
RMN (CDCl₃, 300 MHz) 0,80 (s, 18 Me) ; 2,10 (s, SMe) ; 4,70 et 4,74 (m et m C=CH₂) ; 5,56 (m, H11) ; 5,80 (s, H4)

### Exemple 28 : 10bêta-[2-(méthylthio) éthyl] spiro (estra-4,6,9-(11)-trièn 17bêta,2alpha-oxiran) 3-one.

On chauffe à 50°C pendant 1 heure 480 mg d'hydrure de sodium dans 10 cm³ de diméthylsulfoxyde. On ajoute 10 cm³ de tétrahydrofuranne, refroidit à 0°C, ajoute 2,04 g d'iodure de triméthylsulfonium et agite 20 minutes. On ajoute alors 300 mg de produit obtenu au stade A de la préparation 3, dissous dans 5 cm³ de tétrahydrofuranne. On laisse revenir à température ambiante et maintient sous agitation pendant 3 heures. On ajoute une solution saturée en bicarbonate de sodium, extrait au chlorure de méthylène, sèche, élimine les solvants sous pression réduite à 25°C, chromatographie sur silice (éluant : acétate d'éthyle-cyclohexane 3-7) et obtient 380 mg d'intermédiaire comportant une fonction époxyde en 17 et une fonction 3-céto protégée sous forme d'éther d'énol. On agite 10 minutes 450 mg de produit obtenu ci-dessus dans 7 cm³ d'acétone et 350 microlitres d'eau. On ajoute 640 mg de chloranile, agite 16 heures à température ambiante, ajoute 100 cm³ de soude 2N, agite 30 minutes, filtre, extrait au chlorure de méthylène, sèche, élimine les solvants sous pression réduite à 25°C et récupère 410 mg de résidu. Après chromatographie (éluant : acétate d'éthyle-cyclohexane 3-7 puis 2-8), on obtient 110 mg de produit attendu.
IR (CHCl₃) 1660, 1622, 1583, 877 cm⁻¹ (delta 4,6,3-one) ; 855, 917 cm⁻¹ (époxyde probable)
RMN (CDCl₃, 300 MHz) 0,95 (s, 18 Me) ; 2,10 (s, SMe) ; 2,69 et 2,93 (d, J=5 CH₂O époxyde) ; 2,83 (large H8) ; 5,83 (m, H11) ; 5,74 (s, H4) ; 6,18 (H6 et H11)

### Exemples : Compositions pharmaceutiques.

On a préparé des comprimés répondant à la formule suivante :

| | |
|---|---|
| - Produit de l'exemple 1 | 50 mg |
| - Excipient q.s. pour un comprimé terminé à | 120 mg |

(Détail de l'excipient : talc, amidon, stéarate de magnésium).

### Etude pharmacologique des produits de l'invention.

### A) Etude in vitro

Inhibition dépendante de la concentration (mesure de la CI₅₀ = concentration de l'inhibiteur nécessaire pour réduire l'activité enzymatique de 50 %).

On utilise des placentas humains qui une heure au plus après l'accouchement sont lavés, perfusés par du sérum physiologique (5 litres) via la veine ombilicale puis congelés à -40°C.
1) Obtention des microsomes placentaires
Les placentas sont décongelés à 4°C puis homogénéisés (1:3) dans un tampon phosphate 10 mM, pH = 7,0 ; contenant 100 millimoles de chlorure de potassium (KC1), 10 millimoles de dithiothreitol (DTT), 10 millimoles d' acide éthylènediaminetétraacétique (EDTA), 40 millimoles de nicotinamide et 250 millimoles de sucrose.
Les homogénats sont ensuite soumis à différentes phases de centrifugation jusqu'à l'obtention du surnageant "9000 g" (correspondant au cytosol et au réticulum endoplasmique).
Ce surnageant est alors soumis à une étape d'ultracentrifugation (1 heure 30 minutes, 105000 g) pour obtenir le culot microsomal.
Les microsomes sont alors resuspendus dans un tampon phosphate 50 millimoles, pH = 7,4, contenant 100 millimoles KC1, 1 millimole EDTA, 1 millimole DTT et du glycérol (10 %).
La suspension microsomale est ensuite aliquotée et les fractions congelées à la température de l'azote liquide.
La concentration en protéines de la suspension microsomale est déterminée par la méthode de BRADFORD (BRADFORD M., Anal. Biochem., 72, (1976) 248).
2) Mesure de la CI₅₀ de chaque inhibiteur
A 960 microlitres de tampon phosphate (50 millimoles, pH = 7,2), 2,5 millimoles glucose-6-phosphate, et contenant 0,16 unité internationale de glucose-6-phosphate déshydrogénase (G-6-PDH), on ajoute dans l'ordre :
1° - 10 microlitres d'inhibiteur, solubilisé dans le diméthylsulfoxyde (DMSO), pour donner des concentrations finales allant de 10⁻⁵M à 10⁻¹⁰M.
2° - 10 microlitres de substrat. Le substrat est de l'Androstènedione 500 nM solubilisée dans l'éthanol et contenant de la 1bêta-2bêta-(³H)-Androstènedione à dilution isotopique connue (∼ 200.000 désintégrations par minute).
3° - 10 microlitres de suspension microsomale, équivalant à 25 microgrammes de protéines par test.
La réaction enzymatique est alors très rapidement initiée par un ajout de 10 microlitres de nicotinamide adénine dinucléotide phosphate réduit (NADPH) solubilisé dans l'eau.
Après agitation, chaque test est incubé à 37°C pendant 10 minutes. La réaction est ensuite stoppée par un ajout de chloroforme (4 ml).
Après agitation vigoureuse des tubes, ceux-ci sont décantés et centrifugés à 4°C pendant 10 minutes à la vitesse de 3000 r.p.m. (rotations par minutes) soit 600 X g.
Après centrifugation, et pour chaque tube, 100 microlitres de surnageant sont prélevés et mis à compter en présence de liquide scintillant.
Cette méthode est dérivée des procédures décrites par REED et Coll. (J.Biol. Chem., 251, (1976), 1625) et THOMPSON et Coll. (J.Biol. Chem., 249, (1974), 5364).
L'activité enzymatique (aromatase), est proportionnelle au pourcentage de tritium relargué sous forme d'eau tritiée (³H₂O) au cours de la réaction.
L'inhibition obtenue pour chaque concentration de chaque produit inhibiteur de l'invention est calculée comme un pourcentage des contrôles (100 % arbitraire, obtenus en absence de tout inhibiteur).
La CI₅₀ est égale à la concentration d'inhibiteur nécessaire pour diminuer de 50 % l'activité enzymatique.
Les valeurs des CI₅₀ obtenues pour les produits inhibiteurs de l'invention sont les suivantes :

| Produit de l'exemple | CI₅₀ |
|---|---|
| 1 | 2x10⁻⁸M. |
| 2 | 8,7x10⁻⁷M. |
| 11 | 5x10⁻⁸M |
| 16 | 3,3x10⁻⁷M |
| 18 (3bêta OH) | 1,65x10⁻⁷M |
| 23 | 5,5x10⁻⁹M |

### B) Etude in vivo

### I - TAUX D'ESTRADIOL CIRCULANT CHEZ LES RATS INDUITS AU P.M.S.G. (Pregnant Mare Serum Gonadotrophine).

### 1) Principe

On induit des rats femelles pesant 150 g par une injection de P.M.S.G. (100 Ui/rat/sc). Après 90 heures, on traite les animaux avec le produit à tester (sc ou po) et on prélève du sang avant et après traitement pour mesurer le taux d'estradiol sérique.

### - Protocole :

- JO,-90 h: : injection de 100 Ui de PMSG/rat.
- JO, 0 h: : prélèvement de sang au plexus choroïde puis traitement par le produit à tester.
- JO, +2 h: : prélèvement de sang
- JO, +6 h: : sacrifice des animaux et recueil du sang.

### - Dosage :

Après coagulation et centrifugation du sang, on récupère le sérum sur lequel on dose l'oestradiol (E2) au moyen d'un kit de dosage RIA (Baxter ER155).

### 2) Résultats :

Les résultats sont exprimés en pourcentage de variation par rapport au taux d'oestradiol au temps 0 (chaque animal est son propre témoin). Le test statistique utilisé est le Mann-Whitney U-test (* : p<0,05 ; ** : p<0,01).

| Produit de l'exemple | VOIE S.C. | | | VOIE P.O. | | |
|---|---|---|---|---|---|---|
| | DOSE mg/kg | + 2h | + 6h | DOSE mg/kg | + 2h | + 6h |
| 1 | 1 | -55 ** | -68 ** | 5 | -51 ** | -51 ** |
| 11 | 1 | -56 ** | -60 ** | 5 | -70 ** | -53 ** |
| 18 3alpha OH | | | | 5 | -53 ** | -53 ** |
| 18 3bêta OH | | | | 5 | -63 ** | -64 ** |
| 19 | 1 | -56 ** | -20 ** | 5 | -58 ** | -45 ** |
| 23 | | | | 5 | -69 ** | -56 ** |

### II - TAUX D'OESTRADIOL CHEZ LE SINGE MACAQUE CYNOMOLGUS

### PROTOCOLE

### 1) ANIMAUX UTILISES :

Pour ce test, on utilise des singes macaques cynomolgus femelles pesant de 3 à 5 kg.

### 2) TRAITEMENT :

Les animaux sont traités entre les jours 8 et 10 du cycle menstruel à raison de 1 mg/kg du produit de l'exemple 1 par voie S.C. en injection unique.

### 3) EXPERIENCE :

| | | |
|---|---|---|
| Temps | O h | Prélèvement sanguin, puis traitement. |
| | 30 mn | Prélèvement sanguin. |
| | 1 h 30 | Prélèvement sanguin. |
| | 2 h | Prélèvement sanguin. |

### 4) DOSAGES :

On dose l'oestradiol dans le sang qui est récupéré sur EDTA, et les dosages sont effectués sur le plasma (RIA).

### RESULTATS :

L'injection du produit de l'exemple 1 à la dose de 1 mg/kg par voie sous cutanée provoque une baisse d'oestradiol d'environ 42 à 75% après 2 h.

## Revendications (Revendications pour l'(les) Etat(s) contractant(s) suivant(s): DE, GB, FR, IT, NL, SE, LI, CH, BE, AT, LU, DK)

1. Les produits de formule générale (I) : 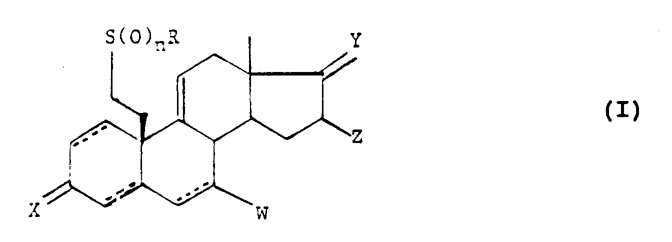 dans laquelle R représente :
un atome d'hydrogène,
un radical alkyle, alcényle, alcynyle, alkylthio ayant au plus 6 atomes de carbone ou arylthio éventuellement substitué ayant au plus 10 atomes de carbone,
un radical, acyle ayant au plus 12 atomes de carbone ou un radical CN,
- X représente un atome d'oxygène, un radical N-O-R₁ dans lequel R₁ représente un atome d'hydrogène ou un radical alkyle ayant de 1 à 6 atomes de carbone ou X représente 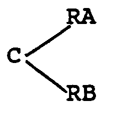 dans lequel RA et RB sont identiques et représentent un atome d'hydrogène, ou =X représente 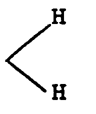 ou un reste 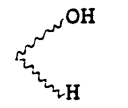 dans lequel le radical OH peut se trouver en position alpha ou bêta, le radical hydroxyle étant éventuellement acylé,
- Y représente un atome d'oxygène, un radical N-O-R₁ dans lequel R₁ a la définition indiquée ci-dessus, dessus, ou =Y représente 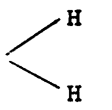 ou un reste 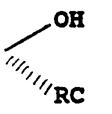 dans lequel RC représente un atome d'hydrogène le radical hydroxyle étant éventuellement acylé ou =Y représente un groupe 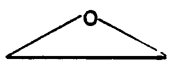 (CH₂)q dans lequel q représente un nombre de 1 à 3,
- W représente un atome d'hydrogène, un radical alkylthio ou arylthio éventuellement substitué renfermant jusqu'à 10 atomes de carbone,
- Z représente un atome d'hydrogène ou un radical alkyle renfermant jusqu'à 10 atomes de carbone,
- n représente un entier de 0 à 1 et les traits pointillés en positions 1(2), 4(5) et 6(7) indiquent la présence éventuelle d'une seconde liaison entre les carbones qui les portent, ainsi que leurs sels,
étant entendu que lorsque n est égal à 1, R représente un radical methyle, X et Y représentent chacun un atome d'oxygène, W et Z représentent chacun un atome d'hydrogène, le trait en pointillé en position 4(5) représente une seconde liaison entre les carbones qui les portent et les traits pointillés en position 1(2) et 6(7) ne représentent pas une seconde liaison entre les carbones qui les portent.

2. Les produits de formule générale (I) telle que définie dans la revendication 1 répondant à la formule générale (I') : 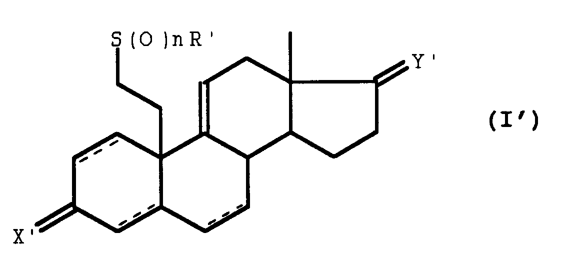 dans laquelle R' représente :
un radical alkyle, alcényle, alcynyle, ou alkylthio ayant au plus 4 atomes de carbone,
un radical CN,
un radical acyle ayant au plus 4 atomes de carbone,
- X' représente un atome d'oxygène, un radical CH₂, un radical N-O-R'₁ dans lequel R'₁ représente un atome d'hydrogène ou un radical alkyle ayant de 1 à 4 atomes de carbone, ou X' représente un reste 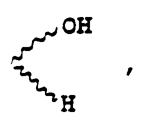
- Y' représente un atome d'oxygène ou =Y' représente un reste 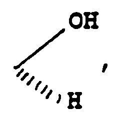 le radical hydroxy étant éventuellement acylé sous forme de radical alcanoyloxy ou d'un radical 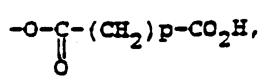 dans lequel p représente un nombre de 2 à 5.
Les traits pointillés en positions 1(2), 4(5) et 6(7) indiquent la présence éventuelle d'une seconde liaison entre les carbones qui les portent, ainsi que leurs sels.

3. Les produits de formule (I') toile que définie à la revendication 2 dans laquelle R' est choisi parmi les radicaux alkyle, alcényle, ou alcynyle ayant de 1 à 4 atomes de carbone et X' représente un atome d'oxygène ou un reste

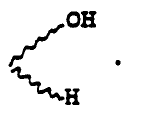

4. Les produits de formule générale (I'), telle que définie à l'une quelconque des revendications 2 ou 3 et répondant aux formules :
- la 10bêta-[2-(méthylthio) éthyl] estra-4,9(11)-diène-3,17-dione,
- la 10bêta-[2-(éthylthio) éthyl] estra-4,9(11)-diène-3,17-dione,
- la 10bêta-[2-(methylthio) éthyl] 17bêta-hydroxy estra-4,9(11)-diène-3-one.
- la 10bêta-[2-(éthénylthio) éthyl] estra-4,9(11)-diène-3,17-dione,
- la 10bêta-[2-(éthynylthio) éthyl] estra-4,9(11)-diène-3,17-dione,
- le butane dioate de 10bêta-[2-(méthylthio) éthyl] 3-oxo estra-4,9(11)-dièn 17bêta-yle et de sodium.
- le 10bêta-[2-(méthylthio) éthyl] estra-4,9(11)-diène-3bêta, 17bêta-diol,
- la 10bêta-[2-(méthylthio) éthyl] estra-4,9(11)-diène-3alpha, 17bêta-diol,
- la 10bêta-[2-(méthylthio) éthyl] estr-9(11)-ène-3,17-dione.

5. Pocédé de préparation des produits de formule générale (I) telle que définie à la revendication 1, caractérisé en ce que l'on soumet à une réduction un produit de formule (II) : 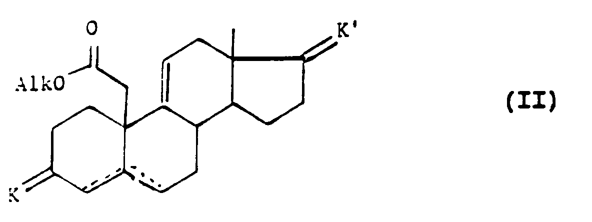 dans laquelle K et K' identiques ou différents représentent un radical céto protégé étant entendu que lorsque K représente un éther d'énol, les doubles liaisons se trouvent en position 3(4) et 5(6) et Alk représente un radical alkyle de 1 à 4 atomes de carbone, les traits pointillés représentant une double liaison en 4(5)ou 5(6), pour obtenir un produit de formule (III) : 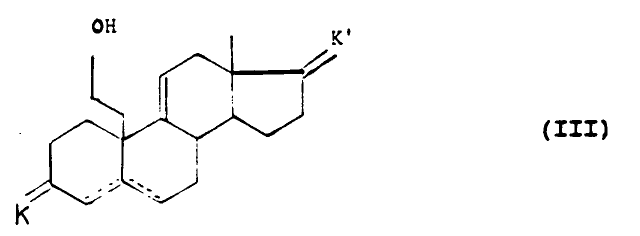 que l'on traite,
ou bien par un réactif de transformation du radical hydroxy en radical thiol pour obtenir un produit de formule (IV) : 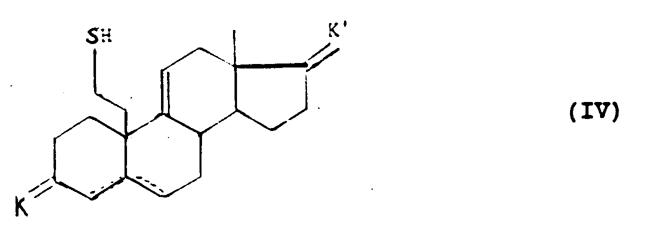 que l'on traite par un dérivé réactif du radical R pour obtenir un produit de formule (V) : 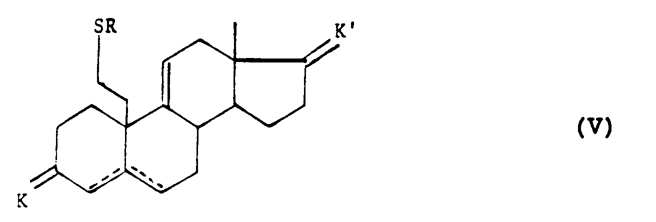 ou bien par un dérivé réactif d'un acide sulfonique pour obtenir un produit de formule (VI) : 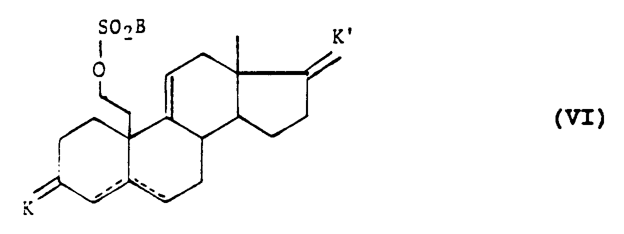 dans laquelle B représente le reste d'un acide sulfonique produit de formule (VI) que l'on traite par un sel de formule RSA dans laquelle A représente un cation monovalent pour obtenir un produit de formule (V) telle que définie ci-dessus, produit de formule (V) que l'on traite par un réactif de déprotection des fonctions cétones protégées pour obtenir un produit de formule (Ia) : 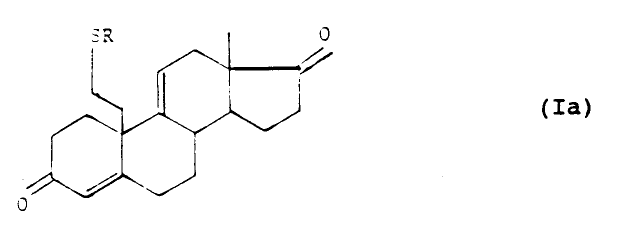 correspondant aux produits de formule (I) dans laquelle R a les significations indiquées ci-dessus, X et Y représentent chacun un atome d'oxygène, n représente le nombre 0, et les traits pointillés en position 4(5) représentent une double liaison et les traits pointillés en positions 1(2) et 6(7) ne représentent pas une seconde liaison entre les carbones qui les portent, produits de formule (Ia) que, si désiré l'on soumet à l'une ou plusieurs des réactions suivantes et dans un ordre quelconque :
- introduction d'une double liaison en position 1(2)
- introduction d'une double liaison en position 6(7)
- oxydation de l'atome de soufre du radical -SR en sulfoxyde
- réduction sélective de la double liaison en position 4(5)
- introduction d'un radical alkyle en position 16
- introduction d'un radical alkylthio ou arylthio éventuellement substitué en position 7,
pour obtenir les produits de formule (Ib) : 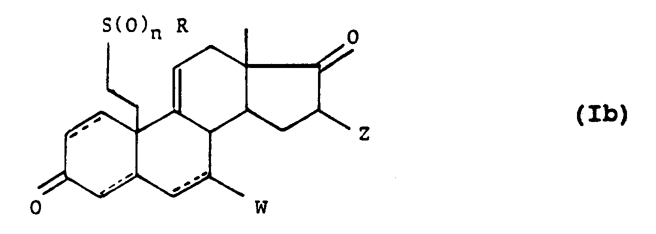 dans laquelle n représente les entiers 0 ou 1 et les traits pointillés en position 1(2), 4(5) et 6(7) indiquent la présence éventuelle d'une double liaison entre les carbones qui les portent et correspondant aux produits de formule (I) dans laquelle R a les significations indiquées ci-dessus, X et Y représentent chacun un atome d'oxygène et W, Z et n et les traits pointillés en positions 1(2), 4(5) et 6(7) ont les significations précédentes à l'exception des produits de formule (Ib) dans laquelle Z=H, W=H, n=O et dans laquelle les traits pointillés en position 1(2) et 6(7) ne representent pas une seconde liaison entre les carbones qui les portent, et les traits pontillés en position 4(5) representent une seconde liaison entre les carbones qui les portent et si désiré soumet les produits de formules (Ia) et (Ib) à l'une ou plusieurs des réactions suivantes dans un ordre quelconque :
- introduction d'un radical 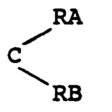 en position 3
- action de l'hydroxylamine ou d'un dérivé de formule H₂N-O-R₁,
- réduction de la fonction cétone en position 3 et/ou 17 et acylation éventuelle de la fonction hydroxy puis si désiré salification de la fonction estérifiée,
- transformation de la fonction cétone en position 17 en groupement 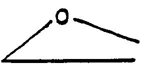 (CH₂)_{q} pour obtenir un produit de formule (Ic) : 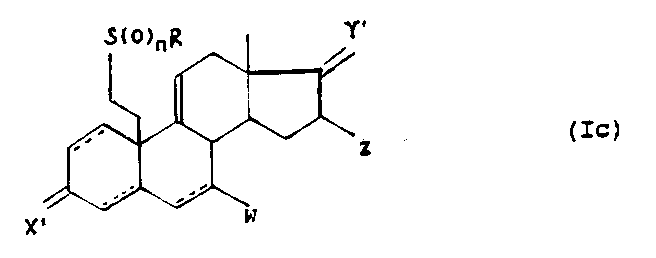 dans laquelle n, R, W, Z et les traits pointillés en positions 1(2), 4(5) et 6(7) ont la signification indiquée ci-dessus et X' et Y' représentent les valeurs indiquées ci-dessus pour X et Y étant entendu que l'un au moins de X ou Y ne représente pas un atome d'oxygène, et étant entendu que =X et/ou =Y ne représente pas

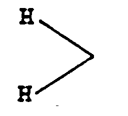

6. Procédé de préparation des produits de formule (I) telle que définie à la revendication 1, caractérisée en ce que l'on soumet un produit de formule (IV) ou (I'a) : 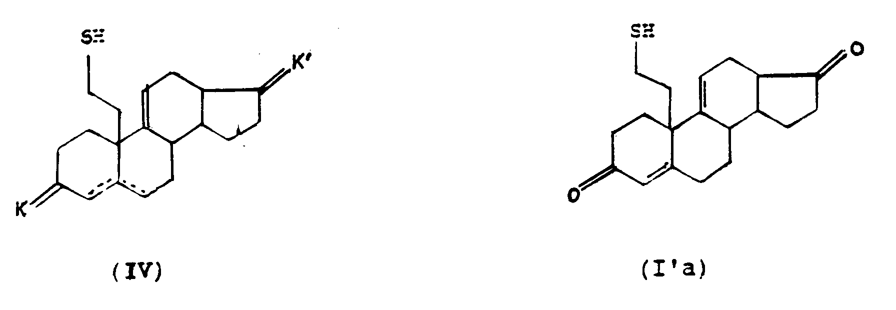 telles que définies précédemment, à l'action d'un halogénure de 2,4-dinitrophenylsulfényle pour obtenir un produit de formule (VII) : 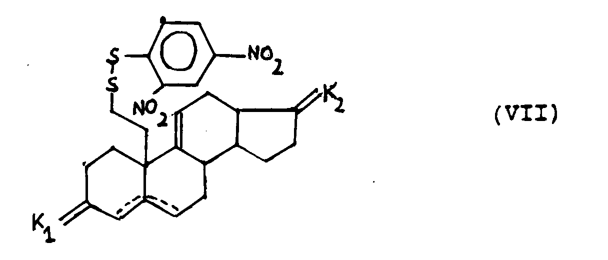 dans laquelle K₁ et K₂ représentent tous deux une fonction cétone ou tous deux une fonction cétone protégée et le trait pointillé représente une double liaison en 4(5) ou 5(6) ou 3(4) et 5(6) lorsque K₁ représente une fonction cétone protégée ou en 4(5) lorsque K₁ représente une fonction cétone, produit de formule (VII) que
1) soit l'on soumet le cas échéant à un réactif de déprotection des fonctions cétone en position 3 ou 17 pour obtenir un produit de formule (Id) : 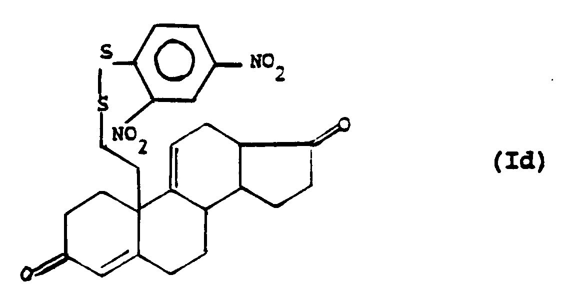
2) soit l'on soumet à l'action d'un réactif organomagnésien de formule R''-Mg-Hal dans laquelle Hal représente un atome d'halogène et R'' représente un radical alkyle, alcényle, alcynyle, ou CN
ou à l'action d'un dérivé organolithien R''Li protégé puis à un agent de déprotection du radical R'' puis le cas échéant à une réaction de déprotection des fonctions cétone en position 3 et/ou 17 pour obtenir un produit de formule (Ie) : 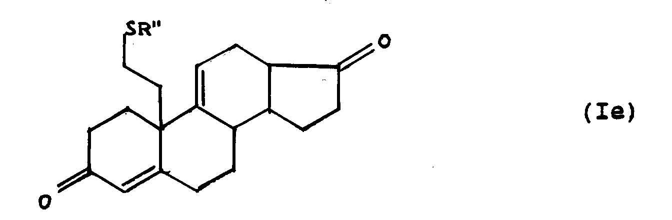 dans laquelle R'' a la signification indiquée ci-dessus,
3) soit l'on soumet à l'action d'un sel de formule R'''SA dans laquelle A a la signification indiquée à la revendication 5 et R''' représente un radical alkyle ayant au plus 6 atomes de carbone ou aryle éventuellement substitué ayant au plus 10 atomes de carbone, puis le cas échéant à une réaction de déprotection des fonctions cétones en position 3 et/ou 17 pour obtenir un produit de formule (If) : 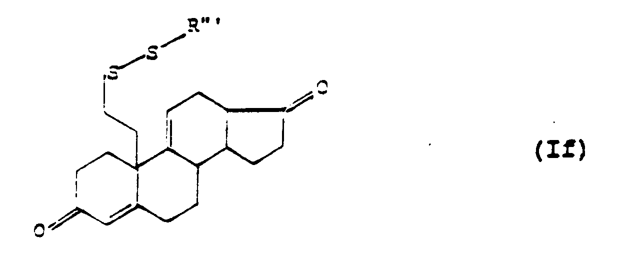 produits de formule (Id), (Ie), (If) que l'on soumet si désiré à une ou plusieurs des réactions indiquées à la revendication 5, pour les produits de formule (Ia) ou (Ib) dans un ordre quelconque.

7. Procédé de préparation des produits de formule (I) dans laquelle =X et/ou =Y représente 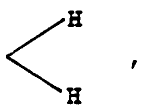 caractérisé en ce que l'on soumet un produit de formule (II) telle que définie à la revendication 5, à un agent de réduction des fonctions cétone protégées pour obtenir un produit de formule (III) dans laquelle =K et/ou =K' représente des atomes d'hydrogène et poursuit la synthèse comme indiqué à la revendication 5 ou 6.

8. A titre de médicaments les produits de formule générale (I) telle que décrits à la revendication 1.

9. A titre de médicaments les produits de formule générale (I') telle que définie à l'une des revendications 2 à 4.

10. Les compositions pharmaceutiques contenant, à titre de principe actif l'un au moins des médicaments définis à l'une des revendications 8 ou 9.

11. A titre de produits industriels, les produits de formules générales (III), (IV), (V) et (VI), telles que définies à la revendication 5, caractérisé en ce que =K et/ou K' représentent les fonctions cétones protégées sous forme des radicaux éthylenedioxy, éthylenedithio ou de l'ethyl ether d'enol du type 3 ethoxy 3(4), 5(6) - diène et caractérisé en ce que B represente la reste d'un acide sulfonique choisi parmi un radical methyle ou tolyle.

## Revendications (Revendications pour l'(les) Etat(s) contractant(s) suivant(s): ES)

1. Procédé de préparation des produits de formule générale (I''): 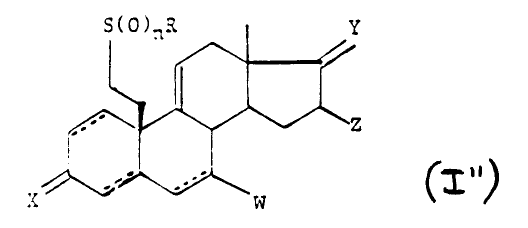 dans laquelle R représente :
un atome d'hydrogène,
un radical alkyle, alcényle, alcynyle, alkylthio ayant au plus 6 atomes de carbone ou arylthio éventuellement substitué ayant au plus 10 atomes de carbone,
un radical, acyle ayant au plus 12 atomes de carbone ou un radical CN,
- X représente un atome d'oxygène, un radical N-O-R₁ dans lequel R₁ représente un atome d'hydrogène ou un radical alkyle ayant de 1 à 6 atomes de carbone ou X représente 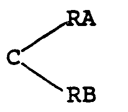 dans lequel RA et RB sont identiques et représentent un atome d'hydrogène, ou =X représente un reste 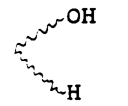 dans lequel le radical OH peut se trouver en position alpha ou bêta, le radical hydroxyle étant éventuellement acylé,
- Y représente un atome d'oxygène, un radical N-O-R₁ dans lequel R₁ a la définition indiquée ci-dessus, dessus, ou =Y représente un reste 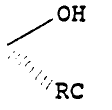 dans lequel RC représente un atome d'hydrogène le radical hydroxyle étant éventuellement acylé ou =Y représente un groupe 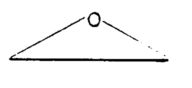 (CH₂)q dans lequel q représente un nombre de 1 à 3,
- W représente un atone d'hydrogène, un radical alkylthio ou arylthio éventuellement substitué renfermant jusqu'à 10 atomes de carbone,
- Z représente un atome d'hydrogène ou un radical alkyle renfermant jusqu'à 10 atomes de carbone,
- n représente un entier de 0 à 1 et les traits pointillés en positions 1(2), 4(5) et 6(7) indiquent la présence éventuelle d'une seconde liaison entre les carbones qui les portent, ainsi que leurs sels,
étant entendu que lorsque n est égal à 1, R représente un radical methyle, X et Y représentent chacun un atome d'oxygène, W et Z représentent chacun un atome d'hydrogène, le trait en pointillé en position 4(5) représente une seconde liaison entre les carbones qui les portent et les traits pointillés en position 1(2) et 6(7) ne représentent pas une seconde liaison entre les carbones qui les portent.
caractérisé en ce que l'on soumet à une réduction un produit de formule (II) : 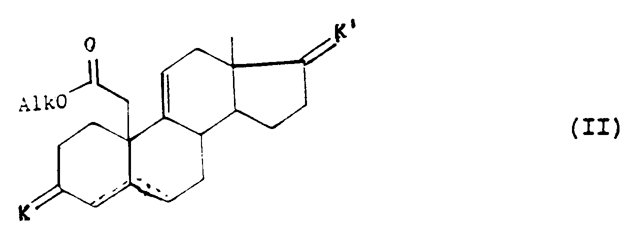 dans laquelle K et K' identiques ou différents représentent un radical céto protégé étant entendu que lorsque K représente un éther d'enol, les doubles liaisons se trouvent en postion 3(4) et 5(6) et Alk représente un radical alkyle de 1 à 4 atomes de carbone, les traits pointillés représentant une double liaison en 4(5)ou 5(6), pour obtenir un produit de formule (III) : 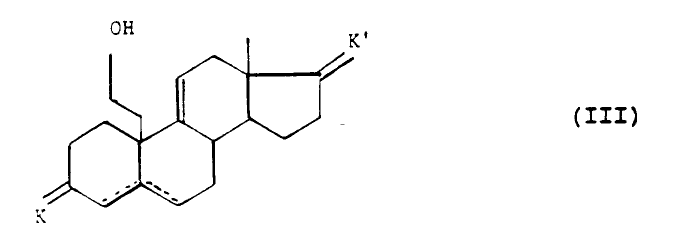 que l'on traite,
ou bien par un réactif de transformation du radical hydroxy radical thiol pour obtenir un produit de formule (IV) : 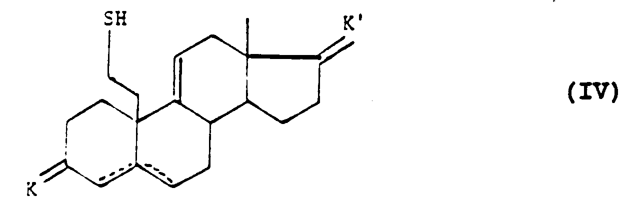 que l'on traite par un dérivé réactif du radical R pour obtenir un produit de formule (V) : 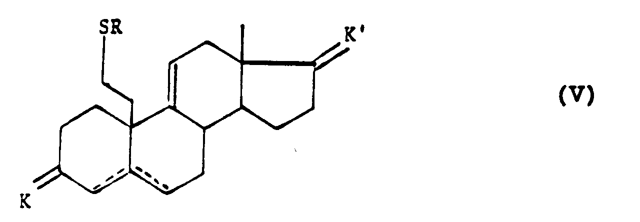 ou bien par un dérivé réactif d'un acide sulfonique pour obtenir un produit de formule (VI) : 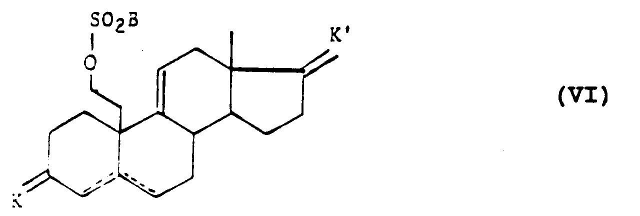 dans laquelle B représente le reste d'un acide sulfonique produit de formule (VI) que l'on traite par un sel de formule RSA dans laquelle A représente un cation monovalent pour obtenir un produit de formule (V) telle que définie ci-dessus, produit de formule (V) que l'on traite par un réactif de déprotection des fonctions cétones protégées pour obtenir un produit de formule (Ia) : 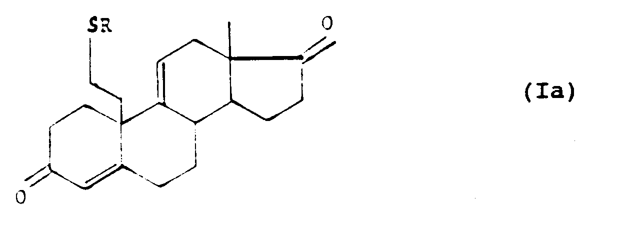 correspondant aux produits de formule (I'') dans laquelle R a les significations indiquées ci-dessus, X et Y représentent chacun un atome d'oxygène, n représente le noire 0, et les traits pointillés en position 4(5) représentent une double liaison et les traits pointillés en positions 1(2) et 6(7) ne représentent pas une seconde liaison entre les carbones qui les portent, produits de formule (Ia) que, si désiré l'on soumet à l'une ou plusieurs des réactions suivantes et dans un ordre quelconque :
- introduction d'une double liaison en position 1(2)
- introduction d'une double liaison en position 6(7)
- oxydation de l'atome de soufre du radical -SR en sulfoxyde
- réduction sélective de la double liaison en position 4(5)
- introduction d'un radical alkyle en position 16
- introduction d'un radical alkylthio ou arylthio éventuellement substitué en position 7,
pour obtenir les produits de formule (Ib) : 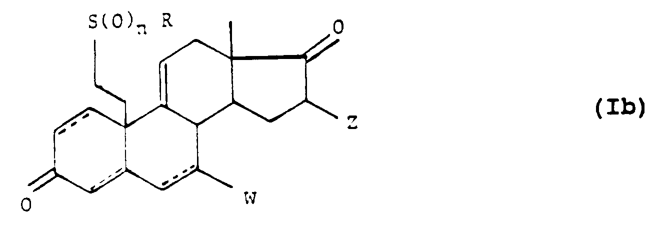 dans laquelle n représente les entiers 0 ou 1 et les traits pointillés en position 1(2), 4(5) et 6(7) indiquent la présence éventuelle d'une double liaison entre les carbones qui les portent et correspondant aux produits de formule (I'') dans laquelle R a les significations indiquées ci-dessus, X et Y représentent chacun un atome d'oxygène et W, Z et n et les traits pointillés en positions 1(2), 4(5) et 6(7) ont les significations précédentes à l'exception des produits de formule (Ib) dans laquelle Z=H, W=H, n=O et dans laquelle les traits pointillés en position 1(2) et 6(7) ne représentent pas une seconde liaison entre les carbones qui les portent et les traits pontillés en position 4(5) représentent une seconde liaison entre les carbones qui les portent et si désiré soumet les produits de formules (Ia) et (Ib) à l'une ou plusieurs des réactions suivantes dans un ordre quelconque :
- introduction d'un radical 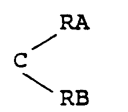 en position 3
- action de l'hydroxylamine ou d'un dérivé de formule H₂N-O-R₁,
- réduction de la fonction cétone en position 3 et/ou 17 et acylation éventuelle de la fonction hydroxy puis si désiré salification de la fonction estérifiée,
- transformation de la fonction cétone en position 17 en groupement 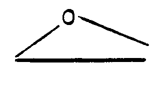 (CH₂)_{q} pour obtenir un produit de formule (Ic) : 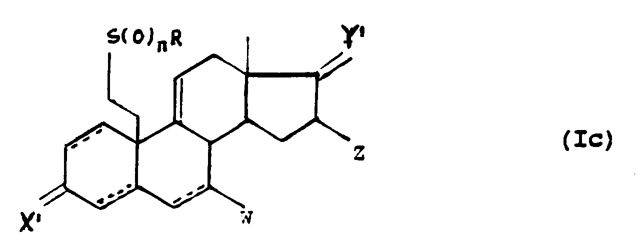 dans laquelle n, R, W, Z et les traits pointillés en positions 1(2), 4(5) et 6(7) ont la signification indiquée ci-dessus et X' et Y' représentent les valeurs indiquées ci-dessus pour X et Y étant entendu que l'un au moins de X ou Y ne représente pas un atome d'oxygène

2. Procédé de préparation des produits de formule (I'') telle que définie à la revendication 1, caractérisée en ce que l'on soumet un produit de formule (IV) ou (I'a) : 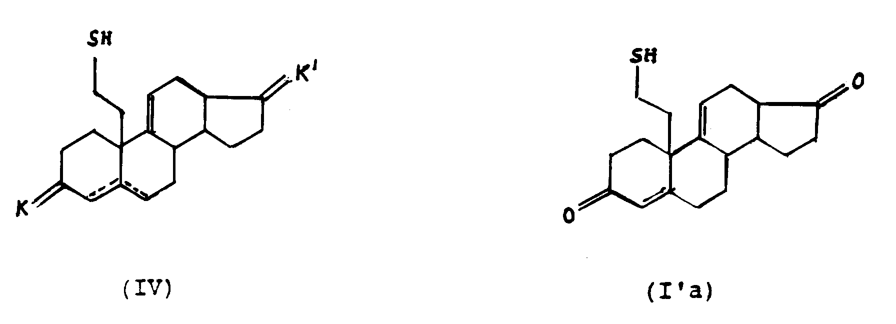 telles que définies precedemment, à l'action d'un halogénure de 2,4-dinitrophénylsulfényle pour obtenir un produit de formule (VII) : 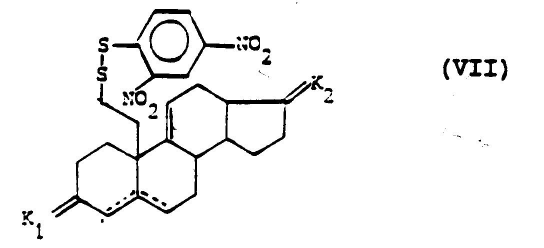 dans laquelle K₁ et K₂ représentent tous deux une fonction cétone ou tous deux une fonction cétone protégée et le trait pointillé représente une double liaison en 4(5) ou 5(6) ou 3(4) et 5(6) lorsque K₁ représente une fonction cétone protégée ou en 4(5) lorsque K₁ représente une fonction cétone, produit de formule (VII) que
1) soit l'on soumet le cas échéant à un réactif de déprotection des fonctions cétone en position 3 ou 17 pour obtenir un produit de formule (Id) : 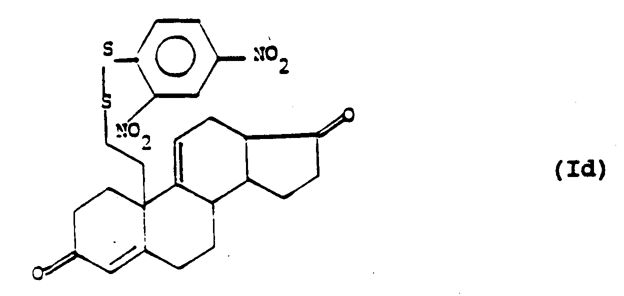
2) soit l'on soumet à l'action d'un réactif organomagnésien de formule R''-Mg-Hal dans laquelle Hal représente un atome d'halogène et R'' représente un radical alkyle, alcényle, alcynyle, ou CN
ou à l'action d'un dérivé organolithien R''Li protégé puis à un agent de déprotection du radical R'' puis le cas échéant à une réaction de déprotection des fonctions cétone en position 3 et/ou 17 pour obtenir un produit de formule (Ie) : 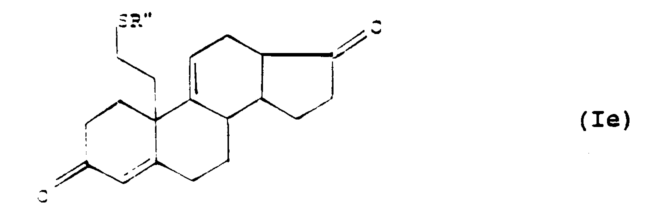 dans laquelle R'' a la signification indiquée ci-dessus,
3) soit l'on soumet à l'action d'un sel de formule R'''SA dans laquelle A a la signification indiquée à la revendication 1 et R''' représente un radical alkyle ayant au plus 6 atomes de carbone ou aryle éventuellement substitué ayant, au plus 10 atomes de carbone, puis le cas échéant, à une réaction de déprotection des fonctions cétones en position 3 et/ou 17 pour obtenir un produit de formule (If) : 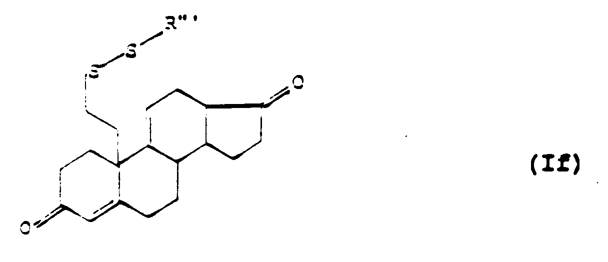 produits de formule (Id), (Ie), (If) que l'on soumet si désiré à une ou plusieurs des réactions indiquées à la revendication 1 pour les produits de formule (Ia) ou (Ib) dans un ordre quelconque.

3. Procédé de préparation des produits correspondant à ceux de formule (I'') dans laquelle =X et/ou =Y représente 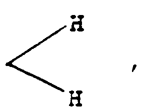 caractérisé en ce que l'on soumet un produit de formule (II) telle que définie à la revendication 1, à un agent de réduction des fonctions cétone protégées pour obtenir un produit de formule (III) dans laquelle =K et/ou =K' représente des atomes d'hydrogène et poursuit la synthèse comme indique à la revendication 1 ou 2.

4. Procédé selon l'une quelconque des revendications 1 à 3, caractérisé en ce que l'on prépare des produits de formule (I') : 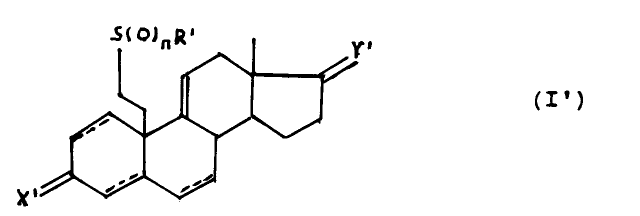 dans laquelle R' représente :
un radical alkyle, alcényle, alcynyle, ou alkylthio ayant au plus 4 atomes de carbone,
un radical CN,
un radical acyle ayant au plus 4 atomes de carbone,
- X' représente un atome d'oxygène, un radical CH₂, un radical N-O-R'₁ dans lequel R'₁ représente un atome d'hydrogène ou un radical alkyle ayant de 1 à 4 atomes de carbone, ou X' représente un reste 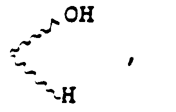
- Y' représente un atome d'oxygène ou =Y' représente un reste 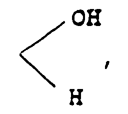 le radical hydroxy étant éventuellement acylé sous forme de radical alcanoyloxy ou d'un radical 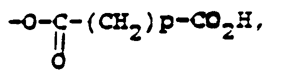 dans lequel p représente en nombre de 2 à 5 les traits pointillés en positions 1(2), 4(5) et 6(7) indiquent la présence éventuelle d'une seconde liaison entre les carbones qui les portent, ainsi que leurs sels.

5. Procédé selon la revendication 4 caractérisé en ce que l'on prepare des produits dans R' lesquels est choisi parmi les radicaux alkyle, alcényle, ou alcynyle ayant de 1 à 4 atomes de carbone et X' représente un atome d'oxygène ou un reste

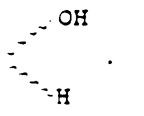

6. Procédé selon la revendication 4 caractérisé en ce que l'on prépare des produits répondant aux formules :
- la 10bêta-[2-(methylthio) éthyl] estra-4,9(11)-diène-3,17-dione.
- la 10bêta-[2-(éthylthio) éthyl] estra-4,9(11)-diène-3,17-dione,
- la 10bêta-[2-(methylthio) éthyl] 17bêta-hydroxy estra-4,9(11)-diène-3-one.
- la 10bêta-[2-(éthenylthio) éthyl] estra-4,9(11)-diène-3,17-dione,
- la 10bêta-[2-(éthynylthio) éthyl] estra-4,9(11)-diène-3,17-dione,
- le butane dioate de 10bêta-[2-(méthylthio) éthyl] 3-oxo estra-4,9(11)-dièn 17bêta-yle et de sodium,
- le 10bêta-[2-(méthylthio) éthyl] estra-4,9(11)-diène-3bêta, 17bêta-diol,
- la 10bêta-[2-(méthylthio) éthyl] estra-4,9(11)-diène-3alpha, 17bêta-diol,
- la 10bêta-[2-(méthylthio) éthyl] estr-9(11)-ène-3,17-dione.

## Revendications (Revendications pour l'(les) Etat(s) contractant(s) suivant(s): GR)

1. Procédé de préparation des produits de formule générale (I''): 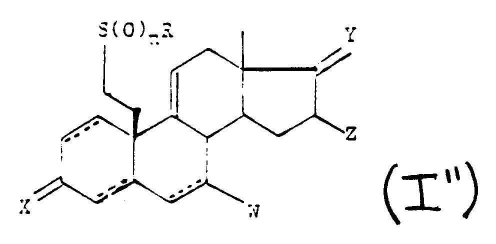 dans laquelle R représente :
un atome d'hydrogène,
un radical alkyle, alcényle, alcynyle, alkylthio ayant au plus 6 atomes de carbone ou arylthio éventuellement substitué ayant au plus 10 atomes de carbone,
un radical, acyle ayant au plus 12 atomes de carbone ou un radical CN,
- X représente un atome d'oxygène, un radical N-O-R₁ dans lequel R₁ représente un atome d'hydrogène ou un radical alkyle ayant de 1 à 6 atomes de carbone ou X représente 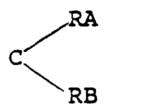 dans lequel RA et RB sont identiques et représentent un atome d'hydrogène, ou =X représente un reste 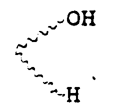 dans lequel le radical OH peut se trouver en position alpha ou bêta, le radical hydroxyle étant éventuellement acylé,
- Y représente un atome d'oxygène, un radical N-O-R₁ dans lequel R₁ a la définition indiquée ci-dessus, dessus, ou =Y représente un reste 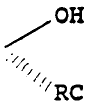 dans lequel RC représente un atome d'hydrogène le radical hydroxyle étant éventuellement acylé ou =Y représente un groupe 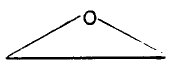 (CH₂)q dans lequel q représente un nombre de 1 à 3,
- W représente un atome d'hydrogène, un radical alkylthio ou arylthio éventuellement substitué renfermant jusqu'à 10 atomes de carbone,
- Z représente un atome d'hydrogène ou un radical alkyle renfermant jusqu'à 10 atomes de carbone,
- n représente un entier de 0 à 1 et les traits pointillés en positions 1(2), 4(5) et 6(7) indiquent la présence éventuelle d'une seconde liaison entre les carbones qui les portent, ainsi que leurs sels,
étant entendu que lorsque n est égal à 1, R représente un radical methyle, X et Y représentent chacun un atone d'oxygène, W et Z représentent chacun un atome d'hydrogène, le trait en pointillé en position 4(5) représente une seconde liaison entre les carbones qui les portent et les traits pointillés en position 1(2) et 6(7) ne représentent pas une seconde liaison entre les carbones qui les portent. caractérisé en ce que l'on soumet à une réduction un produit de formule (II) : 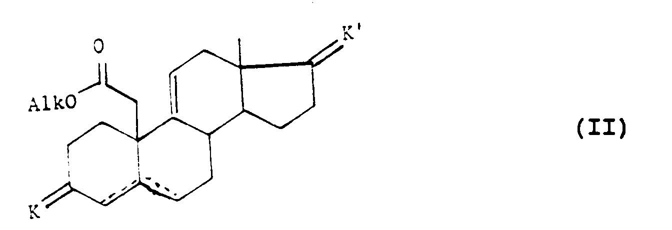 dans laquelle K et K' identiques ou différents représentent un radical céto protégé étant entendu que lorsque K représente un éther d'enol, les doubles liaisons se trouvent en position 3(4) et 5(6) et Alk représente un radical alkyle de 1 à 4 atomes de carbone, les traits pointillés représentant une double liaison en 4(5)ou 5(6), pour obtenir un produit de formule (III) : 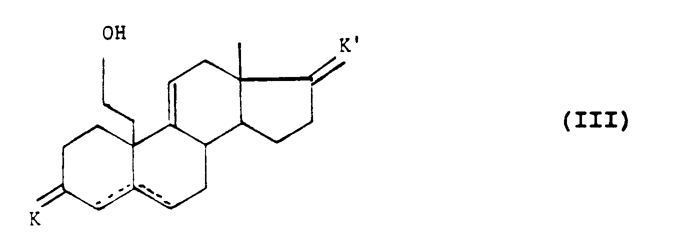 que l'on traite,
ou bien par un réactif de transformation du radical hydroxy en radical thiol pour obtenir un produit de formule (IV) : 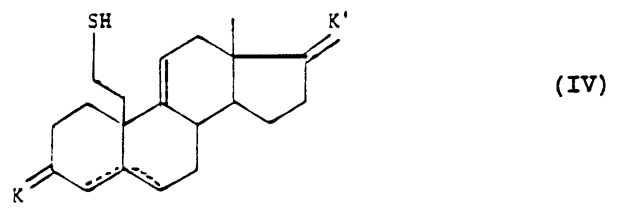 que l'on traite par un dérivé réactif du radical R pour obtenir un produit de formule (V) : 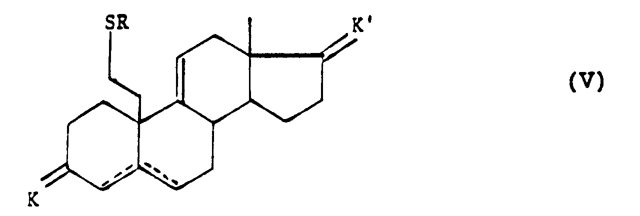 ou bien par un dérivé réactif d'un acide sulfonique pour obtenir un produit de formule (VI) : 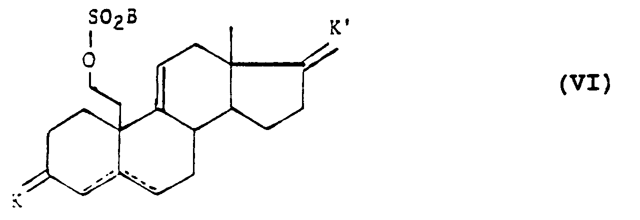 dans laquelle B représente le reste d'un acide sulfonique produit de formule (VI) que l'on traite par un sel de formule RSA dans laquelle A représente un cation monovalent pour obtenir un produit de formule (V) telle que définie ci-dessus, produit de formule (V) que l'on traite par un réactif de déprotection des fonctions cétones protégées pour obtenir un produit de formule (Ia) : 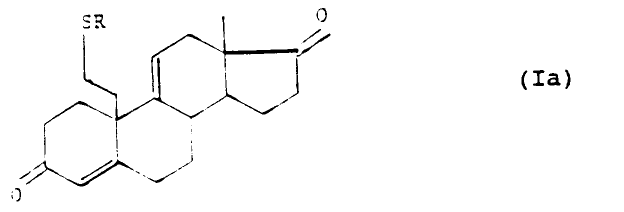 correspondant aux produits de formule (I'') dans laquelle R a les significations indiquées ci-dessus, X et Y représentent chacun un atome d'oxygène, n représente le nombre 0, et les traits pointillés en position 4(5) représentent une double liaison et les traits pointillés en positions 1(2) et 6(7) ne représentent pas une seconde liaison entre les carbones qui les portent, produits de formule (Ia) que, si désiré l'on soumet à l'une ou plusieurs des réactions suivantes et dans un ordre quelconque :
- introduction d'une double liaison en position 1(2)
- introduction d'une double liaison en position 6(7)
- oxydation de l'atome de soufre du radical -SR en sulfoxyde
- réduction sélective de la double liaison en position 4(5)
- introduction d'un radical alkyle en position 16
- introduction d'un radical alkylthio ou arylthio éventuellement substitué en position 7,
pour obtenir les produits de formule (Ib) : 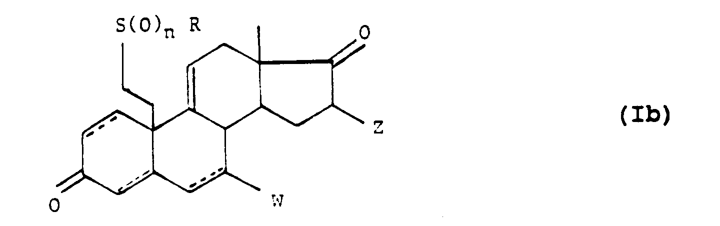 dans laquelle n représente les entiers 0 ou 1 et les traits pointillés en position 1(2), 4(5) et 6(7) indiquent la présence éventuelle d'une double liaison entre les carbones qui les portent et correspondant aux produits de formule (I'') dans laquelle R a les significations indiquées ci-dessus, X et Y représentent chacun un atome d'oxygène et W, Z et n et les traits pointillés en positions 1(2), 4(5) et 6(7) ont les significations précédentes à l'exception des produits de formule (Ib) dans laquelle Z=H, W=H, n=O et dans laquelle les traits pointilles en position 1(2) et 6(7) ne representent pas une seconde liaison entre les carbones qui les portent et les traits pontillés en position 4(5) representent une seconde liaison entre les carbones qui les portent et si désiré soumet les produits de formules (Ia) et (Ib) à l'une ou plusieurs des réactions suivantes dans un ordre quelconque :
- introduction d'un radical 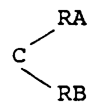 en position 3
- action de l'hydroxylamine ou d'un dérivé de formule H₂N-O-R₁,
- réduction de la fonction cétone en position 3 et/ou 17 et acylation éventuelle de la fonction hydroxy puis si désiré salification de la fonction estérifiée,
- transformation de la fonction cétone en position 17 en groupement 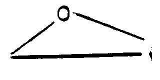 (CH₂)_{q} pour obtenir un produit de formule (Ic) : 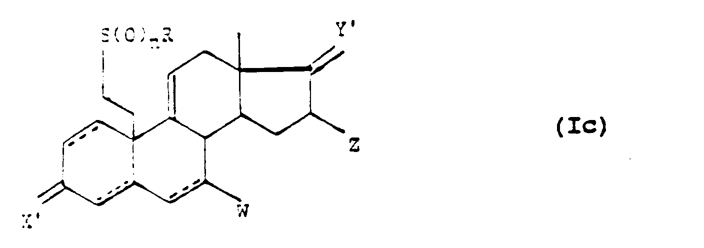 dans laquelle n, R, W, Z et les traits pointillés en positions 1(2), 4(5) et 6(7) ont la signification indiquée ci-dessus et X' et Y' représentent les valeurs indiquées ci-dessus pour X et Y étant entendu que l'un au moins de X ou Y ne représente pas un atome d'oxygène

2. Procédé de préparation des produits de formule (I'') telle que définie à la revendication 1, caractérisée en ce que l'on soumet un produit de formule (IV) ou (I'a) : 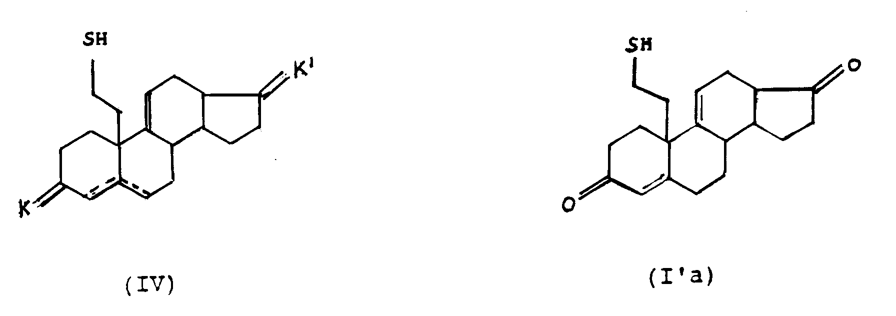 telles que définies precédemment, à l'action d'un halogénure de 2,4-dinitrophénylsulfényle pour obtenir un produit de formule (VII) : 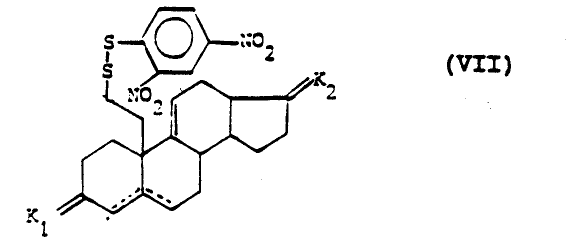 dans laquelle K₁ et K₂ représentent tous deux une fonction cétone ou tous deux une fonction cétone protégée et le trait pointillé représente une double liaison en 4(5) ou 5(6) ou 3(4) et 5(6) lorsque K₁ représente une fonction cétone protégée ou en 4(5) lorsque K₁ représente une fonction cétone, produit de formule (VII) que
1) soit l'on soumet le cas échéant à un réactif de déprotection des fonctions cétone en position 3 ou 17 pour obtenir un produit de formule (Id) : 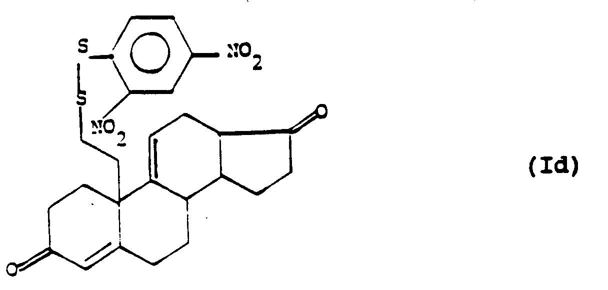
2) soit l'on soumet à l'action d'un réactif organomagnésien de formule R''-Mg-Hal dans laquelle Hal représente un atome d'halogène et R'' représente un radical alkyle, alcényle, alcynyle, CN
ou à l'action d'un dérivé organolithien R''Li protégé puis à un agent de déprotection du radical R'' puis le cas échéant à une réaction de déprotection des fonctions cétone en position 3 et/ou 17 pour obtenir un produit de formule (Ie) : 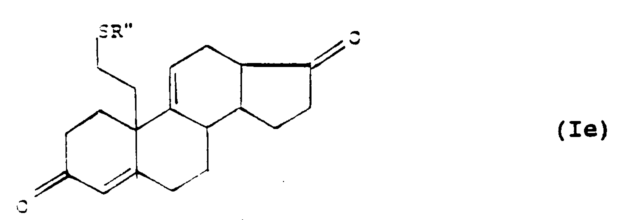 dans laquelle R'' a la signification indiquée ci-dessus,
3) soit l'on soumet à l'action d'un sel de formule R'''SA dans laquelle A a la signification indiquée à la revendication 1 et R''' représente un radical alkyle ayant au plus 6 atomes de carbone ou aryle éventuellement substitué ayant au plus 10 atomes de carbone, puis le cas échéant, à une réaction de déprotection des fonctions cétones en position 3 et/ou 17 pour obtenir un produit de formule (If) : 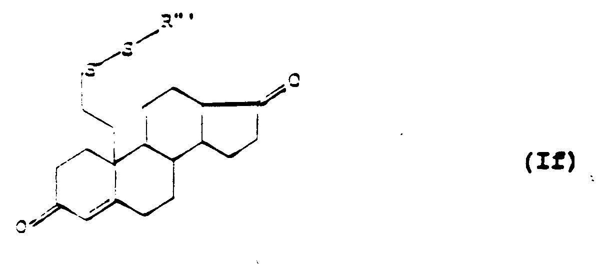 produits de formule (Id), (Ie), (If) que l'on soumet si désiré à une ou plusieurs des réactions indiquées à la revendication 1 pour les produits de formule (Ia) ou (Ib) dans un ordre quelconque.

3. Procédé de préparation des produits correspondant à ceux de formule (I'') dans laquelle =X et/ou =Y représente 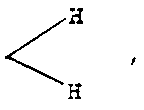 caractérisé en ce que l'on soumet un produit de formule (II) telle que définie à la revendication 1, à un agent de réduction des fonctions cétone protégées pour obtenir un produit de formule (III) dans laquelle =K et/ou =K' représente des atomes d'hydrogène et poursuit la synthèse comme indique à la revendication 1 ou 2.

4. Procédé selon l'une quelconque des revendications 1 à 3, caractérisé en ce que l'on prépare des produits de formule (I') : 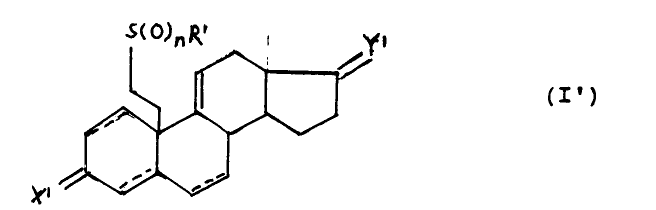 dans laquelle R' représente :
un radical alkyle, alcényle, alcynyle, ou alkylthio ayant au plus 4 atomes de carbone,
un radical CN,
un radical acyle ayant au plus 4 atomes de carbone,
- X' représente un atome d'oxygène, un radical CH₂, un radical N-O-R'₁ dans lequel R'₁ représente un atome d'hydrogène ou un radical alkyle ayant de 1 à 4 atomes de carbone, ou X' représente un reste 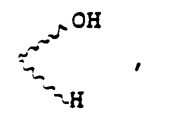
- Y' représente un atome d'oxygène ou =Y' représente un reste 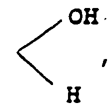 le radical hydroxy étant éventuellement acylé sous forme de radical alcanoyloxy ou d'un radical 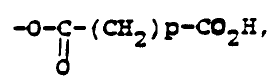 dans lequel p représente un nombre de 2 à 5 les traits pointillés en positions 1(2), 4(5) et 6(7) indiquent la présence éventuelle d'une seconde liaison entre les carbones qui les portent, ainsi que leurs sels.

5. Procédé selon la revendication 4 caractérisé en ce que l'on prépare des produits dans R' lesquels est choisi parmi les radicaux alkyle, alcényle, ou alcynyle ayant de 1 à 4 atomes de carbone et X' représente un atome d'oxygène ou un reste

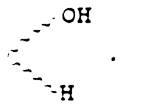

6. Procédé selon la revendication 4 caractérisé en ce que l'on prepare des produits répondant aux formules :
- la 10bêta-[2-(methylthio) éthyl] estra-4,9(11)-diène-3,17-dione,
- la 10bêta-[2-(éthylthio) éthyl] estra-4,9(11)-diène-3,17-dione,
- la 10bêta-[2-(methylthio) éthyl] 17bêta-hydroxy estra-4,9(11)-diène-3-one,
- la 10bêta-[2-(éthenylthio) éthyl] estra-4,9(11)-diène-3,17-dione,
- la 10bêta-[2-(éthynylthio) éthyl] estra-4,9(11)-diène-3,17-dione,
- le butane dioate de 10bêta-[2-(méthylthio) éthyl] 3-oxo estra-4,9(11)-dièn 17bêta-yle et de sodium,
- le 10bêta-[2-(méthylthio) éthyl] estra-4,9(11)-diène-3bêta, 17bêta-diol,
- la 10bêta-[2-(méthylthio) éthyl] estra-4,9(11)-diène-3alpha, 17bêta-diol,
- la 10bêta-[2-(méthylthio) éthyl] estr-9(11)-ène-3,17-dione.

7. Procédé de préparation de compositions pharmaceutiques, caractérisé en ce que l'on met à titre de principe actif l'un au moins des composés de formule (I'') telle que définie à la revendication 1 ou l'un au moins de leurs sels pharmaceutiquement acceptables sous une forme destinée à cet usage.

8. Procédé de préparation de compositions pharmaceutiques, caractérisé en ce que l'on met à titre de principe actif l'un au moins des composés de formule (I'') ou les composés correspondant à ceux de la formule (I'') dans laquelle X et/ou Y representent deux atomes d'hydrogène telle que définie à l'une quelconque des revendications 2 à 5, ou l'un au moins de leurs sels pharmaceutiquement acceptables sous une forme destinée à cet usage.

9. Procédé selon la revendication 7 ou 8, caractérisé en ce que le composé de formule (I) est choisi parmi les produits répondant aux formules :
- la 10bêta-[2-(méthylthio) éthyl] estra-4,9(11)-diène-3,17-dione,
- la 10bêta-[2-(éthylthio) éthyl] estra-4,9(11)-diène-3,17-dione,
- la 10bêta-[2-(méthylthio) éthyl] 17bêta-hydroxy estra-4,9(11)-diène-3-one,
- la 10bêta-[2-(éthénylthio) éthyl] estra-4,9(11)-diène-3,17-dione,
- la 10bêta-[2-(éthynylthio) éthyl] estra-4,9(11)-diène-3,17⁻dione,
- le butane dioate de 10bêta-[2-(méthylthio) éthyl] 3-oxo estra-4,9(11)-dièn 17bêta-yle et de sodium,
- le 10bêta-[2-(méthylthio) éthyl] estra-4,9(11)-diène-3bêta, 17bêta-diol,
- la 10bêta-[2-(méthylthio) éthyl] estra-4,9(11)-diène-3alpha, 17bêta-diol,
- la 10bêta-[2-(méthylthio) éthyl] estr-9(11)-ène-3,17-dione.

10. A titre de produits industriels, les produits de formules générales (III), (IV), (V) et (VI), telles que définies à la revendication 1, caractérisé en ce que =K et/ou =K' representent les fonctions cétones protégées sous forme des radicaux éthylenedioxy, éthylenedithio ou de l'ethyle ether d'enol du type 3-ethoxy-3(4),5(6)-diène, et caractérisé en ce que B represente le reste d'un acide sulfonique choisi parmi un radical methyle ou tolyle.

## Claims (Claims for the following Contracting State(s): DE, GB, FR, IT, NL, SE, LI, CH, BE, AT, LU, DK)

1. The products of general formula (I): 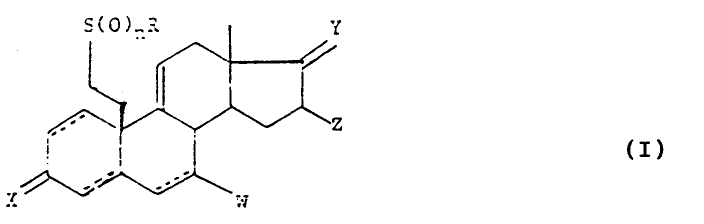 in which R represents:
a hydrogen atom,
an alkyl, alkenyl, alkynyl, alkylthio radical having at most 6 carbon atoms or an optionally substituted arylthio radical having at most 10 carbon atoms,
an acyl radical having at most 12 carbon atoms or a CN radical,
- X represents an oxygen atom, an N-O-R₁ radical in which R₁ represents a hydrogen atom or an alkyl radical having 1 to 6 carbon atoms or X represents 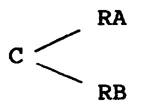 in which RA and RB are identical and represent a hydrogen atom, or =X represents 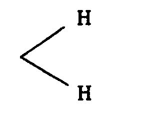 or a 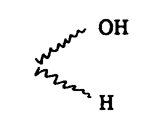 remainder
in which the OH radical can be found in alpha or beta position, the hydroxyl radical being optionally acylated,
- Y represents an oxygen atom, an N-O-R₁ radical in which R₁ has the definition indicated above, or =Y represents 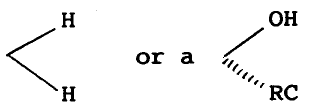 remainder in which RC represents a hydrogen atom, the hydroxyl radical being optionally acylated, or =Y
represents a group 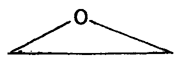 (CH₂)q in which q represents a number from 1 to 3,
- W represents a hydrogen atom an alkylthio or arylthio radical, optionally substituted containing up to 10 carbon atoms,
- Z represents a hydrogen atom or an alkyl radical containing up to 10 carbon atoms,
- n represents a whole number from 0 to 1 and the dotted lines in positions 1(2), 4(5) and 6(7) indicate the optional presence of a second bond between the carbons that carry them, as well as their salts,
it being understood that when n is equal to 1, R represents a methyl radical, X and Y each represent an oxygen atom, W and Z each represent a hydrogen atom, the dotted line in position 4(5) represents a second bond between the carbons which carry them and the dotted lines in position 1(2) and 6(7) do not represent a second bond between the carbons which carry them.

2. The products of general formula (I) as defined in claim 1 corresponding to general formula (I'): 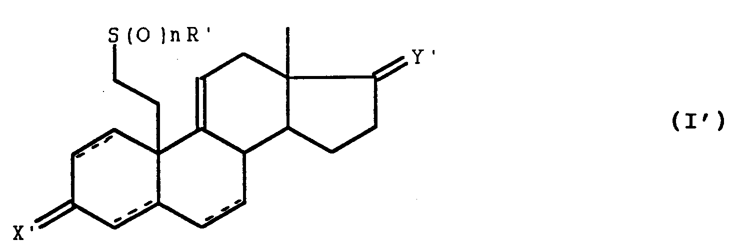 in which R' represents:
an alkyl, alkenyl, alkynyl or alkylthio radical having at most 4 carbon atoms,
a CN radical,
an acyl radical having at most 4 carbon atoms,
- X' represents an oxygen atom, a CH₂ radical, an N-O-R'₁ radical in which R₁ represents a hydrogen atom or an alkyl radical having 1 to 4 carbon atoms, or X' represents a 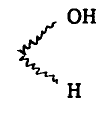 remainder
- Y' represents an oxygen atom or =Y' represents a 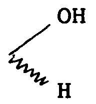 remainder, the hydroxy radical being optionally acylated in the form of an alkanoyloxy radical or an 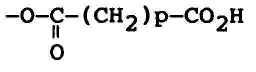 radical, in which p represents an integer from 2 to 5.
The dotted lines in positions 1(2), 4(5) and 6(7) indicate the optional presence of a second bond between the carbons which carry them, as well as their salts.

3. The products of formula (I') as defined in claim 2 in which R' is chosen from alkyl, alkenyl or alkynyl radicals having 1 to 4 carbon atoms and X' represents an oxygen atom or a 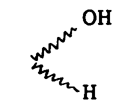 remainder.

4. The products of general formula (I'), as defined in any one of claims 2 or 3 and corresponding to the formulae:
- 10beta-[2-(methylthio)-ethyl]-estra-4,9(11)-diene-3,17-dione,
- 10beta-[2-(ethylthio)-ethyl]-estra-4,9(11)-diene-3,17-dione,
- 10beta-[2-(methylthio)-ethyl]-17beta-hydroxy-estra-4,9(11)-diene-3-one,
- 10beta-[2-(ethenylthio)-ethyl]-estra-4,9(11)-diene-3,17-dione,
- 10beta-[2-(ethynylthio)-ethyl]-estra-4,9(11)-diene-3,17-dione,
- the butane dioate of 10beta-[2-(methylthio)-ethyl]-3-oxo-estra-4,9(11)-dien-17beta-yl and of sodium,
- 10beta-[2-(methylthio)-ethyl]-estra-4,9(11)-diene-3beta, 17beta-diol,
- 10beta-[2-(methylthio)-ethyl]-estra-4,9(11)-diene-3alpha, 17beta-diol,
- 10beta-[2-(methylthio)-ethyl]-estr-9(11)-diene-3,17-dione.

5. Preparation process for the products of general formula (I) as defined in claim 1, and characterized in that a product of formula (II): 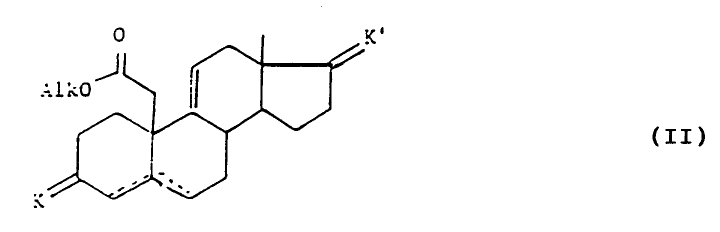 in which K and K', identical or different, represent a protected keto radical, it being understood that when K represents an enol ether, the double bonds are found in position 3(4) and 5(6) and Alk represents an alkyl radical with 1 to 4 carbon atoms, the dotted lines representing a double bond in position 4(5) or 5(6), is subjected to a reduction in order to obtain a product of formula (III): 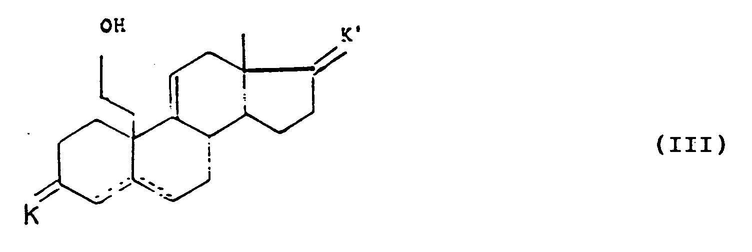 which is treated,
either with a transformation reagent of the hydroxy radical into a thiol radical in order to obtain a product of formula (IV): 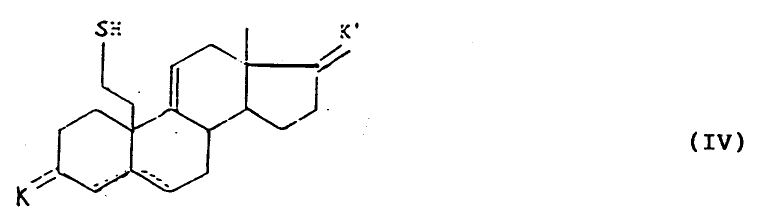 which is treated with a reactive derivative of the R radical in order to obtain a product of formula (V): 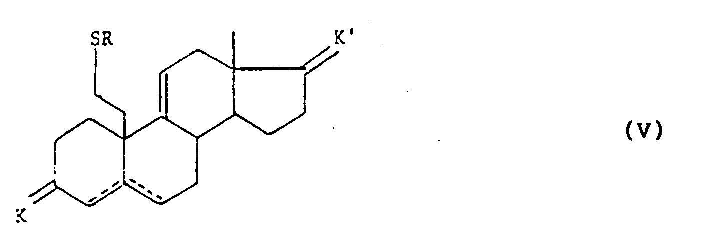 or with a reactive derivative of a sulphonic acid in order to obtain a product of formula (VI): 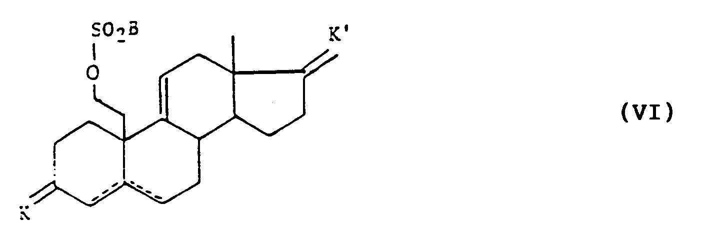 in which B represents the remainder of a sulphonic acid, which product of formula (VI) is treated with a salt of formula RSA in which A represents a monovalent cation, in order to obtain a product of formula (V) as defined above, which product of formula (V) is treated with a deprotection reagent of the protected ketone functions in order to obtain a product of formula (Ia): 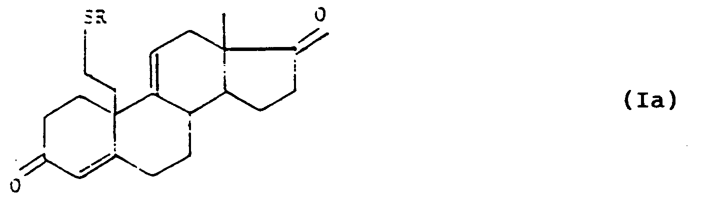 corresponding to the products of formula (I) in which R has the meanings indicated above, X and Y each represents an oxygen atom, n represents the number 0, and the dotted lines in position 4(5) represent a double bond and the dotted lines in positions 1(2) and 6(7) do not represent a second bond between the carbons that carry them, and the dotted lines in position 4(5) represent a second bond between the carbons which carry them, which products of formula (Ia) are, if desired, subjected to one or more of the following reactions and in any order:
- introduction of a double bond in position 1(2)
- introduction of a double bond in position 6(7)
- oxidation of the sulphur atom of the -SR radical into sulphoxide,
- selective reduction of the double bond in position 4(5)
- introduction of an alkyl radical in position 16
- introduction of an optionally substituted alkylthio or arylthio radical in position 7,
in order to obtain the products of formula (Ib): 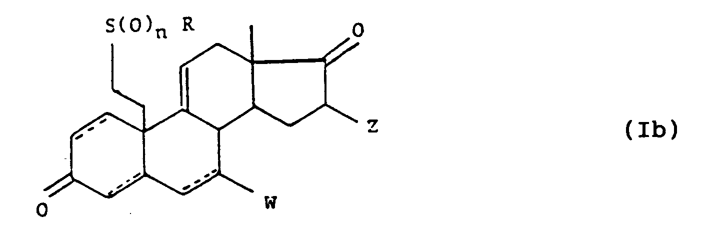 in which n represents the integers 0 or 1 and the dotted lines in position 1(2), 4(5) and 6(7) indicate the optional presence of a double bond between the carbons that carry them and corresponding to the products of formula (I) in which R has the meanings indicated above, X and Y each represents an oxygen atom and W, Z and n and the dotted lines in positions 1(2), 4(5) and 6(7) have the previous meanings, with the exception of the products of formula (Ib) in which Z = H, W = H, n = 0 and in which the dotted lines in position 1(2) and 6(7) do not represent a second bond between the carbons which carry them and if desired the products of formulae (Ia) and (Ib) are subjected to one or more of the following reactions in any order:
- introduction of a 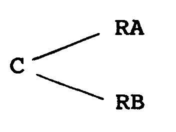 radical
in position 3,
- action of hydroxylamine or of a derivative of formula H₂N-O-R₁,
- reduction of the ketone function in position 3 and/or 17 and optional acylation of the hydroxy function then if desired salification of the esterified function,
- conversion of the ketone function in position 17 into a 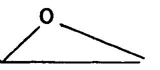 (CH₂)_{q} group in order to obtain a product of formula (Ic): 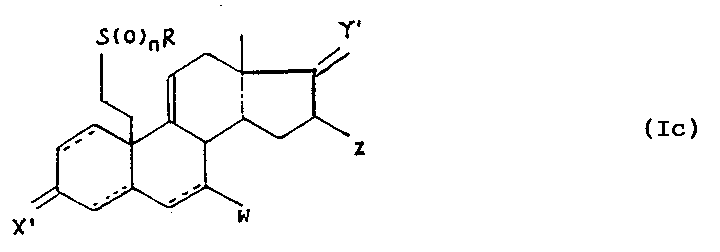 in which n, R, W, Z and the dotted lines in positions 1(2), 4(5) and 6(7) have the meaning indicated above and X' and Y' represent the values indicated above for X and Y, it being understood that at least one of X or Y does not represent an oxygen atom, and it being understood that =X and/or =Y does not represent

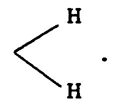

6. Preparation process for the products of formula (I) as defined in claim 1, characterized in that a product of formula (IV) or (I'a): 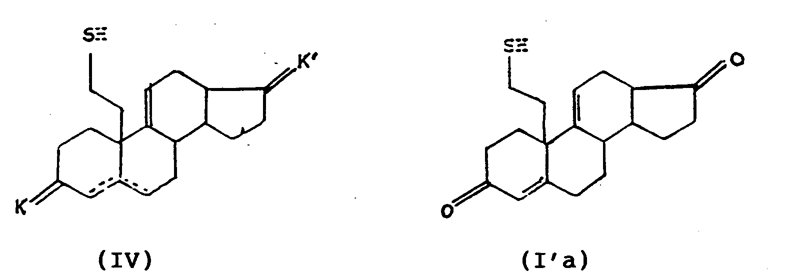 as defined previously, is subjected to the action of a [2a] 4-dinitrophenylsulphenyl halide in order to obtain a product of formula (VII): 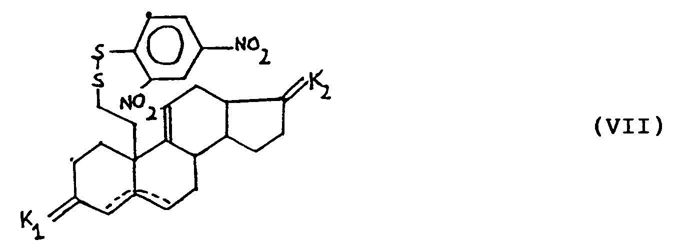 in which K₁ and K₂ both represent a ketone function or both represent a protected ketone function and the dotted line represents a double bond in position 4(5) or 5(6) or 3(4) and 5(6) when K₁ represents a protected ketone function or in position 4(5) when K₁ represents a ketone function, which product of formula (VII)
1) either is subjected, if appropriate, to a deprotection reagent of the ketone functions in position 3 or 17 in order to obtain a product of formula (Id): 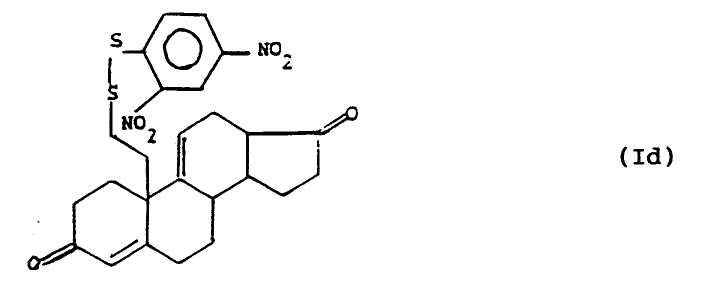
2) or is subjected to the action of an organo-magnesium compound reagent of formula R''-Mg-Hal in which Hal represents a halogen atom and R'' represents an alkyl, alkenyl, alkynyl, or CN radical,
or to the action of a protected organo-lithium compound derivative R''Li then to a deprotection agent of the R'' radical then, if appropriate, to a deprotection reaction of the ketone functions in position 3 and/or 17 in order to obtain a product of formula (Ie): 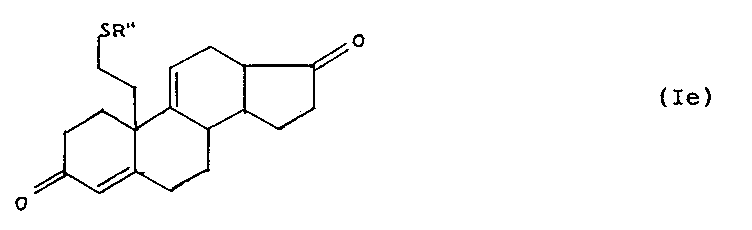 in which R'' has the meaning indicated above,
3) or is subjected to the action of a salt of formula R'''SA in which A has the meaning indicated in claim 5 and R''' represents an alkyl radical having at most 6 carbon atoms or an optionally substituted aryl radical having at most 10 carbon atoms, then if appropriate, to a deprotection reaction of the ketone functions in position 3 and/or 17 in order to obtain a product of formula (If): 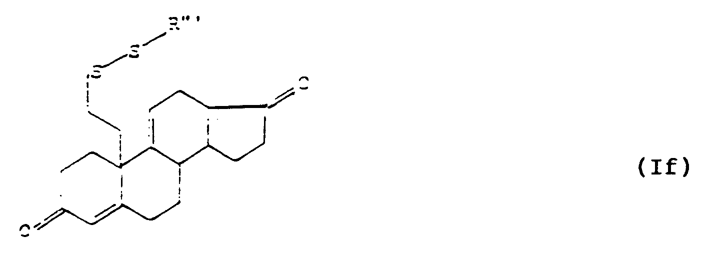 which products of formulae (Id), (Ie), (If) are subjected if desired to one or more of the reactions indicated in claim 5 for the products of formula (Ia) or (Ib), in an any order.

7. Preparation process for products of formula (I) in which =X and/or =Y represents 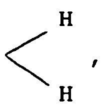 characterized in that a product of formula (II) as defined in claim 5, is subjected to a reducing agent of the protected ketone functions in order to obtain a product of formula (III) in which =K and/or =K' represents hydrogen atoms and the synthesis is continued as indicated in claim 5 or 6.

8. As medicaments, the products of general formula (I) as described in claim 1.

9. As medicaments, the products of general formula (I') as defined in one of claims 2 to 4.

10. Pharmaceutical compositions containing, as active ingredient, at least one of the medicaments defined in one of claims 8 or 9.

11. As industrial products, the products of general formulae (III), (IV), (V) and (VI), as defined in claim 5, characterized in that =K and/or =K' represent ketone functions protected in the form of ethylenedioxy, ethylenedithio or enol ethyl ether radicals of 3-ethoxy-3(4),5(6)-diene type and characterized in that B represents the remainder of a sulphonic acid chosen from a methyl or tolyl radical.

## Claims (Claims for the following Contracting State(s): ES)

1. Preparation process for the products of general formula (I''): 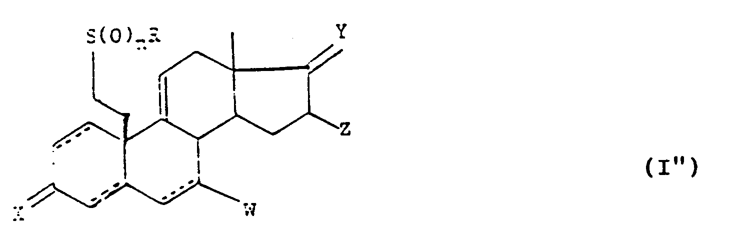 in which R represents:
a hydrogen atom,
an alkyl, alkenyl, alkynyl, alkylthio radical having at most 6 carbon atoms or an optionally substituted arylthio radical having at most 10 carbon atoms,
an acyl radical having at most 12 carbon atoms or a CN radical,
- X represents an oxygen atom, an N-O-R₁ radical in which R₁ represents a hydrogen atom or an alkyl radical having 1 to 6 carbon atoms or X represents 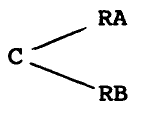 in which RA and RB are identical and represent a hydrogen atom,
or =X represents a 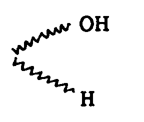 remainder
in which the OH radical can be found in alpha or beta position, the hydroxyl radical being optionally acylated,
- Y represents an oxygen atom, an N-O-R₁ radical in which R₁ has the definition indicated above,
or =Y represents a 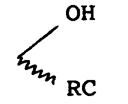 remainder
in which RC represents a hydrogen atom, the hydroxyl radical being optionally acylated, or =Y
represents a group 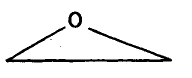 (CH₂)q in which q represents a number from 1 to 3,
- W represents a hydrogen atom an alkylthio or arylthio radical, optionally substituted containing up to 10 carbon atoms,
- Z represents a hydrogen atom or an alkyl radical containing up to 10 carbon atoms,
- n represents a whole number from 0 to 1 and the dotted lines in positions 1(2), 4(5) and 6(7) indicate the optional presence of a second bond between the carbons that carry them, as well as their salts,
it being understood that when n is equal to 1, R represents a methyl radical, X and Y each represent an oxygen atom, W and Z each represent a hydrogen atom, the dotted line in position 4(5) represents a second bond between the carbons which carry them and the dotted lines in position 1(2) and 6(7) do not represent a second bond between the carbons which carry them, characterized in that a product of formula (II): 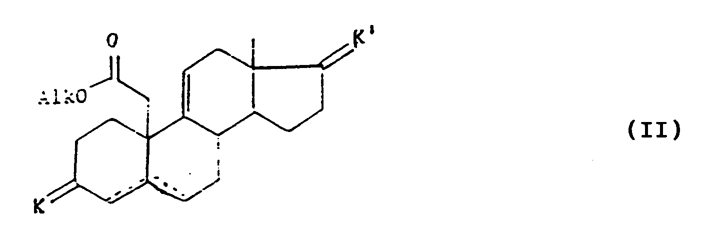 in which K and K', identical or different, represent a protected keto radical, it being understood that when K represents an enol ether, the double bonds are found in position 3(4) and 5(6) and Alk represents an alkyl radical with 1 to 4 carbon atoms, the dotted lines representing a double bond in position 4(5) or 5(6), is subjected to a reduction in order to obtain a product of formula (III): 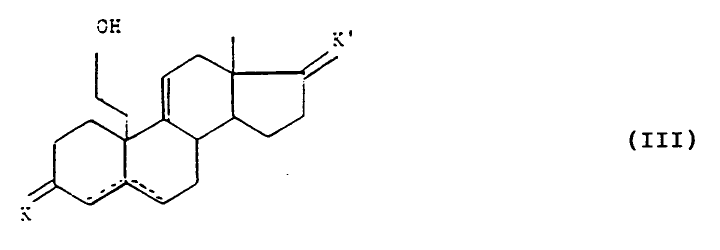 which is treated,
either with a transformation reagent of the hydroxy radical into a thiol radical in order to obtain a product of formula (IV): 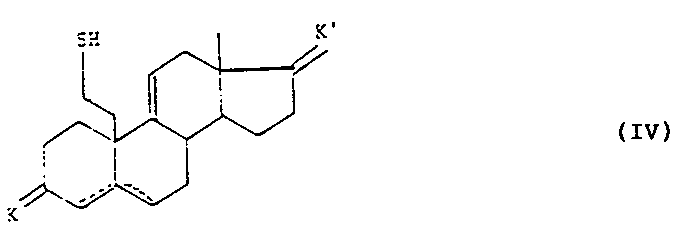 which is treated with a reactive derivative of the R radical in order to obtain a product of formula (V): 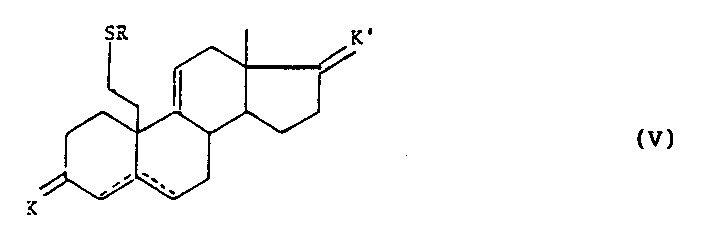 or with a reactive derivative of a sulphonic acid in order to obtain a product of formula (VI): 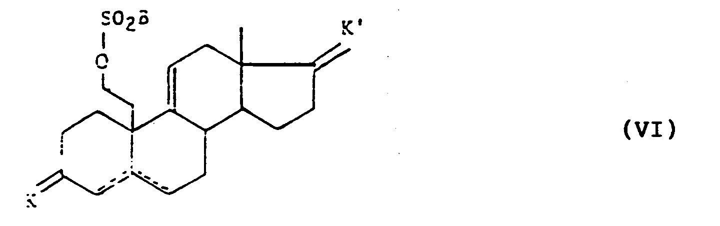 in which B represents the remainder of a sulphonic acid, which product of formula (VI) is treated with a salt of formula RSA in which A represents a monovalent cation, in order to obtain a product of formula (V) as defined above, which product of formula (V) is treated with a deprotection reagent of the protected ketone functions in order to obtain a product of formula (Ia): 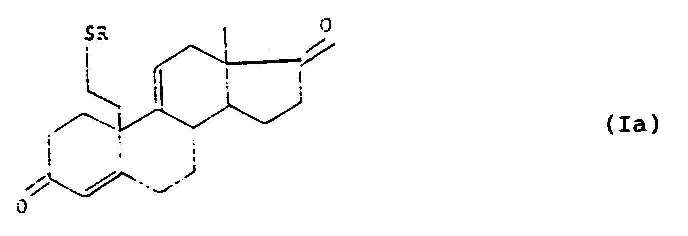 corresponding to the products of formula (I'') in which R has the meanings indicated above, X and Y each represents an oxygen atom, n represents the number 0, and the dotted lines in position 4(5) represent a double bond and the dotted lines in positions 1(2) and 6(7) do not represent a second bond between the carbons that carry them, and the dotted lines in position 4(5) represent a second bond between the carbons which carry them, which products of formula (Ia) are, if desired, subjected to one or more of the following reactions and in any order:
- introduction of a double bond in position 1(2)
- introduction of a double bond in position 6(7)
- oxidation of the sulphur atom of the -SR radical into sulphoxide,
- selective reduction of the double bond in position 4(5)
- introduction of an alkyl radical in position 16
- introduction of an optionally substituted alkylthio or arylthio radical in position 7,
in order to obtain the products of formula (Ib): 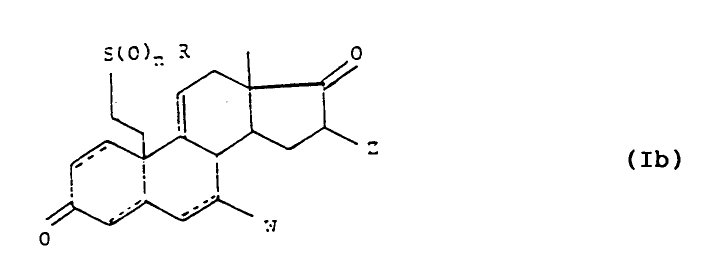 in which n represents the integers 0 or 1 and the dotted lines in position 1(2), 4(5) and 6(7) indicate the optional presence of a double bond between the carbons that carry them and corresponding to the products of formula (I'') in which R has the meanings indicated above, X and Y each represents an oxygen atom and W, Z and n and the dotted lines in positions 1(2), 4(5) and 6(7) have the previous meanings, with the exception of the products of formula (Ib) in which Z = H, W = H, n = 0 and in which the dotted lines in position 1(2) and 6(7) do not represent a second bond between the carbons which carry them and if desired the products of formulae (Ia) and (Ib) are subjected to one or more of the following reactions in any order:
- introduction of a 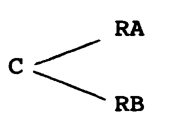 radical
in position 3,
- action of hydroxylamine or of a derivative of formula H₂N-O-R₁,
- reduction of the ketone function in position 3 and/or 17 and optional acylation of the hydroxy function then if desired salification of the esterified function,
- conversion of the ketone function in position 17 into a 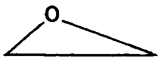 (CH₂)_{q} group in order to obtain a product of formula (Ic): 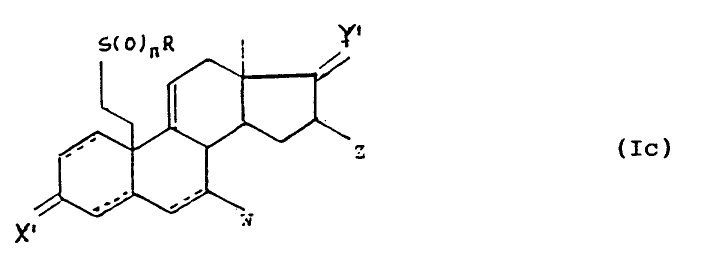 in which n, R, W, Z and the dotted lines in positions 1(2), 4(5) and 6(7) have the meaning indicated above and X' and Y' represent the values indicated above for X and Y, it being understood that at least one of X or Y does not represent an oxygen atom.

2. Preparation process for the products of formula (I'') as defined in claim 1, characterized in that a product of formula (IV) or (I'a): 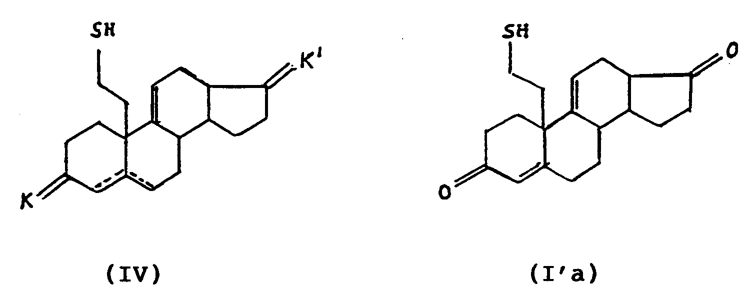 as defined previously, is subjected to the action of a [2a] 4-dinitrophenylsulphenyl halide in order to obtain a product of formula (VII): 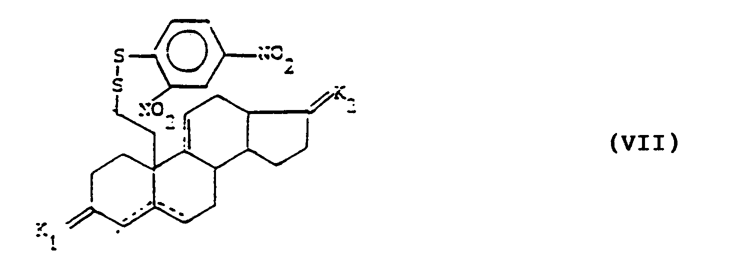 in which K₁ and K₂ both represent a ketone function or both represent a protected ketone function and the dotted line represents a double bond in position 4(5) or 5(6) or 3(4) and 5(6) when K₁ represents a protected ketone function or in position 4(5) when K₁ represents a ketone function, which product of formula (VII)
1) either is subjected, if appropriate, to a deprotection reagent of the ketone functions in position 3 or 17 in order to obtain a product of formula (Id): 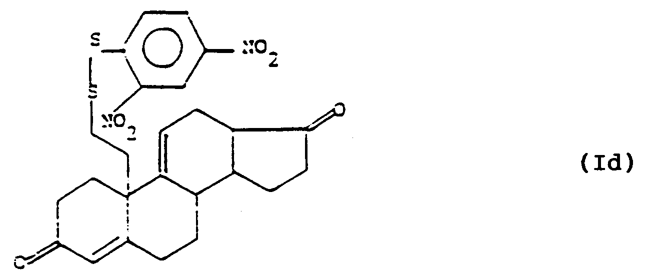
2) or is subjected to the action of an organo-magnesium compound reagent of formula R''-Mg-Hal in which Hal represents a halogen atom and R'' represents an alkyl, alkenyl, alkynyl, or CN radical,
or to the action of a protected organo-lithium compound derivative R''Li then to a deprotection agent of the R'' radical then, if appropriate, to a deprotection reaction of the ketone functions in position 3 and/or 17 in order to obtain a product of formula (Ie): 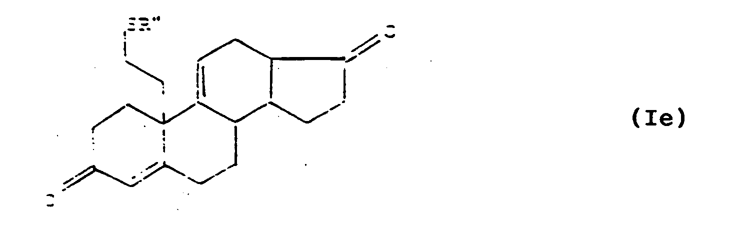 in which R'' has the meaning indicated above,
3) or is subjected to the action of a salt of formula R'''SA in which A has the meaning indicated in claim 1 and R''' represents an alkyl radical having at most 6 carbon atoms or an optionally substituted aryl radical having at most 10 carbon atoms, then if appropriate, to a deprotection reaction of the ketone functions in position 3 and/or 17 in order to obtain a product of formula (If): 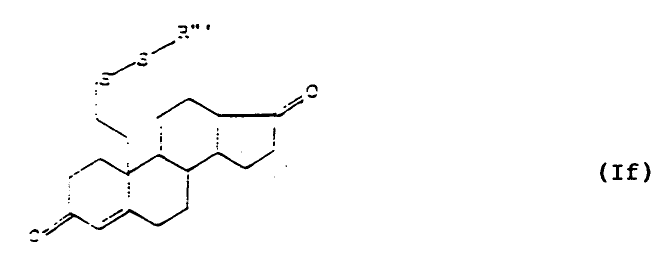 which products of formulae (Id), (Ie), (If) are subjected if desired to one or more of the reactions indicated in claim 1 for the products of formula (Ia) or (Ib), in an any order.

3. Preparation process for products corresponding to those of formula (I'') in which =X and/or =Y
represents 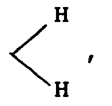 characterized in that a product of formula (II) as defined in claim 1, is subjected to a reducing agent of the protected ketone functions in order to obtain a product of formula (III) in which =K and/or =K' represents hydrogen atoms and the synthesis is continued as indicated in claim 1 or 2.

4. Process according to any one of claims 1 to 3, characterized in that products of formula (I') are prepared: 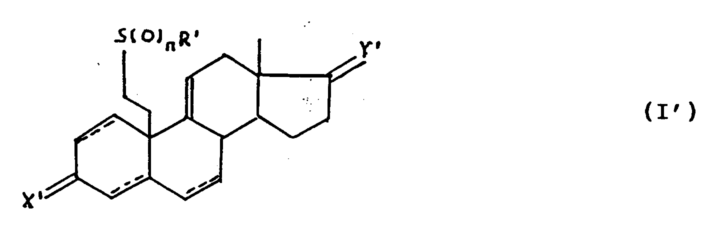 in which R' represents:
an alkyl, alkenyl, alkynyl or alkylthio radical having at most 4 carbon atoms,
a CN radical,
an acyl radical having at most 4 carbon atoms,
- X' represents an oxygen atom, a CH₂ radical, an N-O-R'₁ radical in which R₁ represents a hydrogen atom or an alkyl radical having 1 to 4 carbon atoms, or X' represents a 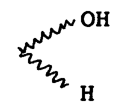 remainder
- Y' represents an oxygen atom or =Y' represents a 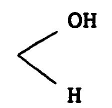 remainder, the hydroxy radical being optionally acylated in the form of an alkanoyloxy radical or an 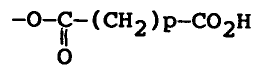 radical, in which p represents an integer from 2 to 5.
the dotted lines in positions 1(2), 4(5) and 6(7) indicate the optional presence of a second bond between the carbons which carry them, as well as their salts.

5. Process according to claim 4 characterized in that products in which R' is chosen from the alkyl, alkenyl or alkynyl radicals having from 1 to 4 carbon atoms and X' represents an oxygen atom or a 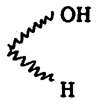 remainder are prepared.

6. Process according to claim 4 characterized in that products corresponding to the following formulae are prepared:
- 10beta-[2-(methylthio)-ethyl]-estra-4,9(11)-diene-3,17-dione,
- 10beta-[2-(ethylthio)-ethyl]-estra-4,9(11)-diene-3,17-dione,
- 10beta-[2-(methylthio)-ethyl]-17beta-hydroxy-estra-4,9(11)-diene-3-one,
- 10beta-[2-(ethenylthio)-ethyl]-estra-4,9(11)-diene-3,17-dione,
- 10beta-[2-(ethynylthio)-ethyl]-estra-4,9(11)-diene-3,17-dione,
- the butane dioate of 10beta-[2-(methylthio)-ethyl]-3-oxo-estra-4,9(11)-dien-17beta-yl and of sodium,
- 10beta-[2-(methylthio)-ethyl]-estra-4,9(11)-diene-3beta, 17beta-diol,
- 10beta-[2-(methylthio)-ethyl]-estra-4,9(11)-diene-3alpha, 17beta-diol,
- 10beta-[2-(methylthio)-ethyl]-estr-9(11)-diene-3,17-dione.

## Claims (Claims for the following Contracting State(s): GR)

1. Preparation process for the products of general formula (I''): 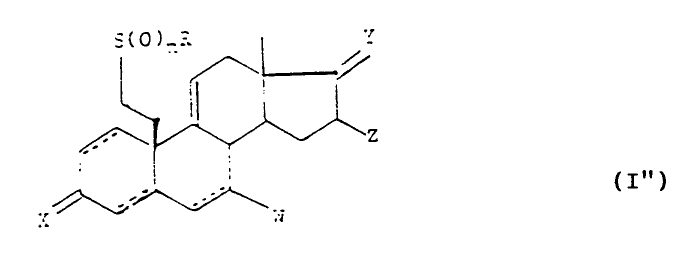 in which R represents:
a hydrogen atom,
an alkyl, alkenyl, alkynyl, alkylthio radical having at most 6 carbon atoms or an optionally substituted arylthio radical having at most 10 carbon atoms,
an acyl radical having at most 12 carbon atoms or a CN radical,
- X represents an oxygen atom, an N-O-R₁ radical in which R₁ represents a hydrogen atom or an alkyl radical having 1 to 6 carbon atoms or X represents 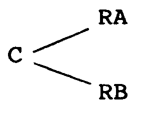 in which RA and RB are identical and represent a hydrogen atom,
or =X represents a 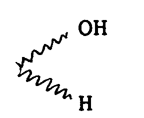 remainder
in which the OH radical can be found in alpha or beta position, the hydroxyl radical being optionally acylated,
- Y represents an oxygen atom, an N-O-R₁ radical in which R₁ has the definition indicated above,
or =Y represents a 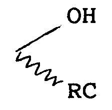 remainder
in which RC represents a hydrogen atom, the hydroxyl radical being optionally acylated, or =Y
represents a group 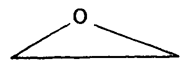 (CH₂)q in which q represents a number from 1 to 3,
- W represents a hydrogen atom an alkylthio or arylthio radical, optionally substituted containing up to 10 carbon atoms,
- Z represents a hydrogen atom or an alkyl radical containing up to 10 carbon atoms,
- n represents a whole number from 0 to 1 and the dotted lines in positions 1(2), 4(5) and 6(7) indicate the optional presence of a second bond between the carbons that carry them, as well as their salts,
it being understood that when n is equal to 1, R represents a methyl radical, X and Y each represent an oxygen atom, W and Z each represent a hydrogen atom, the dotted line in position 4(5) represents a second bond between the carbons which carry them and the dotted lines in position 1(2) and 6(7) do not represent a second bond between the carbons which carry them, characterized in that a product of formula (II): 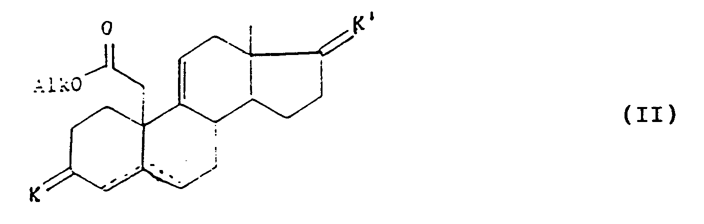 in which K and K', identical or different, represent a protected keto radical, it being understood that when K represents an enol ether, the double bonds are found in position 3(4) and 5(6) and Alk represents an alkyl radical with 1 to 4 carbon atoms, the dotted lines representing a double bond in position 4(5) or 5(6), is subjected to a reduction in order to obtain a product of formula (III): 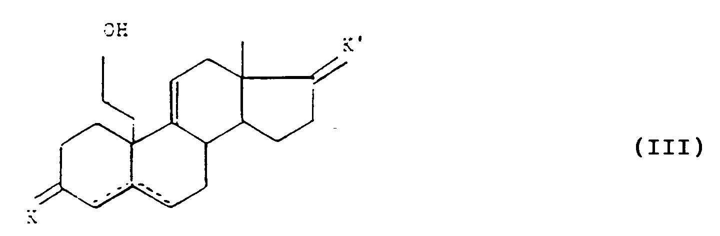 which is treated,
either with a transformation reagent of the hydroxy radical into a thiol radical in order to obtain a product of formula (IV): 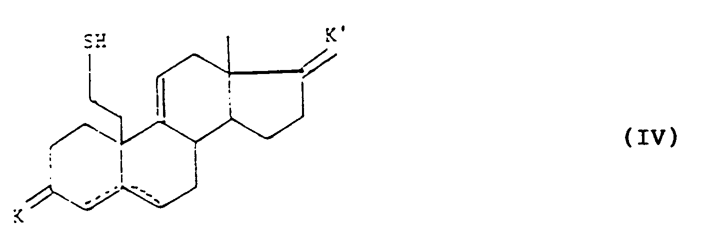 which is treated with a reactive derivative of the R radical in order to obtain a product of formula (V): 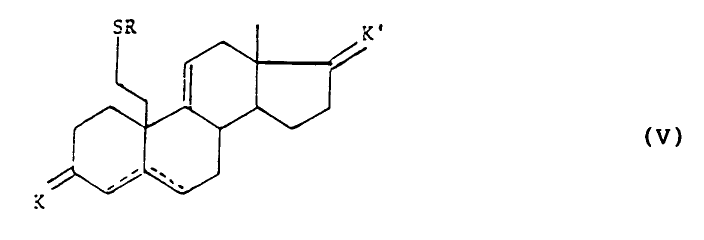 or with a reactive derivative of a sulphonic acid in order to obtain a product of formula (VI): 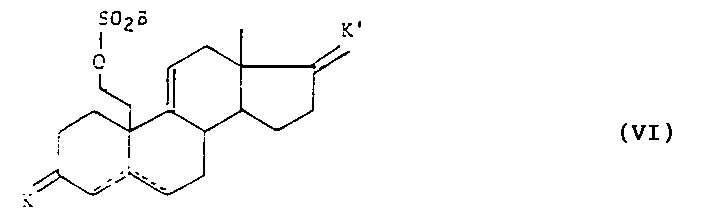 in which B represents the remainder of a sulphonic acid, which product of formula (VI) is treated with a salt of formula RSA in which A represents a monovalent cation, in order to obtain a product of formula (V) as defined above, which product of formula (V) is treated with a deprotection reagent of the protected ketone functions in order to obtain a product of formula (Ia): 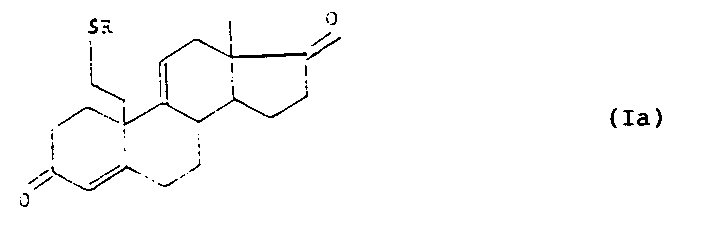 corresponding to the products of formula (I'') in which R has the meanings indicated above, X and Y each represents an oxygen atom, n represents the number 0, and the dotted lines in position 4(5) represent a double bond and the dotted lines in positions 1(2) and 6(7) do not represent a second bond between the carbons that carry them, and the dotted lines in position 4(5) represent a second bond between the carbons which carry them, which products of formula (Ia) are, if desired, subjected to one or more of the following reactions and in any order:
- introduction of a double bond in position 1(2)
- introduction of a double bond in position 6(7)
- oxidation of the sulphur atom of the -SR radical into sulphoxide,
- selective reduction of the double bond in position 4(5)
- introduction of an alkyl radical in position 16
- introduction of an optionally substituted alkylthio or arylthio radical in position 7,
in order to obtain the products of formula (Ib): 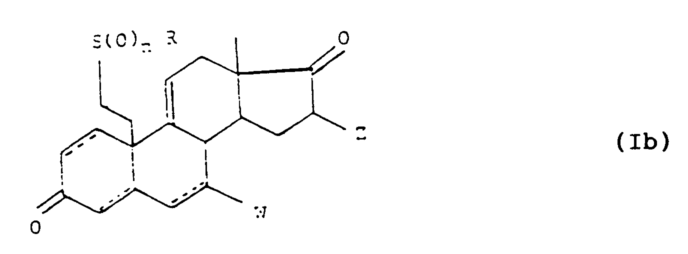 in which n represents the integers 0 or 1 and the dotted lines in position 1(2), 4(5) and 6(7) indicate the optional presence of a double bond between the carbons that carry them and corresponding to the products of formula (I'') in which R has the meanings indicated above, X and Y each represents an oxygen atom and W, Z and n and the dotted lines in positions 1(2), 4(5) and 6(7) have the previous meanings, with the exception of the products of formula (Ib) in which Z = H, W = H, n = 0 and in which the dotted lines in position 1(2) and 6(7) do not represent a second bond between the carbons which carry them and if desired the products of formulae (Ia) and (Ib) are subjected to one or more of the following reactions in any order:
- introduction of a 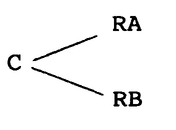 radical
in position 3,
- action of hydroxylamine or of a derivative of formula H₂N-O-R₁,
- reduction of the ketone function in position 3 and/or 17 and optional acylation of the hydroxy function then if desired salification of the esterified function,
- conversion of the ketone function in position 17 into a 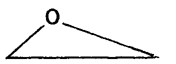 (CH₂)_{q} group in order to obtain a product of formula (Ic): 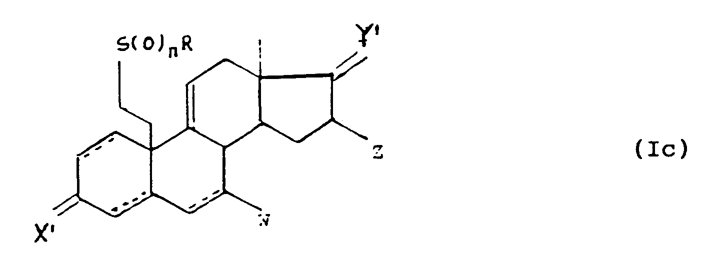 in which n, R, W, Z and the dotted lines in positions 1(2), 4(5) and 6(7) have the meaning indicated above and X' and Y' represent the values indicated above for X and Y, it being understood that at least one of X or Y does not represent an oxygen atom.

2. Preparation process for the products of formula (I'') as defined in claim 1, characterized in that a product of formula (IV) or (I'a): 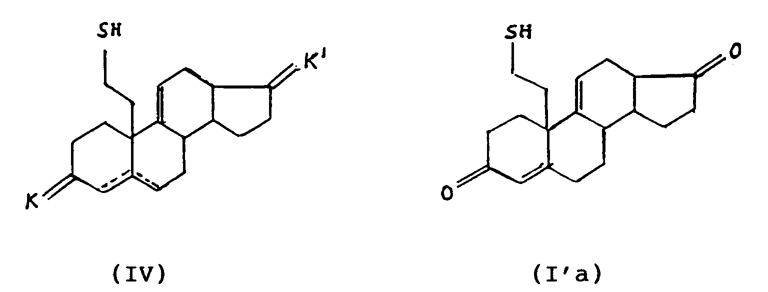 as defined previously, is subjected to the action of a [2a] 4-dinitrophenylsulphenyl halide in order to obtain a product of formula (VII): 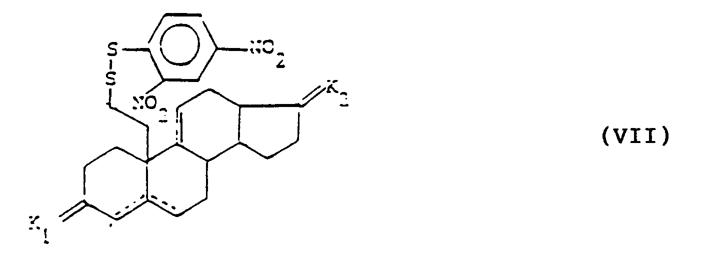 in which K₁ and K₂ both represent a ketone function or both represent a protected ketone function and the dotted line represents a double bond in position 4(5) or 5(6) or 3(4) and 5(6) when K₁ represents a protected ketone function or in position 4(5) when K₁ represents a ketone function, which product of formula (VII)
1) either is subjected, if appropriate, to a deprotection reagent of the ketone functions in position 3 or 17 in order to obtain a product of formula (Id): 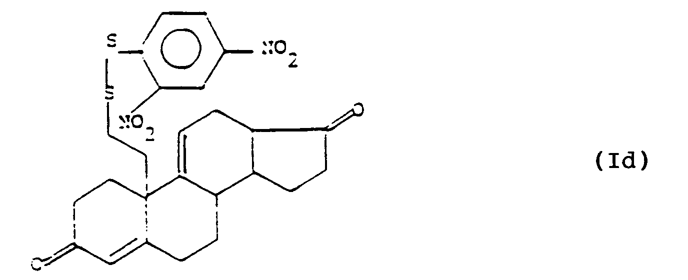
2) or is subjected to the action of an organo-magnesium compound reagent of formula R''-Mg-Hal in which Hal represents a halogen atom and R'' represents an alkyl, alkenyl, alkynyl, or CN radical,
or to the action of a protected organo-lithium compound derivative R''Li then to a deprotection agent of the R'' radical then, if appropriate, to a deprotection reaction of the ketone functions in position 3 and/or 17 in order to obtain a product of formula (Ie): 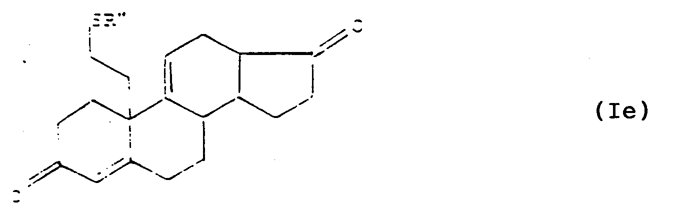 in which R'' has the meaning indicated above,
3) or is subjected to the action of a salt of formula R'''SA in which A has the meaning indicated in claim 1 and R''' represents an alkyl radical having at most 6 carbon atoms or an optionally substituted aryl radical having at most 10 carbon atoms, then if appropriate, to a deprotection reaction of the ketone functions in position 3 and/or 17 in order to obtain a product of formula (If): 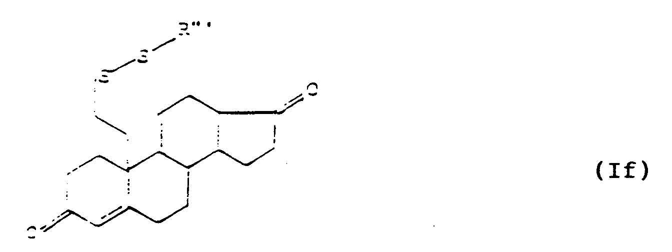 which products of formulae (Id), (Ie), (If) are subjected if desired to one or more of the reactions indicated in claim 1 for the products of formula (Ia) or (Ib), in an any order.

3. Preparation process for products corresponding to those of formula (I'') in which =X and/or =Y
represents 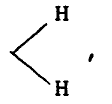 characterized in that a product of formula (II) as defined in claim 1, is subjected to a reducing agent of the protected ketone functions in order to obtain a product of formula (III) in which =K and/or =K' represents hydrogen atoms and the synthesis is continued as indicated in claim 1 or 2.

4. Process according to any one of claims 1 to 3, characterized in that products of formula (I') are prepared: 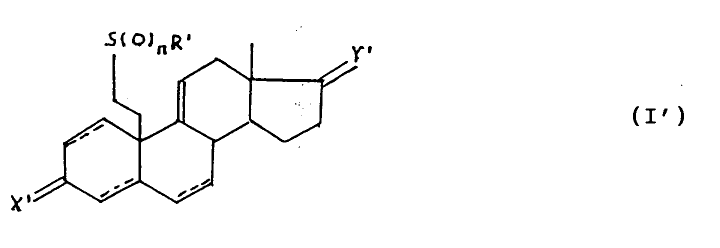 in which R' represents:
an alkyl, alkenyl, alkynyl or alkylthio radical having at most 4 carbon atoms,
a CN radical,
an acyl radical having at most 4 carbon atoms,
- X' represents an oxygen atom, a CH₂ radical, an N-O-R'₁ radical in which R₁ represents a hydrogen atom or an alkyl radical having 1 to 4 carbon atoms, or X' represents a 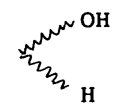 remainder
- Y' represents an oxygen atom or =Y' represents a 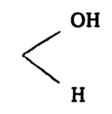 remainder, the hydroxy radical being optionally acylated in the form of an alkanoyloxy radical or an 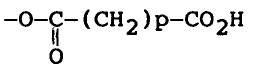 radical, in which p represents an integer from 2 to 5.
the dotted lines in positions 1(2), 4(5) and 6(7) indicate the optional presence of a second bond between the carbons which carry them, as well as their salts.

5. Process according to claim 4 characterized in that products in which R' is chosen from the alkyl, alkenyl or alkynyl radicals having from 1 to 4 carbon atoms and X' represents an oxygen atom or a 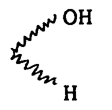 remainder are prepared.

6. Process according to claim 4 characterized in that products corresponding to the following formulae are prepared:
- 10beta-[2-(methylthio)-ethyl]-estra-4,9(11)-diene-3,17-dione,
- 10beta-[2-(ethylthio)-ethyl]-estra-4,9(11)-diene-3,17-dione,
- 10beta-[2-(methylthio)-ethyl]-17beta-hydroxy-estra-4,9(11)-diene-3-one,
- 10beta-[2-(ethenylthio)-ethyl]-estra-4,9(11)-diene-3,17-dione,
- 10beta-[2-(ethynylthio)-ethyl]-estra-4,9(11)-diene-3,17-dione,
- the butane dioate of 10beta-[2-(methylthio)-ethyl]-3-oxo-estra-4,9(11)-dien-17beta-yl and of sodium,
- 10beta-[2-(methylthio)-ethyl]-estra-4,9(11)-diene-3beta, 17beta-diol,
- 10beta-[2-(methylthio)-ethyl]-estra-4,9(11)-diene-3alpha, 17beta-diol,
- 10beta-[2-(methylthio)-ethyl]-estr-9(11)-diene-3,17-dione.

7. Preparation process for pharmaceutical compositions characterized in that at least one of the compounds of formula (I'') as defined in claim 1 or at least one of their pharmaceutically acceptable salts are used as active ingredient, in a form intended for this use.

8. Preparation process for pharmaceutical compositions characterized in that at least one of the compounds of formula (I'') or the compounds corresponding to those of formula (I'') in which =X and/or =Y represent two hydrogen atoms as defined in claims 2 to 5 or at least one of their pharmaceutically acceptable salts are used as active ingredient, in a form intended for this use.

9. Process according to claim 7 or 8, characterized in that the compound of formula (I) is chosen from the products corresponding to formulae:
- 10beta-[2-(methylthio)-ethyl]-estra-4,9(11)-diene-3,17-dione,
- 10beta-[2-(ethylthio)-ethyl]-estra-4,9(11)-diene-3,17-dione,
- 10beta-[2-(methylthio)-ethyl]-17beta-hydroxy-estra-4,9(11)-diene-3-one,
- 10beta-[2-(ethenylthio)-ethyl]-estra-4,9(11)-diene-3,17-dione,
- 10beta-[2-(ethynylthio)-ethyl]-estra-4,9(11)-diene-3,17-dione,
- the butane dioate of 10beta-[2-(methylthio)-ethyl]-3-oxo-estra-4,9(11)-dien-17beta-yl and of sodium,
- 10beta-[2-(methylthio)-ethyl]-estra-4,9(11)-diene-3beta, 17beta-diol,
- 10beta-[2-(methylthio)-ethyl]-estra-4,9(11)-diene-3alpha, 17beta-diol,
- 10beta-[2-(methylthio)-ethyl]-estr-9(11)-diene-3,17-dione.

10. As industrial products, the products of general formulae (III), (IV), (V) and (VI), as defined in claim 1, characterized in that =K and/or =K' represent ketone functions protected in the form of ethylenedioxy, ethylenedithio or enol ethyl ether radicals of 3-ethoxy-3(4),5(6)-diene type and characterized in that B represents the remainder of a sulphonic acid chosen from a methyl or tolyl radical.

## Patentansprüche (Patentansprüche für folgende(n) Vertragsstaat(en): DE, GB, FR, IT, NL, SE, LI, CH, BE, AT, LU, DK)

1. Produkte der allgemeinen Formel (I) 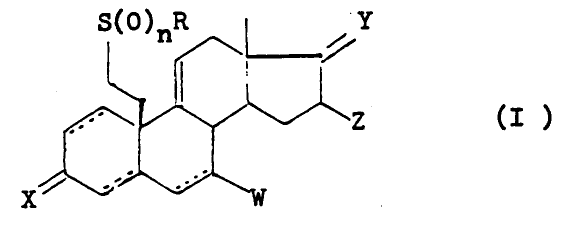 worin
R bedeutet: ein Wasserstoffatom, einen Alkyl-, Alkenyl-, Alkinyl-, Alkylthiorest mit höchstens 6 Kohlenstoffatomen oder einen gegebenenfalls substituierten Arylthiorest mit höchstens 10 Kohlenstoffatomen, einen Acylrest mit höchstens 12 Kohlenstoffatomen oder einem Rest CN,
X ein Sauerstoffatom, einen Rest N-O-R₁ bedeutet, worin R₁ ein Wasserstoffatom oder einen Alkylrest mit 1 bis 6 Kohlenstoffatomen darstellt, oder X für 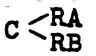 steht, worin RA und RB identisch sind und ein Wasserstoffatom darstellen, oder =X für 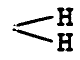 oder einen Rest 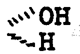 steht,worin der Rest OH sich in der α- oder β-Stellung befinden kann, wobei die Hydroxylgruppe gegebenenfalls acyliert ist,
Y ein Sauerstoffatom, einen Rest N-O-R₁, worin R₁ die vorstehend angegebene Bedeutung hat, bedeutet,oder =Y für 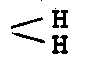 oder einen Rest 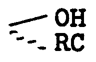 steht,worin RC ein Wasserstoffatom bedeutet und wobei die Hydroxylgruppe gegebenenfalls acyliert ist, oder =Y für eine Gruppe 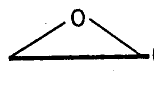 (CH₂)_{q} steht, in der q für eine Zahl von 1 bis 3 steht,
W ein Wasserstoffatom oder einen gegebenenfalls substituierten Alkylthio- oder Arylthiorest mit bis zu 10 Kohlenstoffatomen bedeutet,
Z für ein Wasserstoffatom oder einen Alkylrest mit bis zu 10 Kohlenstoffatomen steht,
n eine ganze Zahl von 0 bis 1 darstellt und die gestrichelten Linien in den 1(2)-, 4(5)- und 6(7)-Positionen die etwaige Anwesenheit einer zweiten Bindung zwischen den sie tragenden Kohlenstoffen anzeigen,
sowie deren Salze,
mit der Maßgabe, daß n für 1 steht, R einen Methylrest bedeutet, X und Y jeweils ein Sauerstoffatom darstellen, W und Z jeweils ein Wasserstoffatom bedeuten, die gestrichelte Linie in 4(5)-Stellung eine zweite Bindung zwischen den sie tragenden Kohlenstoffen darstellt und die gestrichelten Linien in 1(2)- und 6(7)-Stellung keine zweite Bindung zwischen den sie tragenden Kohlenstoffen aufweisen.

2. Produkte der allgemeinen Formel (I), wie in Anspruch 1 definiert, entsprechend der allgemeinen Formel (I') 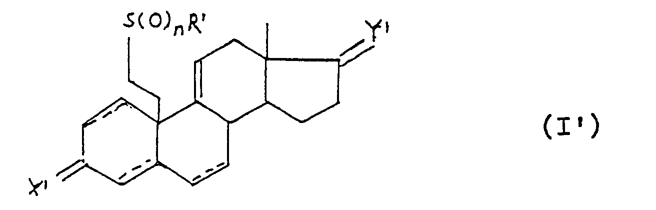 worin R' bedeutet:
einen Alkyl-, Alkenyl-, Alkinyl- oder Alkylthiorest mit höchstens 4 Kohlenstoffatomen,
einen Rest CN,
einen Acylrest mit höchstens 4 Kohlenstoffatomen,
X' ein Sauerstoffatom, einen Rest CH₂, einen Rest N-O-R'₁, worin R'₁ für ein Wasserstoffatom oder einen Alkylrest mit 1 bis 4 Kohlenstoffatomen steht, bedeutet oder X' einen Rest 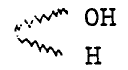 wiedergibt,
Y' ein Wasserstoffatom bedeutet oder =Y' einen Rest 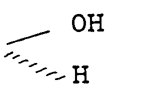 wiedergibt, wobei die Hydroxygruppe gegebenenfalls in Form eines Alkanoyloxyrestes oder eines Restes 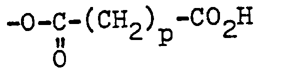 acyliert sein kann, worin p eine Zahl von 2 bis 5 bedeutet, die gestrichelten Linien in 1(2)-, 4(5)- und 6(7)-Position die etwaige Anwesenheit einer zweiten Bindung zwischen den sie tragenden Kohlenstoffen anzeigen, sowie deren Salze.

3. Produkte der Formel (I'), wie in Anspruch 2 definiert, worin R' ausgewählt ist unter den Alkyl-, Alkenyl- oder Alkinylresten mit 1 bis 4 Kohlenstoffatomen und X' ein Sauerstoffatom oder einen Rest 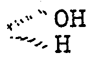 darstellt.

4. Produkte der allgemeinen Formel(I'), wie in einem der Ansprüche 2 oder 3 definiert und den folgenden Bezeichnungen entsprechend:
10β-[2-(Methylthio)-ethyl]-östra-4,9(11)-dien-3,17-dion,
10β-[2-(Ethylthio)-ethyl]-östra-4,9(11)-dien-3,17-dion,
10β-[2-(Methylthio)-ethyl]-17β-hydroxy-östra-4,9(11)-dien-3-on,
10β-[2-(Ethenylthio)-ethyl]-östra-4,9(11)-dien-3,17-dion,
10β-[2-(Ethinylthio)-ethyl]-östra-4,9(11)-dien-3,17-dion,
10β-[2-(Methylthio)-ethyl]-3-oxo-östra-4,9(11)-dien-17β-yl-natriumbutandioat,
10β-[2-(Methylthio)-ethyl]-östra-4,9(11)-dien-3β, 17β-diol,
10β-[2-(Methylthio)-ethyl]-östra-4,9(11)-dien-3α, 17β-diol,
10β-[2-(Methylthio)-ethyl]-östr-9(11)-en-3,17-dion.

5. Verfahren zur Herstellung der Produkte der allgemeinen Formel (I), wie in Anspruch 1 definiert, dadurch gekennzeichnet, daß man ein Produkt der Formel (II) 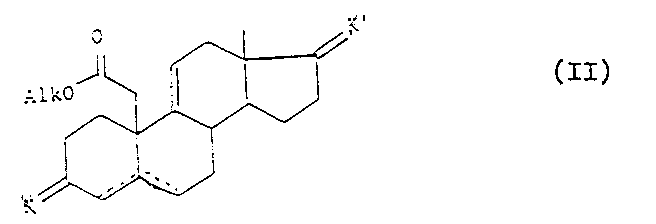 worin K und K',identisch oder voneinander verschieden, eine geschützte Ketogruppe bedeuten, mit der Maßgabe, daß, wenn K einen Enolether darstellt, sich die Doppelbindungen in der 3(4)- und 5(6)-Position befinden, und Alk für einen Alkylrest mit 1 bis 4 Kohlenstoffatomen steht, wobei die gestrichelten Linien eine Doppelbindung in der 4(5)- oder 5(6)-Position bedeuten, einer Reduktion unterzieht, um zu einem Produkt der Formel (III) 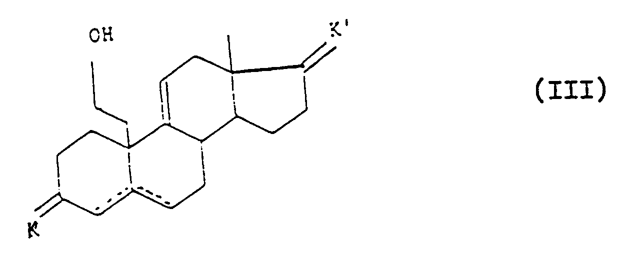 zu gelangen, welches man behandelt,
entweder mit einem Reagens für die Überführung der Hydroxygruppe in eine Thiolgruppe, um zu einem Produkt der Formel (IV) 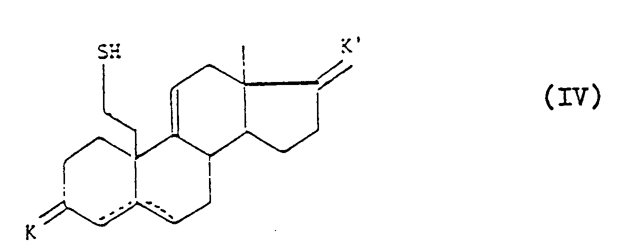 zu gelangen, welches man mit einem reaktiven Derivat des Restes R behandelt, um ein Produkt der Formel (V) 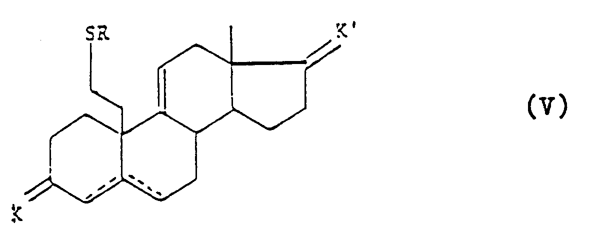 zu erhalten,
oder mit einem reaktiven Derivat einer Sulfonsäure, um zu einem Produkt der Formel (VI) 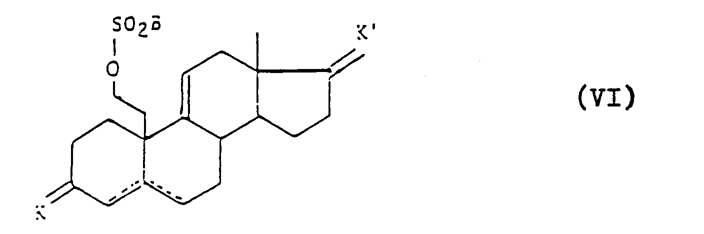 zu gelangen, worin B den Rest einer Sulfonsäure wiedergibt, welches Produkt der Formel (VI) man mit einem Salz der Formel RSA behandelt, worin A ein einwertiges Kation darstellt, um zu einem Produkt der Formel (V), wie vorstehend definiert, zu gelangen, welches Produkt der Formel (V) man mit einem Reagens für die Abspaltung der Schutzgruppen der geschützten Ketonfunktionen behandelt, um zu einem Produkt der Formel (Ia) 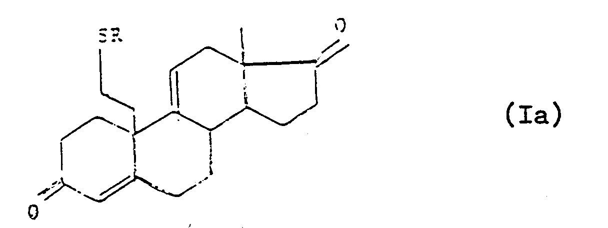 zu gelangen, welches den Produkten der Formel (I ) entspricht, worin R die vorstehend angegebenen Bedeutungen besitzt, X und Y jeweils ein Sauerstoffatom bedeuten, n die Zahl 0 wiedergibt und die gestrichelten Linien in der 4(5)-Position eine Doppelbindung anzeigen und die gestrichelten Linien in den 1(2)- und 6(7)-Positionen keine zweite Bindung zwischen den sie tragenden Kohlenstoffen wiedergeben, wobei man die Produkte der Formel (Ia) gewünschtenfalls einer oder mehreren der folgenden Reaktionen in beliebiger Reihenfolge unterzieht:
- das Einführen einer Doppelbindung in 1(2)-Position
- das Einführen einer Doppelbindung in 6(7)-Position
- die Oxidation des Schwefelatoms des Restes -SR zum Sulfoxid
- die selektive Reduktion der Doppelbindung in 4(5)-Position
- das Einführen eines Alkylrestes in 16-Stellung
- das Einführen eines gegebenenfalls substituierten Alkylthio- oder Arylthiorestes in 7-Stellung,
um zu den Produkten der Formel (Ib) 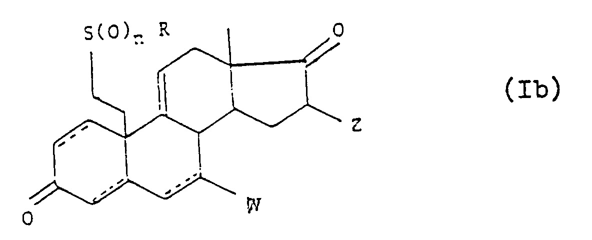 zu gelangen, worin n die ganzen Zahlen 0 oder 1 bedeutet und die gestrichelten Linien in 1(2)-, 4(5)- und 6(7)-Position die etwaige Anwesenheit einer Doppelbindung zwischen den sie tragenden Kohlenstoffen anzeigen, entsprechend den Produkten der Formel (I ), worin R die vorstehend angegebenen Bedeutungen besitzt, X und Y jeweils ein Sauerstoffatom bedeuten und W, Z und n sowie die gestrichelten Linien in 1(2)-, 4(5)-und 6(7)-Position die vorstehend angegebenen Bedeutungen besitzen, mit Ausnahme der Produkte der Formel (Ib), worin Z = H, W = H, n = O und worin die gestrichelten Linien in 1(2)- und 6(7)-Position keine zweite Bindung zwischen den sie tragenden Kohlenstoffen wiedergeben und die gestrichelten Linien in der 4(5)-Position eine zweite Bindung zwischen den sie tragenden Kohlenstoffen wiedergeben, und gewünschtenfalls die Produkte der Formel (Ia) und (Ib) einer oder mehreren der folgenden Reaktionen in beliebiger Reihenfolge unterzieht:
- das Einführen eines Restes 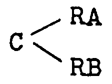 in 3-Stellung
- die Umsetzung mit Hydroxylamin oder einem Derivat der Formel H₂N-O-R₁,
- die Reduktion der Ketonfunktion in 3- und/oder 17-Stellung und die etwaige Acylierung der Hydroxyfunktion und gewünschtenfalls anschließend die Überführung der veresterten Funktion in ein Salz,
- die Umwandlung der Ketonfunktion in 17-Stellung in eine Gruppe 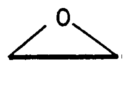 (CH₂)_{q}, um zu einem Produkt der Formel (Ic) 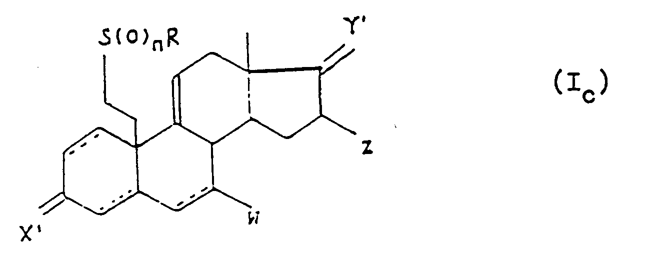 zu gelangen, worin n, R, W, Z und die gestrichelten Linien in 1(2)-, 4(5)- und 6(7)-Position die vorstehend angegebene Bedeutung besitzen und X' und Y' die vorstehend für X und Y angegebenen Bedeutungen besitzen, mit der Maßgabe, daß zumindest eines von X oder Y kein Sauerstoffatom bedeutet, und mit der Maßgabe, daß =X und/oder =Y nicht für 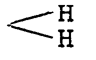 stehen.

6. Verfahren zur Herstellung der Produkte der Formel (I), wie in Anspruch 1 definiert, dadurch gekennzeichnet, daß man ein Produkt der Formel (IV) oder (I'a) 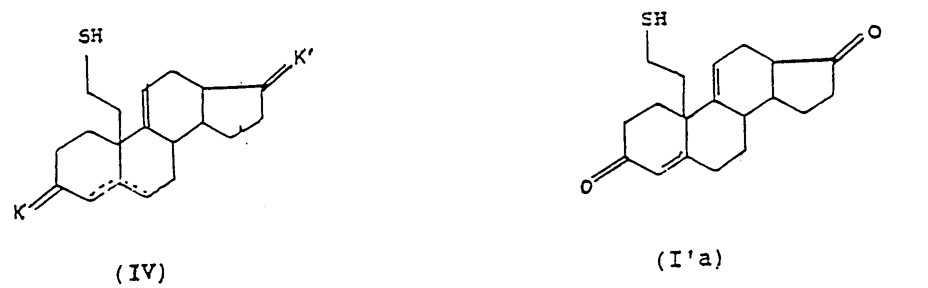 wie vorstehend definiert, der Einwirkung eines 2,4-Dinitrophenylsulfenylhalogenids unterzieht, um zu einem Produkt der Formel (VII) 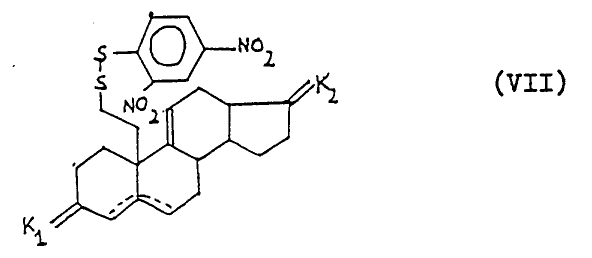 zu gelangen, worin K₁ und K₂ alle beide eine Ketonfunktion oder alle beide eine geschützte Ketonfunktion wiedergeben und die gestrichelte Linie eine Doppelbindung in 4(5)- oder 5(6)- oder 3(4)- und 5(6)-Position wiedergibt, wenn K₁ eine geschützte Ketonfunktion bedeutet, oder in 4(5)-Position, wenn K₁ eine Ketonfunktion bedeutet, welches Produkt der Formel (VII) man
1) entweder gegebenenfalls einem Reagens zur Abspaltung der Schutzgruppen der Ketonfunktionen in 3- oder 17-Stellung unterzieht, um zu einem Produkt der Formel (Id) 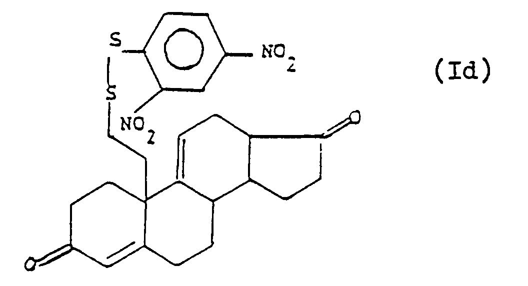 zu gelangen,
2) oder der Einwirkung eines Organomagnesiumreagens der Formel R''-Mg-Hal, worin Hal für ein Halogenatom steht und R'' einen Alkyl-, Alkenyl-, Alkinyl- oder CN-Rest bedeutet, oder der Einwirkung eines geschützten Organolithiumderivats R''Li, danach einem Mittel für die Schutzgruppenentfernung des Restes R'', anschließend gegebenenfalls einer Schutzgruppenabspaltungsreaktion der Ketonfunktionen in 3- und/oder 17-Stellung unterzieht, um zu einem Produkt der Formel (Ie) 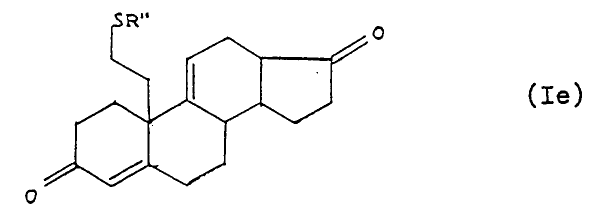 zu gelangen, worin R'' die vorstehend angegebene Bedeutung besitzt,
3) oder der Einwirkung eines Salzes der Formel R'''SA, worin A die in Anspruch 5 angegebene Bedeutung besitzt und R''' einen Alkylrest mit höchstens 6 Kohlenstoffatomen oder einen gegebenenfalls substituierten Arylrest mit höchstens 10 Kohlenstoffatomen bedeutet, danach gegebenenfalls einer Reaktion für die Schutzgruppenabspaltung der Ketonfunktionen in 3- und/oder 17-Stellung unterzieht, um zu einem Produkt der Formel (If) 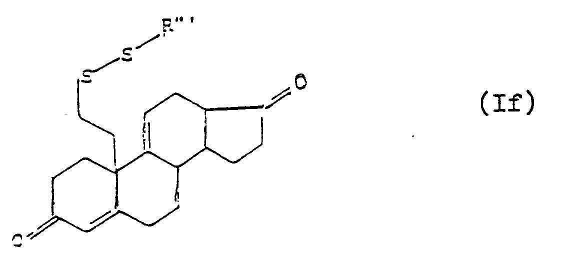 zu gelangen, und die Produkte der Formel (Id), (Ie), (If) gewünschtenfalls einer oder mehreren der in Anspruch 5 für die Produkte der Formel (Ia) oder (Ib) angegebenen Reaktionen in beliebiger Reihenfolge unterzieht.

7. Verfahren zur Herstellung von Produkten der Formel (I), worin =X und/oder =Y für 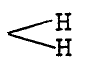 stehen, dadurch gekennzeichnet, daß man ein Produkt der Formel (II), wie in Anspruch 5 definiert, der Einwirkung eines Reduktionsmittels für die geschützten Ketonfunktionen unterzieht, um zu einem Produkt der Formel (III) zu gelangen, worin =K und/oder =K' Wasserstoffatome bedeuten,und die Synthese wie in Anspruch 5 oder 6 angegeben fortführt.

8. Als Arzneimittel die Produkte der allgemeinen Formel (I), wie in Anspruch 1 beschrieben.

9. Als Arzneimittel die Produkte der allgemeinen Formel (I'), wie in einem der Ansprüche 2 bis 4 definiert.

10. Pharmazeutische Zusammensetzungen, enthaltend als Wirkstoff zumindest eines der Arzneimittel, wie in einem der Ansprüche 8 oder 9 definiert.

11. Als industrielle Produkte die Produkte der allgemeinen Formeln (III), (IV), (V) und (VI), wie in Anspruch 5 definiert, dadurch gekennzeichnet, daß =K und/oder =K' in Form der Ethylendioxy-, Ethylendithio-Reste oder in Form des Ethylenolethers vom 3-Ethoxy-3(4),5(6)-dien-Typ geschützte Ketonfunktionen bedeuten, und daß B den Rest einer Sulfonsäure, ausgewählt unter einem Methyl- oder Tolylrest, darstellt.

## Patentansprüche (Patentansprüche für folgende(n) Vertragsstaat(en): ES)

1. Verfahren zur Herstellung von Produkten der allgemeinen Formel (I'') 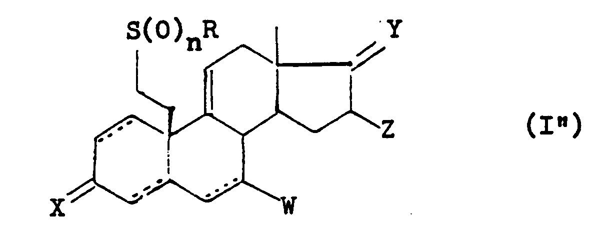 worin
R bedeutet: ein Wasserstoffatom, einen Alkyl-, Alkenyl-, Alkinyl-, Alkylthiorest mit höchstens 6 Kohlenstoffatomen oder einen gegebenenfalls substituierten Arylthiorest mit höchstens 10 Kohlenstoffatomen, einen Acylrest mit höchstens 12 Kohlenstoffatomen oder einen Rest CN,
X ein Sauerstoffatom, einen Rest N-O-R₁ bedeutet, worin R₁ ein Wasserstoffatom oder einen Alkylrest mit 1 bis 6 Kohlenstoffatomen darstellt, oder X für 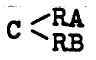 steht, worin RA und RB identisch sind und ein Wasserstoffatom darstellen, oder =X einen Rest 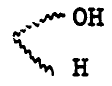 wiedergibt, worin der Rest OH sich in der α- oder β-Stellung befinden kann, wobei die Hydroxylgruppe gegebenenfalls acyliert ist,
Y ein Sauerstoffatom, einen Rest N-O-R₁, worin R₁ die vorstehend angegebene Bedeutung besitzt, bedeutet oder =Y für einen Rest 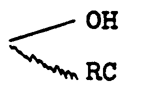 steht, worin RC ein Wasserstoffatom darstellt und wobei die Hydroxylgruppe gegebenenfalls acyliert ist, oder =Y für eine Gruppe 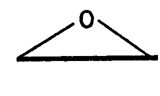 (CH₂)_{q} steht, in der q für eine Zahl von 1 bis 3 steht,
W ein Wasserstoffatom oder einen gegebenenfalls substituierten Alkylthio- oder Arylthiorest mit bis zu 10 Kohlenstoffatomen bedeutet,
Z für ein Wasserstoffatom oder einen Alkylrest mit bis zu 10 Kohlenstoffatomen steht,
n eine ganze Zahl von 0 bis 1 darstellt und die gestrichelten Linien in den 1(2)-, 4(5)- und 6(7)-Positionen die etwaige Anwesenheit einer zweiten Bindung zwischen den sie tragenden Kohlenstoffen anzeigen,
sowie von deren Salzen,
mit der Maßgabe, daß n für 1 steht, R einen Methylrest bedeutet, X und Y jeweils ein Sauerstoffatom darstellen, W und Z jeweils ein Wasserstoffatom bedeuten, die gestrichelte Linie in 4(5)-Stellung eine zweite Bindung zwischen den sie tragenden Kohlenstoffen darstellt und die gestrichelten Linien in 1(2)- und 6(7)-Stellung keine zweite Bindung zwischen den sie tragenden Kohlenstoffen aufweisen,
dadurch gekennzeichnet,
daß man ein Produkt der Formel (II) 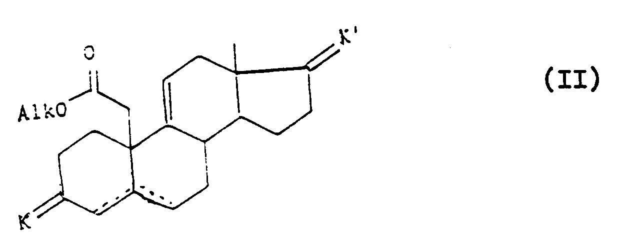 worin K und K',identisch oder voneinander verschieden, eine geschützte Ketogruppe bedeuten, mit der Maßgabe, daß, wenn K einen Enolether darstellt, sich die Doppelbindungen in der 3(4)- und 5(6)-Position befinden, und Alk für einen Alkylrest mit 1 bis 4 Kohlenstoffatomen steht, wobei die gestrichelten Linien eine Doppelbindung in der 4(5)- oder 5(6)-Position aufweisen, einer Reduktion unterzieht, um zu einem Produkt der Formel (III) 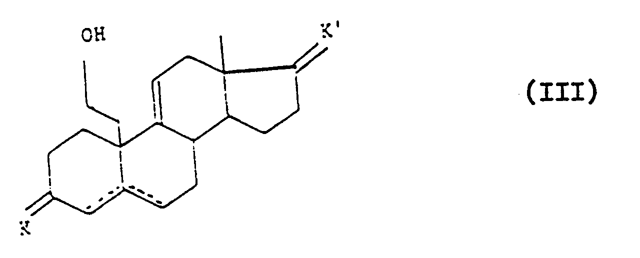 zu gelangen, welches man behandelt,
entweder mit einem Reagens für die Überführung der Hydroxygruppe in eine Thiolgruppe, um zu einem Produkt der Formel (IV) 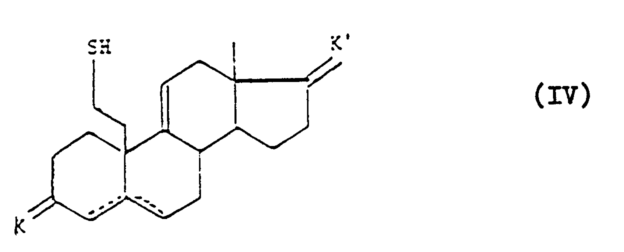 zu gelangen, welches man mit einem reaktiven Derivat des Restes R behandelt, um ein Produkt der Formel (V) 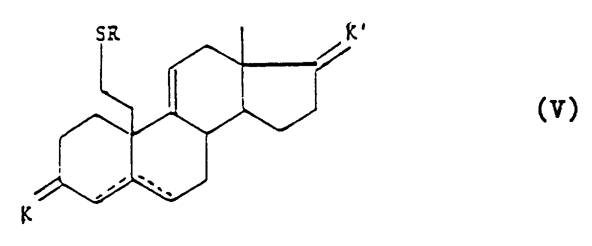 zu erhalten,
oder mit einem reaktiven Derivat einer Sulfonsäure, um zu einem Produkt der Formel (VI) 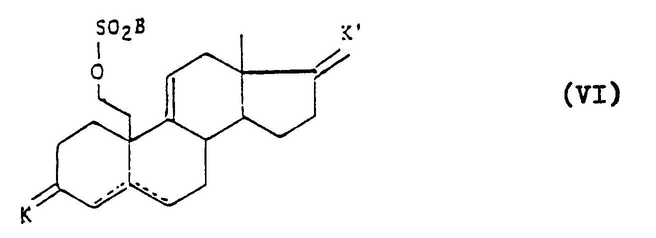 zu gelangen, worin B den Rest einer Sulfonsäure wiedergibt, welches Produkt der Formel (VI) man mit einem Salz der Formel RSA behandelt, worin A ein einwertiges Kation darstellt, um zu einem Produkt der Formel (V), wie vorstehend definiert, zu gelangen, welches Produkt der Formel (V) man mit einem Reagens für die Abspaltung der Schutzgruppen der geschützten Ketonfunktionen behandelt, um zu einem Produkt der Formel (Ia) 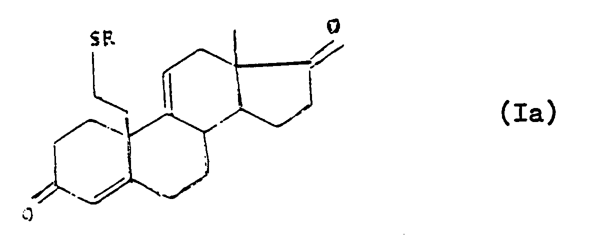 zu gelangen, welches den Produkten der Formel (I'') entspricht, worin R die vorstehend angegebenen Bedeutungen besitzt, X und Y jeweils ein Sauerstoffatom bedeuten, n die Zahl 0 wiedergibt und die gestrichelten Linien in der 4(5)-Position eine Doppelbindung anzeigen und die gestrichelten Linien in den 1(2)- und 6(7)-Positionen keine zweite Bindung zwischen den sie tragenden Kohlenstoffen wiedergeben, wobei man die Produkte der Formel (Ia) gewünschtenfalls einer oder mehreren der folgenden Reaktionen in beliebiger Reihenfolge unterzieht:
- das Einführen einer Doppelbindung in 1(2)-Position
- das Einführen einer Doppelbindung in 6(7)-Position
- die Oxidation des Schwefelatoms des Restes -SR zum Sulfoxid
- die selektive Reduktion der Doppelbindung in 4(5)-Position
- das Einführen eines Alkylrestes in 16-Stellung
- das Einführen eines gegebenenfalls substituierten Alkylthio- oder Arylthiorestes in 7-Stellung,
um zu den Produkten der Formel (Ib) 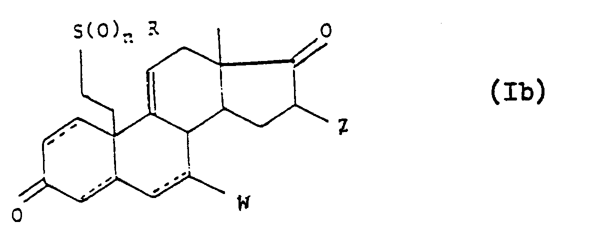 zu gelangen, worin n die ganzen Zahlen 0 oder 1 bedeutet und die gestrichelten Linien in 1(2)-, 4(5)- und 6(7)-Position die etwaige Anwesenheit einer Doppelbindung zwischen den sie tragenden Kohlenstoffen anzeigen, entsprechend den Produkten der Formel (I''), worin R die vorstehend angegebenen Bedeutungen besitzt, X und Y jeweils ein Sauerstoffatom bedeuten und W, Z und n sowie die gestrichelten Linien in 1(2)-, 4(5)-und 6(7)-Position die vorstehend angegebenen Bedeutungen besitzen, mit Ausnahme der Produkte der Formel (Ib), worin Z = H, W = H, n = O und worin die gestrichelten Linien in 1(2)- und 6(7)-Position keine zweite Bindung zwischen den sie tragenden Kohlenstoffen wiedergeben und die gestrichelten Linien in der 4(5)-Position eine zweite Bindung zwischen den sie tragenden Kohlenstoffen wiedergeben, und gewünschtenfalls die Produkte der Formel (Ia) und (Ib) einer oder mehreren der folgenden Reaktionen in beliebiger Reihenfolge unterzieht:
- das Einführen eines Restes 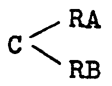 in 3-Stellung
- die Umsetzung mit Hydroxylamin oder einem Derivat der Formel H₂N-O-R₁,
- die Reduktion der Ketonfunktion in 3- und/oder 17-Stellung und die etwaige Acylierung der Hydroxyfunktion und gewünschtenfalls anschließend die Überführung der veresterten Funktion in ein Salz,
- die Umwandlung der Ketonfunktion in 17-Stellung in eine Gruppe 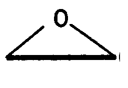 (CH₂)_{q}, um zu einem Produkt der Formel (Ic) 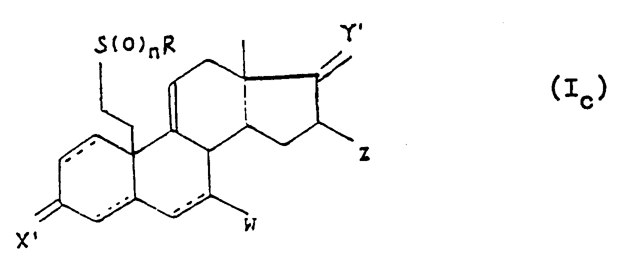 zu gelangen, worin n, R, W, Z und die gestrichelten Linien in 1(2)-, 4(5)- und 6(7)-Position die vorstehend angegebene Bedeutung besitzen und X' und Y' die vorstehend für X und Y angegebenen Bedeutungen besitzen, mit der Maßgabe, daß zumindest eines von X oder Y kein Sauerstoffatom bedeutet.

2. Verfahren zur Herstellung von Produkten der Formel (I''), wie in Anspruch 1 definiert, dadurch gekennzeichnet, daß man ein Produkt der Formel(IV) oder (I'a) 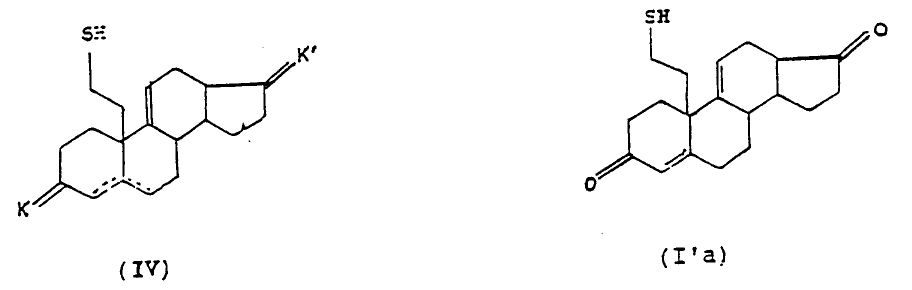 wie vorstehend definiert, der Einwirkung eines 2,4-Dinitrophenylsulfenylhalogenids unterzieht, um zu einem Produkt der Formel (VII) 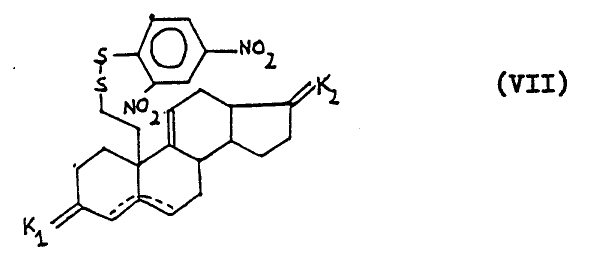 zu gelangen, worin K₁ und K₂ alle beide eine Ketonfunktion oder alle beide eine geschützte Ketonfunktion wiedergeben und die gestrichelte Linie eine Doppelbindung in 4(5)- oder 5(6)- oder 3(4)- und 5(6)-Position wiedergibt, wenn K₁ eine geschützte Ketonfunktion bedeutet,oder in 4(5)-Position, wenn K₁ eine Ketonfunktion bedeutet, welches Produkt der Formel (VII) man
1) entweder gegebenenfalls einem Reagens zur Abspaltung der Schutzgruppen der Ketonfunktionen in 3- oder 17-Stellung unterzieht, um zu einem Produkt der Formel (Id) 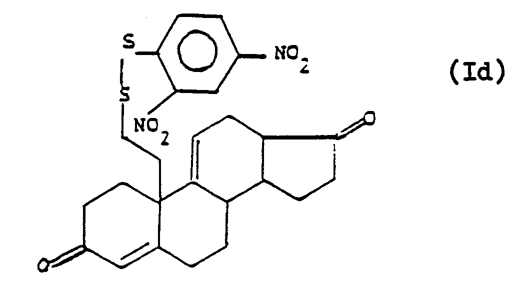 zu gelangen,
2) oder der Einwirkung eines Organomagnesiumreagens der Formel R''-Mg-Hal, worin Hal für ein Halogenatom steht und R'' einen Alkyl-, Alkenyl-, Alkinyl- oder CN-Rest bedeutet, oder der Einwirkung eines geschützten Organolithiumderivats R''Li, danach einem Mittel für die Schutzgruppenentfernung des Restes R'', anschließend gegebenenfalls einer Schutzgruppenabspaltungsreaktion der Ketonfunktionen in 3- und/oder 17-Stellung unterzieht, um zu einem Produkt der Formel (Ie) 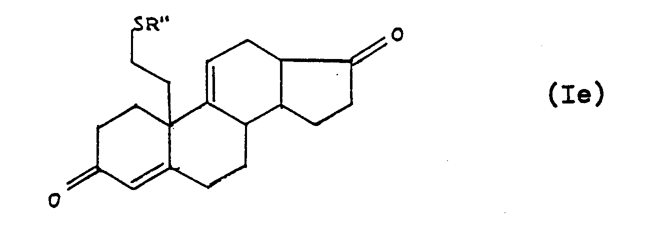 zu gelangen, worin R'' die vorstehend angegebene Bedeutung besitzt,
3) oder der Einwirkung eines Salzes der Formel R'''SA, worin A die in Anspruch 1 angegebene Bedeutung besitzt und R''' einen Alkylrest mit höchstens 6 Kohlenstoffatomen oder einen gegebenenfalls substituierten Arylrest mit höchstens 10 Kohlenstoffatomen bedeutet, danach gegebenenfalls einer Reaktion für die Schutzgruppenabspaltung der Ketonfunktionen in 3- und/oder 17-Stellung unterzieht, um zu einem Produkt der Formel (If) 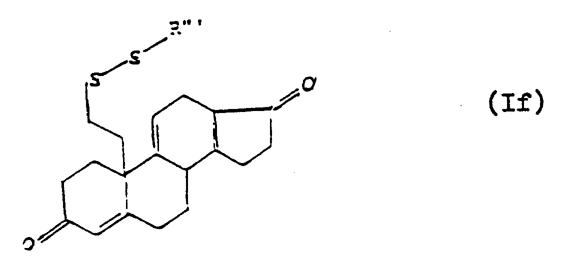 zu gelangen, und die Produkte der Formel (Id), (Ie), (If) gewünschtenfalls einer oder mehreren der in Anspruch 1 für die Produkte der Formel (Ia) oder (Ib) angegebenen Reaktionen in beliebiger Reihenfolge unterzieht.

3. Verfahren zur Herstellung der der Formel (I'') entsprechenden Produkte, worin =X und/oder =Y für 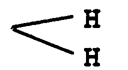 stehen, dadurch gekennzeichnet, daß man ein Produkt der Formel (II), wie in Anspruch 1 definiert, einem Reduktionsmittel für die geschützten Ketonfunktionen unterzieht, um zu einem Produkt der Formel (III) zu gelangen, worin =K und/oder =K' Wasserstoffatome darstellen, und die Synthese, wie in Anspruch 1 oder 2 angegeben, fortführt.

4. Verfahren gemäß einem der Ansprüche 1 bis 3, dadurch gekennzeichnet, daß man Produkte der Formel (I') 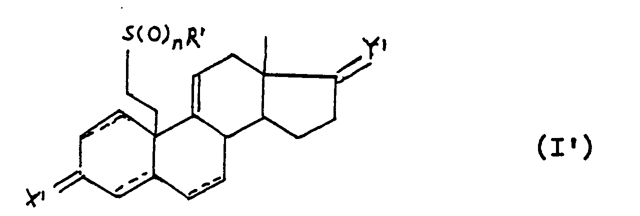 herstellt, worin R' bedeutet:
einen Alkyl-, Alkenyl-, Alkinyl- oder Alkylthiorest mit höchstens 4 Kohlenstoffatomen,
einen Rest CN,
einen Acylrest mit höchstens 4 Kohlenstoffatoman,
X' ein Sauerstoffatom, einen Rest CH₂, einen Rest N-O-R'₁, worin R'₁ für ein Wasserstoffatom oder einen Alkylrest mit 1 bis 4 Kohlenstoffatomen steht, bedeutet oder X' einen Rest 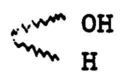 wiedergibt,
Y' ein Sauerstoffatom bedeutet oder =Y' einen Rest 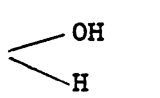 wiedergibt, wobei die Hydroxygruppe gegebenenfalls in Form eines Alkanoyloxyrestes oder eines Restes 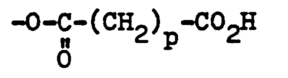 acyliert sein kann, worin p eine Zahl von 2 bis 5 bedeutet, die gestrichelten Linien in 1(2)-, 4(5)- und 6(7)-Position die etwaige Anwesenheit einer zweiten Bindung zwischen den sie tragenden Kohlenstoffen anzeigen, sowie deren Salze.

5. Verfahren gemäß Anspruch 4, dadurch gekennzeichnet, daß man Produkte herstellt, worin R' ausgewählt ist unter den Alkyl-, Alkenyl- oder Alkinylresten mit 1 bis 4 Kohlenstoffatomen und X' ein Sauerstoffatom oder einen Rest 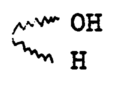 darstellt.

6. Verfahren gemäß Anspruch 4, dadurch gekennzeichnet, daß man die den folgenden Bezeichnungen entsprechenden Produkte herstellt:
10β-[2-(Methylthio)-ethyl]-östra-4,9(11)-dien-3,17-dion,
10β-[2-(Ethylthio)-ethyl]-östra-4,9(11)-dien-3,17-dion,
10β-[2-(Methylthio)-ethyl]-17β-hydroxy-östra-4,9(11)-dien-3-on,
10β-[2-(Ethenylthio)-ethyl]-östra-4,9(11)-dien-3,17-dion,
10β-[2-(Ethinylthio)-ethyl]-östra-4,9(11)-dien-3,17-dion,
10β-[2-(Methylthio)-ethyl]-3-oxo-östra-4,9(11)-dien-17β-yl-natriumbutandioat,
10β-[2-(Methylthio)-ethyl]-östra-4,9(11)-dien-3β, 17β-diol,
10β-[2-(Methylthio)-ethyl]-östra-4,9(11)-dien-3α, 17β-diol,
10β-[2-(Methylthio)-ethyl]-östr-9(11)-en-3,17-dion.

## Patentansprüche (Patentansprüche für folgende(n) Vertragsstaat(en): GR)

1. Verfahren zur Herstellung von Produkten der allgemeinen Formel (I'') 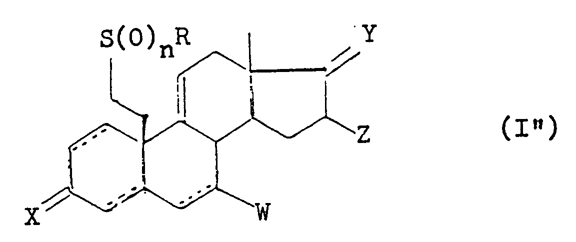 worin
R bedeutet: ein Wasserstoffatom, einen Alkyl-, Alkenyl-, Alkinyl-, Alkylthiorest mit höchstens 6 Kohlenstoffatomen oder einen gegebenenfalls substituierten Arylthiorest mit höchstens 10 Kohlenstoffatomen, einen Acylrest mit höchstens 12 Kohlenstoffatomen oder einen Rest CN,
X ein Sauerstoffatom, einen Rest N-O-R₁ bedeutet, worin R₁ ein Wasserstoffatom oder einen Alkylrest mit 1 bis 6 Kohlenstoffatomen darstellt, oder X für 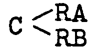 steht, worin RA und RB identisch sind und ein Wasserstoffatom darstellen, oder =X einen Rest 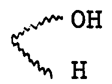 wiedergibt, worin der Rest OH sich in der α- oder β-Stellung befinden kann, wobei die Hydroxylgruppe gegebenenfalls acyliert ist,
Y ein Sauerstoffatom, einen Rest N-O-R₁, worin R₁ die vorstehend angegebene Bedeutung besitzt, bedeutet oder =Y für einen Rest 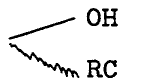 steht, worin RC ein Wasserstoffatom darstellt und wobei die Hydroxylgruppe gegebenenfalls acyliert ist, oder =Y für eine Gruppe 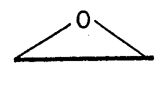 (CH₂)_{q} steht, in der q für eine Zahl von 1 bis 3 steht,
W ein Wasserstoffatom oder einen gegebenenfalls substituierten Alkylthio- oder Arylthiorest mit bis zu 10 Kohlenstoffatomen bedeutet,
Z für ein Wasserstoffatom oder einen Alkylrest mit bis zu 10 Kohlenstoffatomen steht,
n eine ganze Zahl von 0 bis 1 darstellt und die gestrichelten Linien in den 1(2)-, 4(5)- und 6(7)-Positionen die etwaige Anwesenheit einer zweiten Bindung zwischen den sie tragenden Kohlenstoffen anzeigen,
sowie von deren Salzen,
mit der Maßgabe, daß n für 1 steht, R einen Methylrest bedeutet, X und Y jeweils ein Sauerstoffatom darstellen, W und Z jeweils ein Wasserstoffatom bedeuten, die gestrichelte Linie in 4(5)-Stellung eine zweite Bindung zwischen den sie tragenden Kohlenstoffen darstellt und die gestrichelten Linien in 1(2)- und 6(7)-Stellung keine zweite Bindung zwischen den sie tragenden Kohlenstoffen aufweisen,
dadurch gekennzeichnet,
daß man ein Produkt der Formel (II) 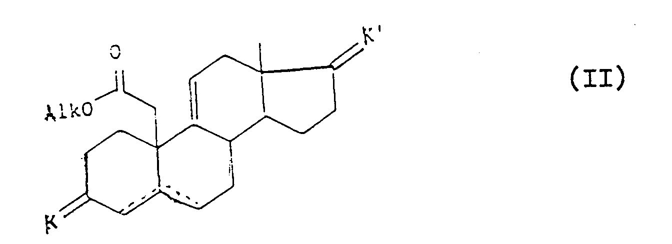 worin K und K',identisch oder voneinander verschieden, eine geschützte Ketogruppe bedeuten, mit der Maßgabe, daß, wenn K einen Enolether darstellt, sich die Doppelbindungen in der 3(4)- und 5(6)-Position befinden, und Alk für einen Alkylrest mit 1 bis 4 Kohlenstoffatomen steht, wobei die gestrichelten Linien eine Doppelbindung in der 4(5)- oder 5(6)-Position bedeuten, einer Reduktion unterzieht, um zu einem Produkt der Formel (III) 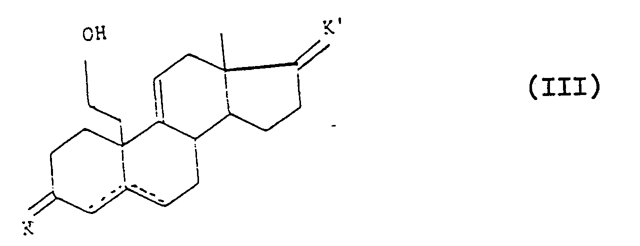 zu gelangen, welches man behandelt,
entweder mit einem Reagens für die Überführung der Hydroxygruppe in eine Thiolgruppe, um zu einem Produkt der Formel (IV) 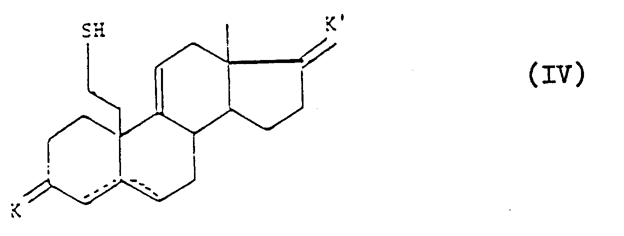 zu gelangen, welches man mit einem reaktiven Derivat des Restes R behandelt, um ein Produkt der Formel (V) 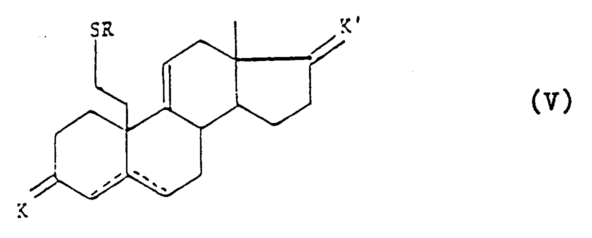 zu erhalten,
oder mit einem reaktiven Derivat einer Sulfonsäure, um zu einem Produkt der Formel (VI) 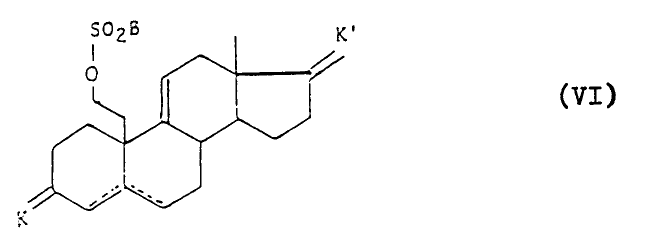 zu gelangen, worin B den Rest einer Sulfonsäure wiedergibt, welches Produkt der Formel (VI) man mit einem Salz der Formel RSA behandelt, worin A ein einwertiges Kation darstellt, um zu einem Produkt der Formel (V), wie vorstehend definiert, zu gelangen, welches Produkt der Formel (V) man mit einem Reagens für die Abspaltung der Schutzgruppen der geschützten Ketonfunktionen behandelt, um zu einem Produkt der Formel (Ia) 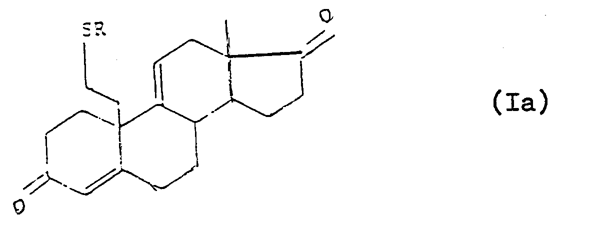 zu gelangen, welches den Produkten der Formel (I'') entspricht, worin R die vorstehend angegebenen Bedeutungen besitzt, X und Y jeweils ein Sauerstoffatom bedeuten, n die Zahl 0 wiedergibt und die gestrichelten Linien in der 4(5)-Position eine Doppelbindung anzeigen und die gestrichelten Linien in den 1(2)- und 6(7)-Positionen keine zweite Bindung zwischen den sie tragenden Kohlenstoffen wiedergeben, wobei man die Produkte der Formel (Ia) gewünschtenfalls einer oder mehreren der folgenden Reaktionen in beliebiger Reihenfolge unterzieht:
- das Einführen einer Doppelbindung in 1(2)-Position
- das Einführen einer Doppelbindung in 6(7)-Position
- die Oxidation des Schwefelatoms des Restes -SR zum Sulfoxid
- die selektive Reduktion der Doppelbindung in 4(5)-Position
- das Einführen eines Alkylrestes in 16-Stellung
- das Einführen eines gegebenenfalls substituierten Alkylthio- oder Arylthiorestes in 7-Stellung,
um zu den Produkten der Formel (Ib) 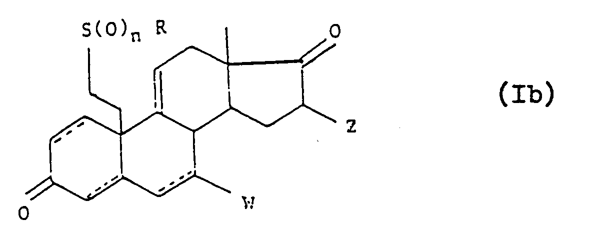 zu gelangen, worin n die ganzen Zahlen 0 oder 1 bedeutet und die gestrichelten Linien in 1(2)-, 4(5)- und 6(7)-Position die etwaige Anwesenheit einer Doppelbindung zwischen den sie tragenden Kohlenstoffen anzeigen, entsprechend den Produkten der Formel (I''), worin R die vorstehend angegebenen Bedeutungen besitzt, X und Y jeweils ein Sauerstoffatom bedeuten und W, Z und n sowie die gestrichelten Linien in 1(2)-, 4(5)-und 6(7)-Position die vorstehend angegebenen Bedeutungen besitzen, mit Ausnahme der Produkte der Formel (Ib), worin Z = H, W = H, n = O und worin die gestrichelten Linien in 1(2)- und 6(7)-Position keine zweite Bindung zwischen den sie tragenden Kohlenstoffen wiedergeben und die gestrichelten Linien in der 4(5)-Position eine zweite Bindung zwischen den sie tragenden Kohlenstoffen wiedergeben, und gewünschtenfalls die Produkte der Formel (Ia) und (Ib) einer oder mehreren der folgenden Reaktionen in beliebiger Reihenfolge unterzieht:
- das Einführen eines Restes 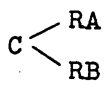 in 3-Stellung
- die Umsetzung mit Hydroxylamin oder einem Derivat der Formel H₂N-O-R₁,
- die Reduktion der Ketonfunktion in 3- und/oder 17-Stellung und die etwaige Acylierung der Hydroxyfunktion und gewünschtenfalls anschließend die Überführung der veresterten Funktion in ein Salz,
- die Umwandlung der Ketonfunktion in 17-Stellung in eine Gruppe 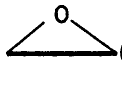 (CH₂)_{q}, um zu einem Produkt der Formel (Ic) 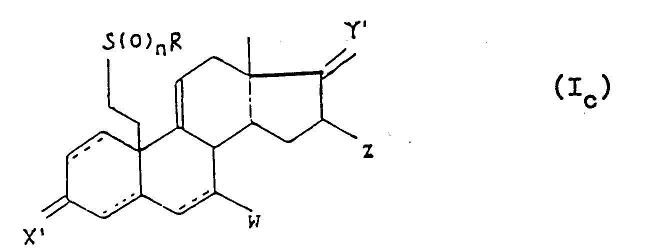 zu gelangen, worin n, R, W, Z und die gestrichelten Linien in 1(2)-, 4(5)- und 6(7)-Position die vorstehend angegebene Bedeutung besitzen und X' und Y' die vorstehend für X und Y angegebenen Bedeutungen besitzen, mit der Maßgabe, daß zumindest eines von X oder Y kein Sauerstoffatom bedeutet.

2. Verfahren zur Herstellung von Produkten der Formel (I''), wie in Anspruch 1 definiert, dadurch gekennzeichnet, daß man ein Produkt der Formel(IV) oder (I'a) 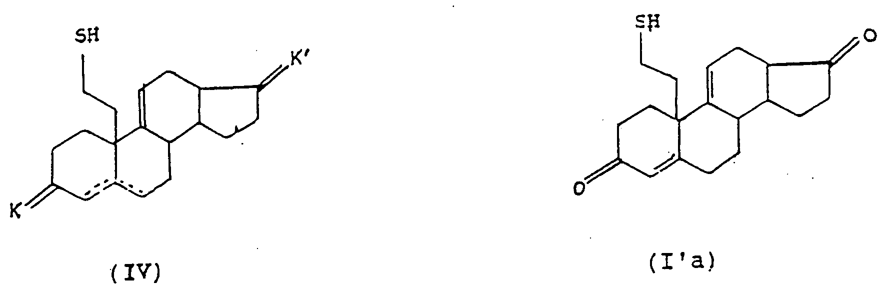 wie vorstehend definiert, der Einwirkung eines 2,4-Dinitrophenylsulfenylhalogenids unterzieht, um zu einem Produkt der Formel (VII) 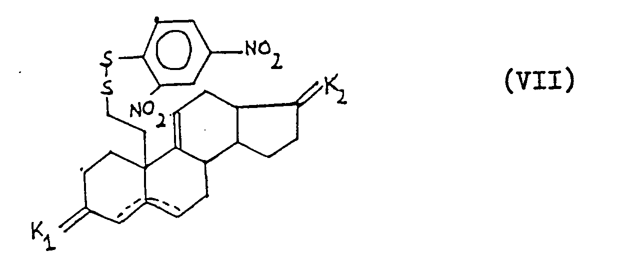 zu gelangen, worin K₁ und K₂ alle beide eine Ketonfunktion oder alle beide eine geschützte Ketonfunktion wiedergeben und die gestrichelte Linie eine Doppelbindung in 4(5)- oder 5(6)- oder 3(4)- und 5(6)-Position wiedergibt, wenn K₁ eine geschützte Ketonfunktion bedeutet,oder in 4(5)-Position, wenn K₁ eine Ketonfunktion bedeutet, welches Produkt der Formel (VII) man
1) entweder gegebenenfalls einem Reagens zur Abspaltung der Schutzgruppen der Ketonfunktionen in 3- oder 17-Stellung unterzieht, um zu einem Produkt der Formel (Id) 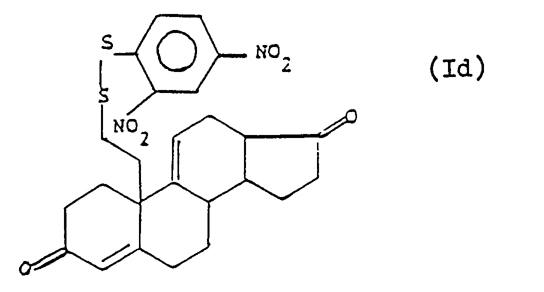 zu gelangen,
2) oder der Einwirkung eines Organomagnesiumreagens der Formel R''-Mg-Hal, worin Hal für ein Halogenatom steht und R'' einen Alkyl-, Alkenyl-, Alkinyl- oder CN-Rest bedeutet, oder der Einwirkung eines geschützten Organolithiumderivats R''Li, danach einem Mittel für die Schutzgruppenentfernung des Restes R'', anschließend gegebenenfalls einer Schutzgruppenabspaltungsreaktion der Ketonfunktionen in 3- und/oder 17-Stellung unterzieht, um zu einem Produkt der Formel (Ie) 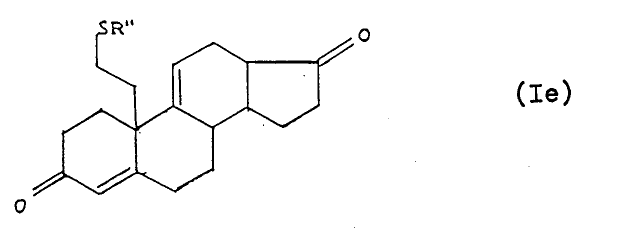 zu gelangen, worin R'' die vorstehend angegebene Bedeutung besitzt,
3) oder der Einwirkung eines Salzes der Formel R'''SA, worin A die in Anspruch 1 angegebene Bedeutung besitzt und R''' einen Alkylrest mit höchstens 6 Kohlenstoffatomen oder einen gegebenenfalls substituierten Arylrest mit höchstens 10 Kohlenstoffatomen bedeutet, danach gegebenenfalls einer Reaktion für die Schutzgruppenabspaltung der Ketonfunktionen in 3- und/oder 17-Stellung unterzieht, um zu einem Produkt der Formel (If) 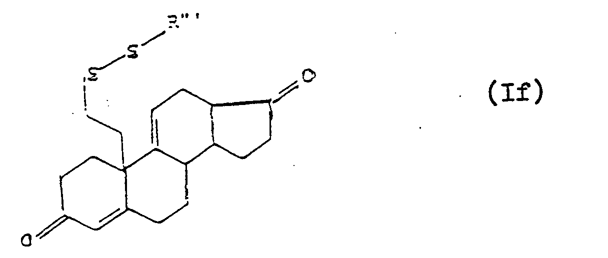 zu gelangen, und die Produkte der Formel (Id), (Ie), (If) gewünschtenfalls einer oder mehreren der in Anspruch 1 für die Produkte der Formel (Ia) oder (Ib) angegebenen Reaktionen in beliebiger Reihenfolge unterzieht.

3. Verfahren zur Herstellung der der Formel (I'') entsprechenden Produkte, worin =X und/oder =Y für 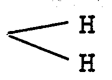 stehen, dadurch gekennzeichnet, daß man ein Produkt der Formel (II), wie in Anspruch 1 definiert, einem Reduktionsmittel für die geschützten Ketonfunktionen unterzieht, um zu einem Produkt der Formel (III) zu gelangen, worin =K und/oder =K' Wasserstoffatome darstellen, und die Synthese, wie in Anspruch 1 oder 2 angegeben, fortführt.

4. Verfahren gemäß einem der Ansprüche 1 bis 3, dadurch gekennzeichnet, daß man Produkte der Formel (I') 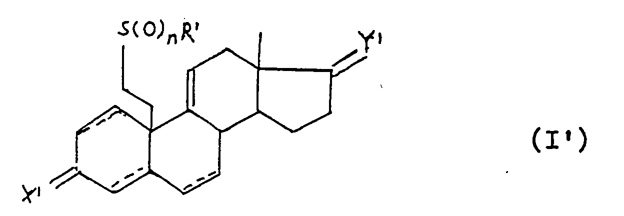 herstellt, worin R' bedeutet:
einen Alkyl-, Alkenyl-, Alkinyl- oder Alkylthiorest mit höchstens 4 Kohlenstoffatomen,
einen Rest CN,
einen Acylrest mit höchstens 4 Kohlenstoffatomen,
X' ein Sauerstoffatom, einen Rest CH₂, einen Rest N-O-R'₁, worin R'₁ für ein Wasserstoffatom oder einen Alkylrest mit 1 bis 4 Kohlenstoffatomen steht, bedeutet oder X' einen Rest 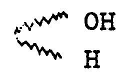 wiedergibt,
Y' ein Sauerstoffatom bedeutet oder =Y' einen Rest 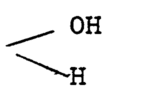 wiedergibt, wobei die Hydroxygruppe gegebenenfalls in Form eines Alkanoyloxyrestes oder eines Restes 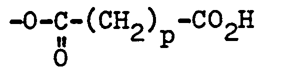 acyliert sein kann, worin p eine Zahl von 2 bis 5 bedeutet, die gestrichelten Linien in 1(2)-, 4(5)- und 6(7)-Position die etwaige Anwesenheit einer zweiten Bindung zwischen den sie tragenden Kohlenstoffen anzeigen, sowie deren Salze.

5. Verfahren gemäß Anspruch 4, dadurch gekennzeichnet, daß man Produkte herstellt, worin R' ausgewählt ist unter den Alkyl-, Alkenyl- oder Alkinylresten mit 1 bis 4 Kohlenstoffatomen und X' ein Sauerstoffatom oder einen Rest 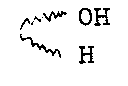 darstellt.

6. Verfahren gemäß Anspruch 4, dadurch gekennzeichnet, daß man die den folgenden Bezeichnungen entsprechenden Produkte herstellt:
10β-[2-(Methylthio)-ethyl]-östra-4,9(11)-dien-3,17-dion,
10β-[2-(Ethylthio)-ethyl]-östra-4,9(11)-dien-3,17-dion,
10β-[2-(Methylthio)-ethyl]-17β-hydroxy-östra-4,9(11)-dien-3-on,
10β-[2-(Ethenylthio)-ethyl]-östra-4,9(11)-dien-3,17-dion,
10β-[2-(Ethinylthio)-ethyl]-östra-4,9(11)-dien-3,17-dion,
10β-[2-(Methylthio)-ethyl]-3-oxo-östra-4,9(11)-dien-17β-yl-natriumbutandioat,
10β-[2-(Methylthio)-ethyl]-östra-4,9(11)-dien-3β, 17β-diol,
10β-[2-(Methylthio)-ethyl]-östra-4,9(11)-dien-3α, 17β-diol,
10β-[2-(Methylthio)-ethyl]-östr-9(11)-en-3,17-dion.

7. Verfahren zur Herstellung von pharmazeutischen Zusammensetzungen, dadurch gekennzeichnet, daß man als Wirkstoff zumindest eine der Verbindungen der Formel (I''), wie in Anspruch 1 definiert, oder zumindest eines ihrer pharmazeutisch verträglichen Salze in eine für diese Verwendung bestimmte Form überführt.

8. Verfahren zur Herstellung von pharmazeutischen Zusammensetzungen, dadurch gekennzeichnet, daß man als Wirkstoff zumindest eine der Verbindungen der Formel (I'') oder der Verbindungen entsprechend denjenigen der Formel (I''), worin =X und/oder =Y zwei Wasserstoffatome bedeuten, wie in einem der Ansprüche 2 bis 5 definiert,oder zumindest eines ihrer pharmazeutisch verträglichen Salze in eine für diese Verwendung bestimmte Form überführt.

9. Verfahren gemäß Anspruch 7 oder 8, dadurch gekennzeichnet, daß die Verbindung der Formel (I) unter den Produkten mit den folgenden Bezeichnungen ausgewählt wird:
10β-[2-(Methylthio)-ethyl]-östra-4,9(11)-dien-3,17-dion,
10β-[2-(Ethylthio)-ethyl]-östra-4,9(11)-dien-3,17-dion,
10β-[2-(Methylthio)-ethyl]-17β-hydroxy-östra-4,9(11)-dien-3-on,
10β-[2-(Ethenylthio)-ethyl]-östra-4,9(11)-dien-3, 17-dion,
10β-[2-(Ethinylthio)-ethyl]-östra-4,9(11)-dien-3, 17-dion,
10β-[2-(Methylthio)-ethyl]-3-oxo-östra-4,9(11)-dien-17β-yl-natriumbutandioat,
10β-[2-(Methylthio)-ethyl]-östra-4,9(11)-dien-3β, 17β-diol,
10β-[2-(Methylthio)-ethyl]-östra-4,9(11)-dien-3α, 17β-diol,
10β-[2-(Methylthio)-ethyl]-östr-9(11)-en-3,17-dion.

10. Als industrielle Produkte die Produkte der allgemeinen Formeln (III), (IV), (V) und (VI), wie in Anspruch 1 definiert, dadurch gekennzeichnet, daß =K und/oder =K' in Form der Ethylendioxy-, Ethylendithio-Reste oder in Form des Ethylenolethers vom 3-Ethoxy-3(4),5(6)-dien-Typ geschützte Ketonfunktionen bedeuten, und daß B den Rest einer Sulfonsäure, ausgewählt unter einem Methyl- oder Tolylrest, darstellt.
